# EUROPÄISCHE PATENTSCHRIFT

(11) **EP 1 699 752 B1**
(45) Veröffentlichungstag und Bekanntmachung des Hinweises auf die Patenterteilung: **26.09.2007**
(21) Anmeldenummer: 04804217.0
(22) Anmeldetag: 22.12.2004
(51) Int. Cl.: C07C 251/38, C07D 213/82, C07D 207/34, C07D 263/34, C07D 277/56, C07D 279/12, C07D 307/30, C07D 307/54, C07D 307/71, C07D 333/38, A01N 43/10, A01N 43/40, A01N 43/56, A01N 43/74

(54) **(HETERO)CYCLYLCARBOXANILIDE ZUR BEKÄMPFUNG VON SCHADPILZEN**
(HETERO)CYCLYL CARBOXANILIDES FOR CONTROLLING HARMFUL FUNGI
(HETERO)CYCLYLCARBOXANILIDES UTILISES POUR LUTTER CONTRE LES CHAMPIGNONS NUISIBLES

(30) Priorität: 23.12.2003 DE 10361005
(43) Veröffentlichungstag der Anmeldung: 13.09.2006
(73) Patentinhaber: BASF Aktiengesellschaft, 67056 Ludwigshafen (DE)
(72) Erfinder: GEWEHR, Markus, 56288 Kastellaun (DE); MÜLLER, Bernd, 67227 Frankenthal (DE); GROTE, Thomas, 67157 Wachenheim (DE); GRAMMENOS, Wassilios, 67071 Ludwigshafen (DE); TORMO I BLASCO, Jordi, 69514 Laudenbach (DE); SCHWÖGLER, Anja, 68165 Mannheim (DE); RHEINHEIMER, Joachim, 67063 Ludwigshafen (DE); BLETTNER, Carsten, 67063 Ludwigshafen (DE); SCHÄFER, Peter, 67308 Ottersheim (DE); SCHIEWECK, Frank, 67258 Hessheim (DE); WAGNER, Oliver, 67433 Neustadt (DE); SPEAKMAN, John-Bryan, 67273 Bobenheim (DE); RETHER, Jan, 67655 Kaiserslautern (DE); STRATHMANN, Siegfried, 67117 Limburgerhof (DE); STIERL, Reinhard, 67251 Freinsheim (DE)
(74) Vertreter: Reitstötter - Kinzebach
(86) Internationale Anmeldenummer: PCT/EP2004/014622
(87) Internationale Veröffentlichungsnummer: WO 2005/063692

(56) Entgegenhaltungen:
- EP-A- 1 118 609
- WO-A-02/08197

## Beschreibung

Die vorliegende Erfindung betrifft (hetero)cyclische Carbonsäureanilide mit einer Oximetherfunktion und deren Verwendung zur Bekämpfung von Schadpilzen.

Die WO 02/08195 beschreibt fungizid wirksame 1,3-Dimethyl-5-fluorpyrazol-4-carbonsäureanilide, die in der 2-Position des Phenylrings eine Phenylgruppe mit Oximethergruppe aufweisen. Aus der WO 02/08197 sind strukturell ähnliche Hetarylanilide bekannt. Die WO 98/03500 beschreibt Carbanilide, die am Phenylring u. a. auch eine Oximarylether-Gruppe aufweisen können. Beispiele werden hierfür jedoch nicht gegeben.

Die dort beschriebenen (Heteroaryl)carbonsäureanilide sind jedoch insbesondere bei niedrigen Aufwandmengen nicht in vollem Umfang zufriedenstellend. Aufgabe der vorliegenden Erfindung war es, neue (Heterocyclyl)carbonsäureanilid-Derivate zur Verfügung zu stellen.

Der vorliegenden Erfindung liegt daher die Aufgabe zugrunde, fungizid wirkende Verbindungen bereitzustellen, die die Nachteile der aus dem Stand der Technik bekannten Verbindungen überwinden und insbesondere eine verbesserte Wirkung bei niedrigen Aufwandmengen zeigen. Außerdem sollten diese Verbindungen eine gute Nutzpflanzenverträglichkeit aufweisen und möglichst keine oder nur eine geringe Schädlichkeit gegenüber tierischen Nützlingen zeigen.

Diese Aufgabe wird durch die im Folgenden beschriebenen (Hetero)cyclylcarboxanilide der allgemeinen Formel I und durch ihre landwirtschaftlich verträglichen Salze gelöst.

Die vorliegende Erfindung betrifft daher (Hetero)cyclylcarboxanilide der allgemeinen Formel 1, 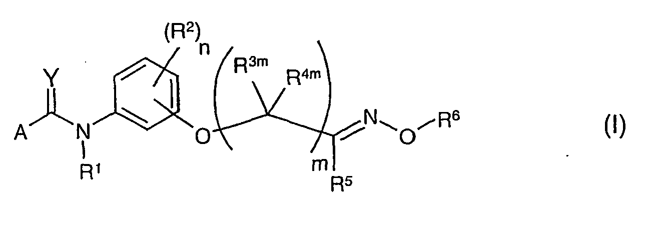 in denen die Variablen die folgenden Bedeutungen haben:
- A: Phenyl oder ein wenigstens einfach ungesättigter 5- oder 6-gliedrigen Heterocyclus mit 1, 2 oder 3, unter N, O, S, S(=O) und S(=O)₂ ausgewählten Heteroatomen als Ringglieder, wobei Phenyl und der wenigstens einfach ungesättigte 5- oder 6-gliedrige Heterocyclus unsubstituiert sein können oder 1, 2 oder 3 Reste R^{a} tragen können, wobei
R^{a} für Halogen, Nitro, CN, C₁-C₄-Alkyl, C₃-C₆-Cycloalkyl, C₂-C₄-Alkenyl, C₂-C₄-Alkinyl, C₁-C₄-Alkoxy, C₁-C₄-Halogenalkyl, C₃-C₆-Halogencycloalkyl, C₂-C₄-Halogenalkenyl, C₂-C₄-Halogenalkinyl, C₁-C₄-Halogenalkoxy oder Phenyl steht, wobei Phenyl unsubstituiert sein kann oder ein, zwei oder drei Reste R^{b} trägt, die ausgewählt sind unter Halogen, Nitro, CN, C₁-C₄-Alkyl, C₃-C₆-Cycloalkyl, C₂-C₄-Alkenyl, C₂-C₄-Alkinyl, C₁-C₄-Alkoxy, C₁-C₄-Halogenalkyl, C₃-C₆-Halogencycloalkyl, C₂-C₄-Halogenalkenyl, C₂-C₄-Halogenalkinyl und C₁-C₄-Halogenalkoxy;
Y Sauerstoff oder Schwefel;
R¹ H, OH, C₁-C₄-Alkyl, C₃-C₆-Cycloalkyl, C₁-C₄-Alkoxy, C₁-C₄-Halogenalkyl, C₃-C₆-Halogencycloalkyl oder C₁-C₄-Halogenalkoxy;
R² Halogen, Nitro, CN, C₁-C₄-Alkyl, C₃-C₆-Cycloalkyl, C₂-C₄-Alkenyl, C₂-C₄-Alkinyl, C₁-C₄-Alkoxy, C₁-C₄-Halogenalkyl, C₃-C₆-Halogencycloalkyl, C₂-C₄-Halogenalkenyl, C₂-C₄-Halogenalkinyl oder C₁-C₄-Halogenalkoxy;
R^{3m}, R^{4m} jeweils unabhängig voneinander Halogen, Wasserstoff, C₁-C₆-Alkyl, C₃-C₆-Cycloalkyl, C₂-C₆-Alkenyl, C₂-C₆-Alkinyl, Phenyl, Phenyl-C₁-C₄-alkyl, Phenyl-C₂-C₄-alkenyl, Phenyl-C₂-C₄-alkinyl, C₁-C₆-Halogenalkyl, C₃-C₆-Halogencycloalkyl, C₂-C₆-Halogenalkenyl, C₂-C₆-Halogenalkinyl, Phenyl-C₁-C₄-halogenalkyl, Phenyl-C₂-C₄-halogenalkenyl oder Phenyl-C₂-C₄-halogenalkinyl, wobei Phenyl oder der Phenylteil von Phenyl-C₁-C₄-alkyl, Phenyl-C₂-C₄-alkenyl, Phenyl-C₂-C₄-alkinyl, Phenyl-C₁-C₄-halogenalkyl, Phenyl-C₂-C₄-halogenalkenyl und Phenyl-C₂-C₄-halogenalkinyl unsubstituiert sein können oder ein, zwei oder drei Reste R^{b} tragen können; für m = 2 oder 3, die Variablen R³², R⁴² beziehungsweise R³³, R⁴³ auch für C₁-C₆-Alkoxy stehen können;
R⁵ Wasserstoff, C₁-C₆-Alkyl, C₃-C₆-Cycloalkyl, C₂-C₆-Alkenyl, C₂-C₆-Alkinyl, Phenyl, Phenyl-C₁-C₄-alkyl, Phenyl-C₂-C₄-alkenyl, Phenyl-C₂-C₄-alkinyl, C₁-C₆-Halogenalkyl, C₃-C₆-Halogencycloalkyl, C₂-C₆-Halogenalkenyl, C₂-C₆-Halogenalkinyl, Phenyl-C₁-C₄-halogenalkyl, Phenyl-C₂-C₄-halogenalkenyl oder Phenyl-C₂-C₄-halogenalkinyl, wobei Phenyl oder der Phenylteil von Phenyl-C₁-C₄-alkyl, Phenyl-C₂-C₄-alkenyl, Phenyl-C₂-C₄-alkinyl, Phenyl-C₁-C₄-halogenalkyl, Phenyl-C₂-C₄-halogenalkenyl, Phenyl-C₂-C₄-halogenalkinyl unsubstituiert sein können oder ein, zwei oder drei Reste R^{b} tragen können;
R⁶ Wasserstoff, C₁-C₈-Alkyl, C₃-C₆-Cycloalkyl, C₂-C₈-Alkenyl, C₂-C₈-Alkinyl, C₁-C₈-Halogenalkyl, C₃-C₆-Halogencycloalkyl, C₂-C₈-Halogenalkenyl, C₂-C₈-Halogenalkinyl, Phenyl, Naphthyl, Phenyl-C₁-C₆-alkyl, Naphthyl-C₁-C₆-alkyl, Phenyl-C₂-C₆-alkenyl, Phenyl-C₂-C₆-alkinyl, Phenyl-C₁-C₆-halogenalkyl, Phenyl-C₂-C₆-halogenalkenyl oder Phenyl-C₂-C₆-halogenalkinyl, wobei Phenyl und Naphthyl in den 9 zuletzt genannten Gruppen unsubstituiert sein können oder 1, 2 oder 3 unter R^{b} und R⁷ ausgewählte Substituenten tragen können, wobei R⁷ für-(CR⁸)=NOR⁹ steht, worin
R⁸ Wasserstoff, C₁-C₆-Alkyl, C₃-C₆-Cycloalkyl, C₂-C₆-Alkenyl, C₂-C₆-Alkinyl, C₁-C₆-Halogenalkyl, C₃-C₆-Halogencycloalkyl, C₂-C₆-Halogenalkenyl, C₂-C₆-Halogenalkinyl, Phenyl, Benzyl; wobei Phenyl und die Phenylgruppe in Benzyl unsubstituiert sein können oder ein, zwei oder drei Reste R^{b} tragen können; und
R⁹ C₁-C₆-Alkyl, C₃-C₆-Cycloalkyl, C₂-C₆-Alkenyl, C₂-C₆-Alkinyl, C₁-C₆-Halogenalkyl, C₃-C₆-Halogencycloalkyl, C₂-C₆-Halogenalkenyl, C₂-C₆-Halogenalkinyl, Phenyl, Phenyl-C₁-C₄-alkyl, Phenyl-C₁-C₄-halogenalkyl, Phenyl-C₂-C₄-alkenyl, Phenyl-C₂-C₄-halogenalkenyl, Phenyl-C₂-C₄-alkinyl, Phenyl-C₂-C₄-halogenalkinyl, wobei Phenyl und die Phenylgruppe in Phenyl-C₁-C₄-alkyl, Phenyl-C₁-C₄-halogenalkyl, Phenyl-C₂-C₄-alkenyl, Phenyl-C₂-C₄-halogenalkenyl, Phenyl-C₂-C₄-alkinyl und Phenyl-C₂-C₄-halogenalkinyl unsubstituiert sein können oder ein, zwei oder drei Reste R^{b} tragen können;
- n: 0, 1, 2, 3 oder 4; und
- m: 1, 2 oder 3;
und deren landwirtschaftlich brauchbaren Salze.

Die vorliegende Erfindung betrifft außerdem die Verwendung der (Hetero)cyclylcarboxanilide der allgemeinen Formel I und deren landwirtschaftlich brauchbaren Salze als Fungizide sowie diese enthaltende Pflanzenschutzmittel.

Die vorliegende Erfindung betrifft weiterhin ein Verfahren zur Bekämpfung von pflanzenpathogenen Pilzen (Schadpilzen), das dadurch gekennzeichnet ist, dass man die Schadpilze, deren Lebensraum oder die von ihnen freizuhaltenden Pflanzen, Flächen, Materialien oder Räume mit einer fungizid wirksamen Menge eines (Hetero)cyclylcarboxanilids der allgemeinen Formel und/oder einem landwirtschaftlich brauchbaren Salz von behandelt.

Die Verbindungen der Formel können je nach Substitutionsmuster ein oder mehrere Chiralitätszentren aufweisen und liegen dann als Enantiomeren- oder Diastereomerengemische vor. Gegenstand der Erfindung sind sowohl die reinen Enantiomere oder Diastereomere als auch deren Gemische. Geeignete Verbindungen der Formel I umfassen auch alle möglichen Stereoisomere (cis/trans-Isomere) und Gemische davon.

Unter landwirtschaftlich brauchbaren Salzen kommen vor allem die Salze derjenigen Kationen oder die Säureadditionssalze derjenigen Säuren in Betracht, deren Kationen beziehungsweise Anionen die fungizide Wirkung der Verbindungen l nicht negativ beeinträchtigen. So kommen als Kationen insbesondere die lonen der Alkalimetalle, vorzugsweise Natrium und Kalium, der Erdalkalimetalle, vorzugsweise Calcium, Magnesium und Barium, und der Übergangsmetalle, vorzugsweise Mangan, Kupfer, Zink und Eisen, sowie das Ammoniumion, das gewünschtenfalls ein bis vier C₁-C₄-Alkylsubstituenten und/oder einen Phenyl- oder Benzylsubstituenten tragen kann, vorzugsweise Diisopropylammonium, Tetramethylammonium, Tetrabutylammonium, Trimethylbenzylammonium, des weiteren Phosphoniumionen, Sulfoniumionen, vorzugsweise Tri(C₁-C₄-alkyl)sulfonium und Sulfoxoniumionen, vorzugsweise Tri(C₁-C₄-alkyl)sulfoxonium, in Betracht.

Anionen von brauchbaren Säureadditionssalzen sind in erster Linie Chlorid, Bromid, Fluorid, Hydrogensulfat, Sulfat, Dihydrogenphosphat, Hydrogenphosphat, Phosphat, Nitrat, Hydrogencarbonat, Carbonat, Hexafluorosilikat, Hexafluorophosphat, Benzoat, sowie die Anionen von C₁-C₄-Alkansäuren, vorzugsweise Formiat, Acetat, Propionat und Butyrat. Sie können durch Reaktion von I mit einer Säure des entsprechenden Anions, vorzugsweise der Chlorwasserstoffsäure, Bromwasserstoffsäure, Schwefelsäure, Phosphorsäure oder Salpetersäure, gebildet werden.

Bei den in den vorstehenden Formeln angegebenen Definitionen der Variablen werden Sammelbegriffe verwendet, die allgemein repräsentativ für die jeweiligen Substituenten stehen. Die Bedeutung Cₙ-Cₘ gibt die jeweils mögliche Anzahl von Kohlenstoffatomen in dem jeweiligen Substituenten oder Substituententeil an. Sämtliche Kohlenstoffketten, also alle Alkyl-, Halogenalkyl-, Phenylalkyl-, Alkenyl-, Halogenalkenyl-, Phenylalkenyl-, Alkinyl-, Halogenalkinyl- und Phenylalkinyl-Teile können geradkettig oder verzweigt sein. Halogenierte Substituenten tragen vorzugsweise ein bis fünf gleiche oder verschiedene Halogenatome. Die Bedeutung Halogen steht jeweils für Fluor, Chlor, Brom oder Iod.

Ferner stehen beispielsweise:
- C₁-C₄-Alkyl für: CH₃, C₂H₅, CH₂-C₂H₅, CH(CH₃)₂, n-Butyl, CH(CH₃)-C₂H₅, CH₂-CH(CH₃)₂ oder C(CH₃)₃;
- C₁-C₄-Halogenalkyl: für einen C₁-C₄-Alkylrest wie vorstehend genannt, der partiell oder vollständig durch Fluor, Chlor, Brom und/oder lod substituiert ist, also z.B. CH₂F, CHF₂, CF₃, CH₂Cl, CH(Cl)₂, C(Cl)₃, Chlorfluormethyl, Dichlorfluormethyl, Chlordifluormethyl, 2-Fluorethyl, 2-Chlorethyl, 2-Bromethyl, 2-Iodethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, C₂F₅, 2-Fluorpropyl, 3-Fluorpropyl, 2,2-Difluorpropyl, 2,3-Difluorpropyl, 2-Chlorpropyl, 3-Chlorpropyl, 2,3-Dichlorpropyl, 2-Brompropyl, 3-Brompropyl, 3,3,3-Trifluorpropyl, 3,3,3-Trichlorpropyl, CH₂-C₂F₅, CF₂-C₂F₅, 1-(Fluormethyl)-2-fluorethyl, 1-(Chlormethyl)-2-chlorethyl, 1-(Brommethyl)-2-bromethyl, 4-Fluorbutyl, 4-Chlorbutyl, 4-Brombutyl oder Nonafluorbutyl;
- C₁-C₈-Alkyl: für einen C₁-C₄-Alkylrest wie vorstehend genannt, oder für z.B. n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl oder 1-Ethyl-2-methylpropyl, vorzugsweise für CH₃, C₂H₅, CH₂-C₂H₅, CH(CH₃)₂, n-Butyl, C(CH₃)₃, n-Pentyl, n-Hexyl, n-Heptyl oder n-Octyl;
- C₁-C₈-Halogenalkyl: für einen C₁-C₈-Alkylrest wie vorstehend genannt, der partiell oder vollständig durch Fluor, Chlor, Brom und/oder lod substituiert ist, also z.B. für einen der unter C₁-C₄-Halogenalkyl genannten Reste oder für 5-Fluor-1-pentyl, 5-Chlor-1-pentyl, 5-Brom-1-pentyl, 5-lod-1-pentyl, 5,5,5-Trichlor-1-pentyl, Undecafluorpentyl, 6-Fluor-1-hexyl, 6-Chlor-1-hexyl, 6-Brom-1-hexyl, 6-lod-1-hexyl, 6,6,6-Trichlor-1-hexyl oder Dodecafluorhexyl;
- C₂-C₄-Alkenyl: ungesättigte, geradkettige oder verzweigte Kohlenwasserstoffreste mit 2 bis 4 Kohlenstoffatomen und einer Doppelbindung in einer beliebigen Position, z.B. Ethenyl, 1-Propenyl, 2-Propenyl, 1-Methylethenyl, 1-Buten-1-yl, 1-Buten-2-yl, 1-Buten-3-yl, 2-Buten-1-yl, 1-Methyl-prop-1-en-1-yl, 2-Methyl-prop-1-en-1-yl, 1-Methyl-prop-2-en-1-yl, 2-Methyl-prop-2-en-1-yl;
- C₂-C₆-Alkenyl: für C₂-C₄-Alkenyl wie vorstehend genannt sowie z. B. für n-Penten-1-yl, n-Penten-2-yl, n-Penten-3-yl, n-Penten-4-yl, 1-Methyl-but-1-en-1-yl, 2-Methyl-but-1-en-1-yl, 3-Methyl-but-1-en-1-yl, 1-Methyl-but-2-en-1-yl, 2-Methyl-but-2-en-1-yl, 3-Methyl-but-2-en-1-yl, 1-Methyl-but-3-en-1-yl, 2-Methyl-but-3-en-1-yl, 3-Methyl-but-3-en-1-yl, 1,1-Dimethyl-prop-2-en-1-yl, 1,2-Dimethyl-prop-1-en-1-yl, 1,2-Dimethyl-prop-2-en-1-yl, 1-Ethyl-prop-1-en-2-yl, 1-Ethyl-prop-2-en-1-yl, n-Hex-1-en-1-yl, n-Hex-2-en-1-yl, n-Hex-3-en-1-yl, n-Hex-4-en-1-yl, n-Hex-5-en-1-yl, 1-Methyl-pent-1-en-1-yl, 2-Methyl-pent-1-en-1-yl, 3-Methyl-pent-1-en-1-yl, 4-Methyl-pent-1-en-1-yl, 1-Methyl-pent-2-en-1-yl, 2-Methyl-pent-2-en-1-yl, 3-Methyl-pent-2-en-1-yl, 4-Methyl-pent-2-en-1-yl, 1-Methyl-pent-3-en-1-yl, 2-Methyl-pent-3-en-1-yl, 3-Methyl-pent-3-en-1-yl, 4-Methyl-pent-3-en-1-yl, 1-Methyl-pent-4-en-1-yl, 2-Methyl-pent-4-en-1-yl, 3-Methyl-pent-4-en-1-yl, 4-Methyl-pent-4-en-1-yl, 1,1-Dimethyl-but-2-en-1-yl, 1,1-Dimethyl-but-3-en-1-yl, 1,2-Dimethyl-but-1-en-1-yl, 1,2-Dimethyl-but-2-en-1 -yl, 1,2-Dimethyl-but-3-en-1-yl, 1,3-Dimethyl-but-1-en-1-yl, 1,3-Dimethyl-but-2-en-1-yl, 1,3-Dimethyl-but-3-en-1-yl, 2,2-Dimethyl-but-3-en-1-yl, 2,3-Dimethyl-but-1-en-1-yl, 2,3-Dimethyl-but-2-en-1-yl, 2,3-Dimethyl-but-3-en-1-yl, 3,3-Dimethyl-but-1-en-1-yl, 3,3-Dimethyl-but-2-en-1-yl, 1-Ethyl-but-1-en-1-yl, 1-Ethyl-but-2-en-1-yl, 1-Ethyl-but-3-en-1-yl, 2-Ethyl-but-1-en-1-yl, 2-Ethyl-but-2-en-1-yl, 2-Ethyl-but-3-en-1-yl, 1,1,2-Trimethyl-prop-2-en-1-yl, 1-Ethyl-1-methyl-prop-2-en-1-yl, 1-Ethyl-2-methyl-prop-1-en-1-yl oder 1-Ethyl-2-methyl-prop-2-en-1-yl;
- C₂-C₄-Halogenalkenyl: für ungesättigte, geradkettige oder verzweigte Kohlenwasserstoffreste mit 2 bis 4 Kohlenstoffatomen und einer Doppelbindung in einer beliebigen Position (wie vorstehend genannt), wobei in diesen Gruppen die Wasserstoffatome teilweise oder vollständig gegen Halogenatome wie vorstehend genannt, insbesondere Fluor, Chlor und Brom, ersetzt sind, also z.B. 2-Chlorallyl, 3-Chlorallyl, 2,3-Dichlorallyl, 3,3-Dichlorallyl, 2,3,3-Trichlorallyl, 2,3-Dichlorbut-2-enyl, 2-Bromallyl, 3-Bromallyl, 2,3-Dibromallyl, 3,3-Dibromallyl, 2,3,3-Tribromallyl oder 2,3-Dibrombut-2-enyl;
- C₂-C₆-Halogenalkenyl: für C₂-C₆-Alkenyl wie vorstehend genannt, das partiell oder vollständig durch Fluor, Chlor, Brom und/oder Iod substituiert ist, z.B für die bei C₂-C₄-Halogenalkenyl genannten Reste;
- C₂-C₄-Alkinyl: geradkettige oder verzweigte Kohlenwasserstoffgruppen mit 2 bis 4 Kohlenstoffatomen und einer Dreifachbindung in einer beliebigen Position, z.B. Ethinyl, 1-Propinyl, 2-Propinyl (=Propargyl), 1-Butinyl, 2-Butinyl, 3-Butinyl und 1-Methyl-2-propinyl;
- C₂-C₆-Alkinyl: für geradkettige oder verzweigte Kohlenwasserstoffgruppen mit 2 bis 6 Kohlenstoffatomen und einer Dreifachbindung in einer beliebigen Position, z.B. Ethinyl, Prop-1-in-1-yl, Prop-2-in-1-yl, n-But-1-in-1-yl, n-But-1-in-3-yl, n-But-1-in-4-yl, n-But-2-in-1-yl, n-Pent-1-in-1-yl, n-Pent-1-in-3-yl, n-Pent-1-in-4-yl, n-Pent-1-in-5-yl, n-Pent-2-in-1-yl, n-Pent-2-in-4-yl, n-Pent-2-in-5-yl, 3-Methyl-but-1-in-3-yl, 3-Methyl-but-1-in-4-yl, n-Hex-1-in-1-yl, n-Hex-1-in-3-yl, n-Hex-1-in-4-yl, n-Hex-1-in-5-yl, n-Hex-1-in-6-yl, n-Hex-2-in-1-yl, n-Hex-2-in-4-yl, n-Hex-2-in-5-yl, n-Hex-2-in-6-yl, n-Hex-3-in-1-yl, n-Hex-3-in-2-yl, 3-Methyl-pent-1-in-1-yl, 3-Methyl-pent-1-in-3-yl, 3-Methyl-pent-1-in-4-yl, 3-Methyl-pent-1-in-5-yl, 4-Methyl-pent-1-in-1-yl, 4-Methyl-pent-2-in-4-yl und 4-Methyl-pent-2-in-5-yl;
- C₂-C₄-Halogenalkinyl: für ungesättigte, geradkettige oder verzweigte Kohlenwasserstoffreste mit 2 bis 4 Kohlenstoffatomen und einer Dreifachbindung in einer beliebigen Position (wie vorstehend genannt), wobei in diesen Gruppen die Wasserstoffatome teilweise oder vollständig gegen Halogenatome wie vorstehend genannt, insbesondere Fluor, Chlor und Brom, ersetzt sein können, also z.B. 1,1-Difluorprop-2-in-1-yl, 4-Fluorbut-2-in-1-yl, 4-Chlorbut-2-in-1-yl oder 1,1-Difluorbut-2-in-1-yl,
- C₂-C₆-Halogenalkinyl: für C₂-C₆-Alkinyl wie vorstehend genannt, das partiell oder vollständig durch Fluor, Chlor, Brom und/oder lod substituiert ist, z.B für die bei C₂-C₄-Halogenalkinyl genannten Reste;
- C₁-C₄-Alkoxy: für OCH₃, OC₂H₅, OCH₂-C₂H₅, OCH(CH₃)₂, n-Butoxy, OCH(CH₃)-C₂H₅, OCH₂-CH(CH₃)₂ oder OC(CH₃)₃;
- C₁-C₄-Halogenalkoxy: für einen C₁-C₄-Alkoxyrest wie vorstehend genannt, der partiell oder vollständig durch Fluor, Chlor, Brom und/oder lod substituiert ist, also z.B. OCH₂F, OCHF₂, OCF₃, OCH₂Cl, OCH(Cl)₂, OC(Cl)₃, Chlorfluormethoxy, Dichlorfluormethoxy, Chlordifluormethoxy, 2-Fluorethoxy, 2-Chlorethoxy, 2-Bromethoxy, 2-lodethoxy, 2,2-Difluorethoxy, 2,2,2-Trifluorethoxy, 2-Chlor-2-fluorethoxy, 2-Chlor-2,2-difluorethoxy, 2,2-Dichlor-2-fluorethoxy, 2,2,2-Trichlorethoxy, OC₂F₅, 2-Fluorpropoxy, 3-Fluorpropoxy, 2,2-Difluorpropoxy, 2,3-Difluorpropoxy, 2-Chlorpropoxy, 3-Chlorpropoxy, 2,3-Dichlorpropoxy, 2-Brompropoxy, 3-Brompropoxy, 3,3,3-Trifluorpropoxy, 3,3,3-Trichlorpropoxy, OCH₂-C₂F₅, OCF₂-C₂F₅, 1-(CH₂F)-2-fluorethoxy, 1-(CH₂Cl)-2-chlorethoxy, 1 -(CH₂Br)-2-bromethoxy, 4-Fluorbutoxy, 4-Chlorbutoxy, 4-Brombutoxy o-der Nonafluorbutoxy, vorzugsweise für OCHF₂, OCF₃, Dichlorfluormethoxy, Chlordifluormethoxy oder 2,2,2-Trifluorethoxy;
- C₃-C₆-Cycloalkyl: für Cyclopropyl, Cyclobutyl, Cyclopentyl oder Cyclohexyl;
- C₃-C₆-Cycloalkyl, das gegebenenfalls mit Halogen ein- oder mehrfach substituiert ist: für einen C₃-C₆-Cycloalkylrest wie vorstehend genannt, der unsubstituiert ist oder partiell oder vollständig durch Fluor, Chlor, Brom und/oder lod substituiert ist, also z. B. für 1-Chlorcyclopropyl, 1-Fluorcyclopropyl, 2-Chlorcyclopropyl, 2-Fluorcyclopropyl, 4-Chlorcyclohexyl, 4-Bromcyclohexyl;
- Phenyl-C₁-C₄-alkyl: für C₁-C₄-Alkyl, welches mit Phenyl substituiert ist, z.B. für Benzyl, 1- oder 2-Phenylethyl, 1-, 2- oder 3-Phenylpropyl, wobei der Phenylteil unsubstituiert sein kann oder 1, 2 oder 3 Reste R^{b} tragen kann, worin R^{b} ausgewählt ist unter Halogen, Nitro, CN, C₁-C₄-Alkyl, C₃-C₆-Cycloalkyl, C₂-C₄-Alkenyl, C₂-C₄-Alkinyl und C₁-C₄-Alkoxy, wobei die 5 zuletzt genannten Gruppen durch Halogen substituiert sein können;
- Phenyl-C₁-C₄-halogenalkyl: für C₁-C₄-Halogenalkyl, welches mit Phenyl substituiert ist, wobei der Phenylteil unsubstituiert sein kann oder 1, 2 oder 3 Reste R^{b} tragen kann, worin R^{b} ausgewählt ist unter Halogen, Nitro, CN, C₁-C₄-Alkyl, C₃-C₆-Cycloalkyl, C₂-C₄-Alkenyl, C₂-C₄-Alkinyl und C₁-C₄-Alkoxy, wobei die 5 zuletzt genannten Gruppen durch Halogen substituiert sein können;
- Phenyl-C₂-C₄-alkenyl: für C₂-C₄-Alkenyl, welches mit Phenyl substituiert ist, z.B. für 1- oder 2-Phenylethenyl, 1-Phenylprop-2-en-1-yl, 3-Phenyl-1-propen-1-yl, 3-Phenyl-2-propen-1-yl, 4-Phenyl-1-buten-1-yl oder 4-Phenyl-2-buten-1-yl; wobei der Phenylteil von unsubstituiert sein oder 1, 2 oder 3 Reste R^{b} tragen kann, worin R^{b} ausgewählt ist unter Halogen, Nitro, CN, C₁-C₄-Alkyl, C₃-C₆-Cycloalkyl, C₂-C₄-Alkenyl, C₂-C₄-Alkinyl und C₁-C₄-Alkoxy, wobei die 5 zuletzt genannten Gruppen durch Halogen substituiert sein können;
- Phenyl-C₂-C₄-halogenalkenyl: für C₂-C₄-Halogenalkenyl, welches mit Phenyl substituiert ist, wobei der Phenylteil unsubstituiert sein kann oder 1, 2 oder 3 Reste R^{b} tragen kann, worin R^{b} ausgewählt ist unter Halogen, Nitro, CN, C₁-C₄-Alkyl, C₃-C₆-Cycloalkyl, C₂-C₄-Alkenyl, C₂-C₄-Alkinyl und C₁-C₄-Alkoxy, wobei die 5 zuletzt genannten Gruppen durch Halogen substituiert sein können;
- Phenyl-C₂-C₄-alkinyl: für C₂-C₄-Alkinyl, welches mit Phenyl substituiert ist, z.B. für 1-Phenyl-2-propin-1-yl, 3-Phenyl-1-propin-1-yl, 3-Phenyl-2-propin-1-yl, 4-Phenyl-1-butin-1-yl oder 4-Phenyl-2-butin-1-yl; wobei der Phenylteil von Phenyl-C₂-C₄-alkinyl unsubstituiert sein oder 1,2 oder 3 Reste R^{b} tragen kann, worin R^{b} ausgewählt ist unter Halogen, Nitro, CN, C₁-C₄-Alkyl, C₃-C₆-Cycloalkyl, C₂-C₄-Alkenyl, C₂-C₄-Alkinyl und C₁-C₄-Alkoxy, wobei die 5 zuletzt genannten Gruppen durch Halogen substituiert sein können;
- Phenyl-C₂-C₄-halogenalkinyl: für C₂-C₄-Halogenalkinyl, welches mit Phenyl substituiert ist, wobei der Phenylteil unsubstituiert sein kann oder 1, 2 oder 3 Reste R^{b} tragen kann, worin R^{b} ausgewählt ist unter Halogen, Nitro, CN, C₁-C₄-Alkyl, C₃-C₆-Cycloalkyl, C₂-C₄-Alkenyl, C₂-C₄-Alkinyl und C₁-C₄-Alkoxy, wobei die 5 zuletzt genannten Gruppen durch Halogen substituiert sein können;
- wenigstens einfach ungesättigter Heterocyclus mit 5 oder 6 Ringgliedern: ein monocyclische Heterocyclus, der ein, zwei oder drei unter O, S, S(=O), S(=O)₂ und N ausgewählte Ringglieder aufweist und der wenigstens einfach ungesättigt oder vollständig ungesättigt, d.h. aromatisch ist. Beispiele hierfür sind Furyl wie 2-Furyl und 3-Furyl, Thienyl wie 2-Thienyl und 3-Thienyl, Pyrrolyl wie 2-Pyrrolyl und 3-Pyrrolyl, lsoxazolyl wie 3-lsoxazolyl, 4-lsoxazolyl und 5-Isoxazolyl, lsothiazolyl wie 3-Isothiazolyl, 4-Isothiazolyl und 5-lsothiazolyl, Pyrazolyl wie 3-Pyrazolyl, 4-Pyrazolyl und 5-Pyrazolyl, Oxazolyl wie 2-Oxazolyl, 4-Oxazolyl und 5-Oxazolyl, Thiazolyl wie 2-Thiazolyl, 4-Thiazolyl und 5-Thiazolyl, Imidazolyl wie 2-Imidazolyl und 4-Imidazolyl, Oxadiazolyl wie 1,2,4-Oxadiazol-3-yl, 1,2,4-Oxadiazol-5-yl und 1,3,4-Oxadiazol-2-yl, Thiadiazolyl wie 1,2,4-Thiadiazol-3-yl, 1,2,4-Thiadiazol-5-yl und 1,3,4-Thiadiazol-2-yl, Triazolyl wie 1,2,4-Triazol-1-yl, 1,2,4-Triazol-3-yl und 1,2,4-Triazol-4-yl, Pyridinyl wie 2-Pyridinyl, 3-Pyridinyl und 4-Pyridinyl, Pyridazinyl wie 3-Pyridazinyl und 4-Pyridazinyl, Pyrimidinyl wie 2-Pyrimidinyl, 4-Pyrimidinyl und 5-Pyrimidinyl, 2-Pyrazinyl, 1,3,5-Triazin-2-yl und 1,2,4-Triazin-3-yl, 1,2-Dihydrofuran-2-yl, 1,2-Dihydrofuran-3-yl, 1,2-Dihydrothiophen-2-yl, 1,2-Dihydrothiophen-3-yl, 2,3-Dihydropyran-4-yl, 2,3-Dihydropyran-5-yl, 2,3-Dihydropyran-6-yl, 5,6-Dihydro-4H-pyran-3-yl, 2,3-Dihydrothiopyran-4-yl, 2,3-Dihydrothiopyran-5-yl, 2,3-Dihydrothiopyran-6-yl, 5,6-Dihydro-4H-thiopyran-3-yl, 5,6-Dihydro-[1,4]dioxin-2-yl, 5,6-Dihydro-[1 ,4]dithiin-2-yl oder 5,6-Dihydro-[1 ,4]oxathiin-3-yl, insbesondere Pyridyl, Thiazolyl und Pyrazolyl.

Im Hinblick auf die fungizide Wirksamkeit der erfindungsgemäßen Verbindungen I sind solche Verbindungen der Formel I bevorzugt, worin A für einen cyclischen Rest A-1 bis A-6: 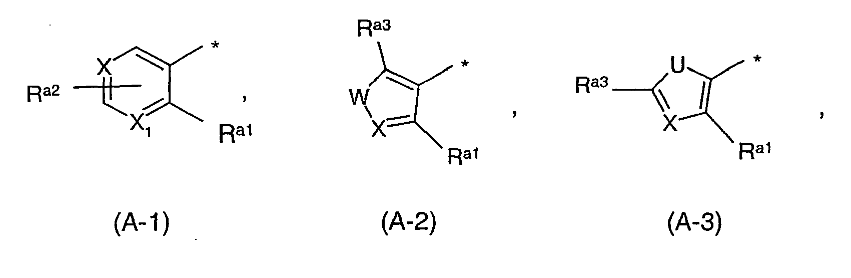 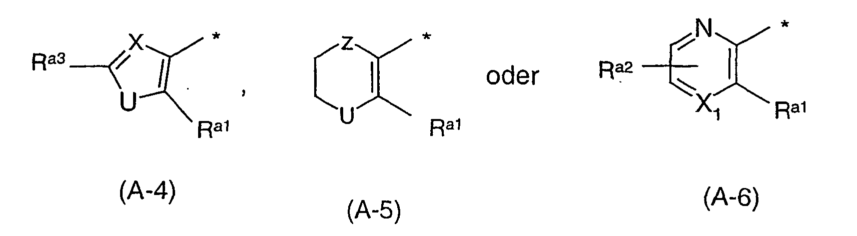 steht, in denen * die Bindungsstelle an C(=Y) angibt und die Variablen die folgende Bedeutung haben:
- X, X₁: jeweils unabhängig voneinander N oder CR^{c}, wobei R^{c} für H steht oder die für R^{b} genannten Bedeutungen aufweist. Insbesondere steht R^{c} für Wasserstoff;
- W: S oder N-R^{a4}, worin R^{a4} für Wasserstoff, C₁-C₄-Alkyl, C₁-C₄-Halogenalkyl, C₁-C₄-Alkoxy, C₁-C₄-Halogenalkyl oder Phenyl steht, das unsubstituiert sein kann oder 1, 2 oder 3 Reste R^{b} tragen kann, wobei R^{a4} insbesondere Wasserstoff, C₁-C₄-Alkyl oder C₁-C₄-Halogenalkyl bedeutet;
- U: Sauerstoff oder Schwefel;
- Z: S, S(=O), S(=O)₂ oder CH₂, besonders bevorzugt S oder CH₂;
- R^{a1}: Wasserstoff, C₁-C₄-Alkyl C₁-C₄-Alkoxy, C₁-C₄-Halogenalkyl, C₁-C₄-Halogenalkoxy oder Halogen, besonders bevorzugt Wasserstoff, Halogen, C₁-C₂-Alkyl, C₁-C₂-Alkoxy, C₁-C₂-Fluoralkoxy oder C₁-C₂-Fluoralkyl;
- R^{a2}: jeweils unabhängig voneinander Wasserstoff, Halogen, Nitro, CN, C₁-C₄-Alkyl, C₃-C₆-Cycloalkyl, C₂-C₄-Alkenyl, C₂-C₄-Alkinyl, C₁-C₄-Alkoxy, wobei die 5 zuletzt genannten Gruppen durch Halogen substituiert sein können; und
- R^{a3}: Wasserstoff, Halogen, Nitro, CN, C₁-C₄-Alkyl, C₃-C₆-Cycloalkyl, C₂-C₄-Alkenyl, C₂-C₄-Alkinyl, C₁-C₄-Alkoxy, wobei die 5 zuletzt genannten Gruppen durch Halogen substituiert sein können, besonders bevorzugt Wasserstoff, Fluor, Chlor oder C₁-C₄-Alkyl.

In den Resten der Formeln A-1, A-2, A-3, A-4, A-5 und A-6 haben die Variablen R^{a1}, R^{a2} und R^{a3} insbesondere die folgenden Bedeutungen:
- R^{a1}: Wasserstoff, Halogen, insbesondere Fluor oder Chlor, C₁-C₄-Alkyl oder C₁-C₄-Halogenalkyl, besonders bevorzugt Halogen, Trifluormethyl oder Methyl;
- R^{a2}: Wasserstoff; und
- R^{a3}: Halogen, insbesondere Fluor oder Chlor, oder Methyl.

In Formel A-2 steht W vorzugsweise für eine Gruppe N-R^{a4}, worin R^{a4} die zuvor genannten Bedeutungen und insbesondere die als bevorzugt angegebenen Bedeutungen aufweist.

Sofern X in den Formeln A-1, A-2, A-3 oder A-4 für eine Gruppe C-R^{c} steht, bedeutet R^{c} vorzugsweise Wasserstoff.

Insbesondere steht X in den Formeln A-2, A-3 und A-4 für N. In Formel A-1 steht X insbesondere für CH.

In den Formeln A-1 und A-6 steht X₁ insbesondere für N.

Beispiele für Reste A-1 sind insbesondere: 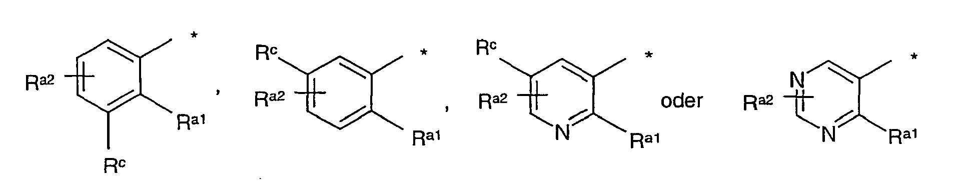 worin *, R^{a1}, R^{a2} und R^{c} die zuvor genannten und insbesondere die bevorzugten Bedeutungen aufweisen.

Beispiele für Reste A-2 sind insbesondere: 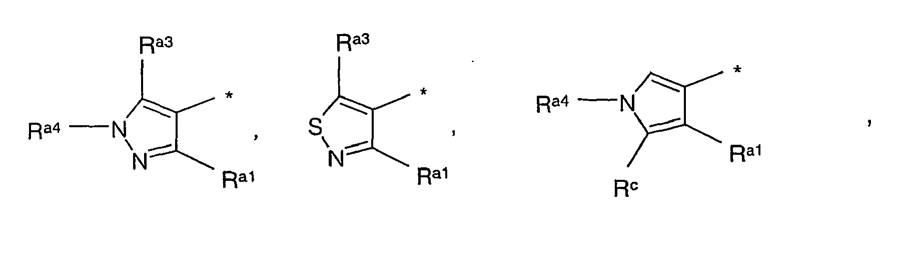 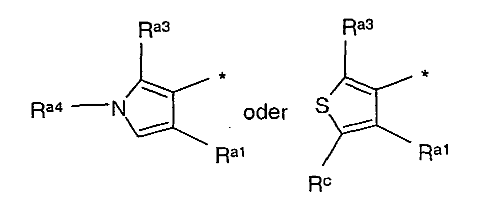 worin *, R^{a1}, R^{a3}, R^{a4} und R^{c} die zuvor genannten und insbesondere die bevorzugten Bedeutungen aufweisen.

Beispiele für Reste A-3 sind insbesondere: 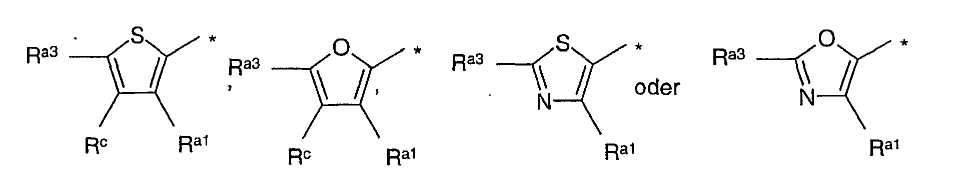 worin *, R^{a1}, R^{a3} und R^{c} die zuvor genannten und insbesondere die bevorzugten Bedeutungen aufweisen.

Beispiele für Reste A-4 sind insbesondere: 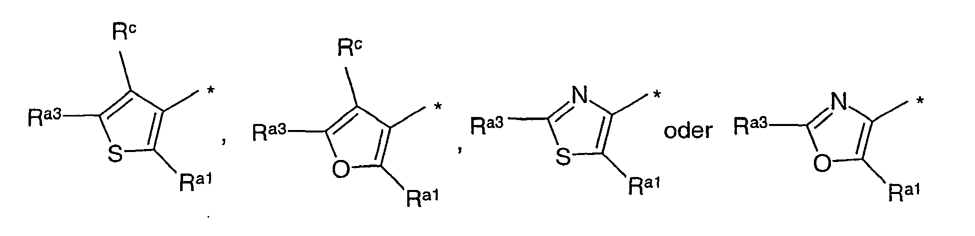 worin *, R^{a1}, R^{a3} und R^{C} die zuvor genannten und insbesondere die bevorzugten Bedeutungen aufweisen.

Beispiele für A-5 sind insbesondere: 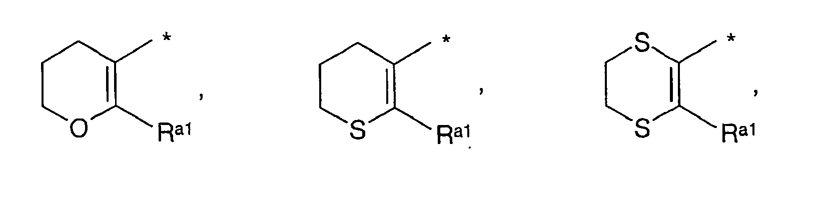 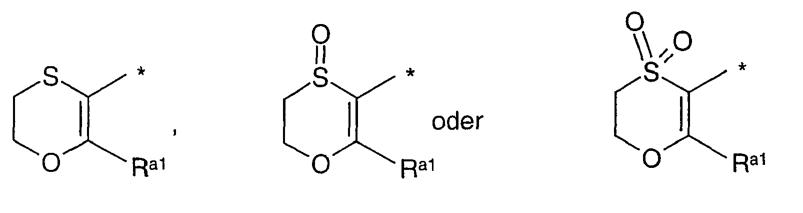 worin * und R^{a1} die zuvor genannten und insbesondere die bevorzugten Bedeutungen aufweisen.

Beispiele für A-6 sind insbesondere: 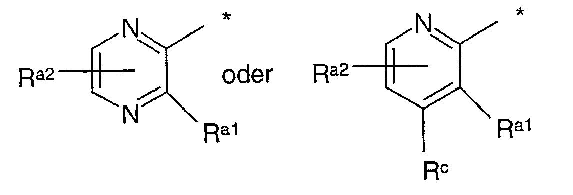 worin *, R^{a1}, R^{a2} und R^{c} die zuvor genannten und insbesondere die bevorzugten Bedeutungen aufweisen.

Beispiele für Reste A sind: 2-Chlorphenyl, 2-Trifluormethyl-phenyl, 2-Difluormethyl-phenyl, 2-Methyl-phenyl, 2-Chlor-pyridin-3-yl, 2-Trifluormethyl-pyridin-3-yl, 2-Difluormethyl-pyridin-3-yl, 2-Methyl-pyridin-3-yl, 4-Methyl-pyrimidin-5-yl, 4-Trifluormethyl-pyrimidin-5-yl, 4-Difluormethyl-pyrimidin-5-yl, 1-Methyl-3-trifluormethyl-pyrazol-4-yl, 1-Methyl-3-difluormethyl-pyrazol-4-yl, 1,3-Dimethyl-pyrazol-4-yl, 1-Methyl-3-trifluormethyl-5-fluor-pyrazol-4-yl, 1-Methyl-3-difluormethyl-5-fluor-pyrazol-4-yl, 1-Methyl-3-trifluormethyl-5-chlor-pyrazol-4-yl, 1-Methyl-3-trifluormethyl-pyrrol-4-yl, 1-Methyl-3-difluormethyl-pyrrol-4-yl, 2-Methyl-4-trifluormethyl-thiazol-5-yl, 2-Methyl-4-difluormethyl-thiazol-5-yl, 2,4-Dimethyl-thiazol-5-yl, 2-Methyl-5-trifluormethyl-thiazol-4-yl, 2-Methyl-5-difluormethyl-thiazol-4-yl, 2,5-Dimethyl-thiazol-4-yl, 2-Methyl-4-trifluormethyl-oxazol-5-yl, 2-Methyl-4-difluormethyl-oxazol-5-yl, 2,4-Dimethyl-oxazol-5-yl, 2-Trifluormethyl-thiophen-3-yl, 5-Methyl-2-trifluormethyl-thiophen-3-yl, 2-Methyl-thiophen-3-yl, 2,5-Dimethyl-thiophen-3-yl, 3-Trifluormethyl-thiophen-2-yl, 3-Methyl-thiophen-2-yl, 3,5-Dimethyl-thiophen-2-yl, 5-Methyl-3-trifluormethyl-thiophen-2-yl, 2-Trifluormethyl-furan-3-yl, 5-Methyl-2-trifluormethyl-furan-3-yl, 2-Methyl-furan-3-yl, 2,5-Dimethyl-furan-3-yl, 2-Methyl-5,6-dihydro-[1,4]oxathiin-3-yl, 2-Methyl-5,6-dihydro-4H-thiopyran-3-yl.

Besonders bevorzugt steht A für einen Rest A-1 a, A-2a oder A-3a, 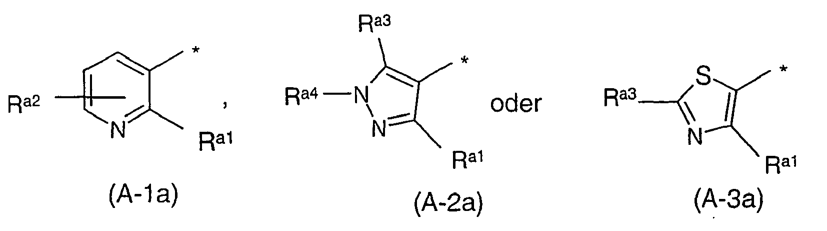 worin *, R^{a1}, R^{a2}, R^{a3} und R^{a4} die zuvor genannten Bedeutungen und insbesondere die bevorzugten Bedeutungen aufweisen.

Bevorzugt sind Reste A-1a mit R^{a1} gleich Wasserstoff, Halogen, C₁-C₂-Alkyl, C₁-C₂-Alkoxy, C₁-C₂-Fluoralkoxy oder C₁-C₂-Fluoralkyl; insbesondere Wasserstoff, Chlor, Brom, Fluor, Methyl, Ethyl, Methoxy, Trifluormethyl, Difluormethyl, Trifluormethoxy oder Difluormethoxy, ganz besonders bevorzugt Fluor, Brom, Chlor, Methyl oder Trifluormethyl, und speziell Chlor; mit R^{a2} gleich Wasserstoff, Halogen, Nitro, CN, C₁-C₄-Alkyl, C₃-C₆-Cycloalkyl, C₂-C₄-Alkenyl, C₂-C₄-Alkinyl, C₁-C₄-Alkoxy, wobei die 5 zuletzt genannten Gruppen durch Halogen substituiert sein können, speziell Wasserstoff.

Bevorzugt sind Reste A-2a mit: R^{a1} gleich Wasserstoff, Halogen, C₁-C₂-Alkyl, C₁-C₂-Alkoxy, C₁-C₂-Fluoralkoxy oder C₁-C₂-Fluoralkyl, insbesondere Wasserstoff, Chlor, Brom, Fluor, Methyl, Ethyl, Methoxy, Trifluormethyl, Difluormethyl, Trifluormethoxy oder Difluormethoxy, ganz besonders bevorzugt Fluor, Brom, Chlor, Methyl oder Trifluormethyl, speziell Trifluormethyl; R^{a3} gleich Wasserstoff, Halogen, Nitro, CN, C₁-C₄-Alkyl, C₃-C₆-Cycloalkyl, C₂-C₄-Alkenyl, C₂-C₄-Alkinyl, C₁-C₄-Alkoxy, wobei die 5 zuletzt genannten Gruppen durch Halogen substituiert sein können, vorzugsweise Wasserstoff, Halogen und C₁-C₄-Alkyl, insbesondere Halogen, Wasserstoff; und speziell Wasserstoff; und R^{a4} gleich Wasserstoff, C₁-C₄-Alkyl, C₁-C₄-Halogenalkyl, C₁-C₄-Alkoxy, C₁-C₄-Halogenalkyl oder Phenyl steht, das unsubstituiert sein kann oder 1, 2 oder 3 Reste R^{b} tragen kann, vorzugsweise Wasserstoff, C₁-C₄-Alkyl oder C₁-C₄-Halogenalkyl, speziell Methyl;

Bevorzugt sind Reste A-3a mit: R^{a1} gleich Wasserstoff, Halogen, C₁-C₂-Alkyl, C₁-C₂-Alkoxy, C₁-C₂-Fluoralkoxy oder C₁-C₂-Fluoralkyl, insbesondere Wasserstoff, Chlor, Brom, Fluor, Methyl, Ethyl, Methoxy, Trifluormethyl, Difluormethyl, Trifluormethoxy oder Difluormethoxy, ganz besonders bevorzugt Fluor, Brom, Chlor, Methyl oder Trifluormethyl, speziell Trifluormethyl; R^{a3} gleich Wasserstoff, Halogen, Nitro, CN, C₁-C₄-Alkyl, C₃-C₆-Cycloalkyl, C₂-C₄-Alkenyl, C₂-C₄-Alkinyl, C₁-C₄-Alkoxy, wobei die 5 zuletzt genannten Gruppen durch Halogen substituiert sein können, vorzugsweise Wasserstoff, Halogen oder C₁-C₄-Alkyl, insbesondere Wasserstoff, Methyl und speziell Methyl.

Besonders bevorzugt ist A ausgewählt unter:
A-1 a mit R^{a1} = Halogen, speziell Chlor und R^{a2} = Wasserstoff;
A-2a mit R^{a1} = C₁-C₂-Fluoralkyl, speziell Trifluormethyl, R^{a3} = Wasserstoff und R^{a4} = C₁-C₄-Alkyl, speziell Methyl; und
A-3a mit R^{a1} = C₁-C₂-Fluoralkyl, speziell Trifluormethyl und R^{a3} = C₁-C₄-Alkyl, speziell Methyl.

Im Hinblick auf ihre fungizide Wirksamkeit sind (Hetero)cyclylcarboxanilide der allgemeinen Formel I bevorzugt, worin die Variablen Y, R¹, R², R^{3m}, R^{4m}, R⁵, R⁶, n und m unabhängig voneinander und vorzugsweise in Kombination die folgenden Bedeutungen aufweisen:
- Y: O;
- R¹: Wasserstoff, OH, C₁-C₄-Alkyl, insbesondere H, OH oder Methyl und speziell H;
- R²: C₁-C₄-Alkyl, C₁-C₄-Alkoxy, C₁-C₄-Halogenalkyl, C₁-C₄-Halogenalkoxy, Nitro, Cyano oder Halogen; besonders bevorzugt C₁-C₄-Alkyl, C₁-C₄-Alkoxy, Nitro, Cyano oder Halogen und speziell Methyl, Methoxy, Fluor, Chlor, Brom, Nitro oder Cyano;
- n: 0 oder 1, besonders bevorzugt 0;
- R^{3m}, R^{4m}: jeweils unabhängig voneinander für Wasserstoff, C₁-C₆-Alkyl, C₁-C₆-Halogenalkyl, C₃-C₆-Cycloalkyl oder C₃-C₆-Halogencycloalkyl, Phenyl, das unsubstituiert sein kann oder ein, zwei oder drei Reste R^{b} tragen kann; vorzugsweise Wasserstoff oder C₁-C₄-Alkyl; speziell: R³¹ und R⁴¹ jeweils unabhängig voneinander Wasserstoff, Methyl, Ethyl;
- m: 1;
- R⁵: Wasserstoff, C₁-C₆-Alkyl, C₁-C₆-Halogenalkyl, C₃-C₆-Cycloalkyl, C₃-C₆-Halogencycloalkyl, Phenyl, Phenyl-C₁-C₄-alkyl, Phenyl-C₁-C₄-halogenalkyl, wobei Phenyl in den drei zuletzt genannten Resten unsubstituiert sein kann oder ein, zwei oder drei Reste R^{b} tragen kann; vorzugsweise Wasserstoff, C₁-C₄-Alkyl, C₁-C₄-Halogenalkyl, Phenyl, das unsubstituiert sein kann oder ein, zwei oder drei Reste R^{b} tragen kann;
- R⁶: C₁-C₆-Alkyl, C₁-C₆-Halogenalkyl, C₃-C₆-Cycloalkyl, C₃-C₆-Halogencycloalkyl, C₂-C₆-Alkenyl, C₂-C₆-Halogenalkenyl, C₂-C₄-Alkinyl, C₂-C₄-Halogenalkinyl, Phenyl-C₁-C₂-alkyl oder Phenyl, wobei Phenyl in den zwei zuletzt genannten Resten unsubstituiert sein kann oder ein oder zwei Halogengruppen, speziell Fluor oder Chlor tragen kann.

Besonders bevorzugt sind außerdem die (Heterocyclyl)carboxanilide der allgemeinen Formel I, worin R¹, R², R^{3m}, R^{4m}, R⁵, R⁶, n und m die zuvor genannten und insbesondere die bevorzugten Bedeutungen aufweisen, Y für Sauerstoff steht und A ausgewählt ist unter:
A-1, worin X und X₁ jeweils für Stickstoff stehen, R^{a1} die zuvor genannten, insbesondere die bevorzugten Bedeutungen aufweist und speziell für Methyl, Trifluormethyl, Chlor, Brom oder Fluor steht; R^{a2} die zuvor genannten Bedeutungen hat und speziell für Wasserstoff steht;
A-2, worin X für N steht, W für S steht, R^{a1} die zuvor genannten, insbesondere die bevorzugten Bedeutungen aufweist und speziell für Methyl, Fluor, Chlor, Brom oder Trifluormethyl steht; R^{a3} die zuvor genannten, insbesondere die bevorzugten Bedeutungen aufweist und speziell für Wasserstoff steht;
A-2, worin X für CH steht, W für N-R^{a4} steht mit R^{a4} gleich C₁-C₄-Alkyl, speziell Methyl, R^{a1} die zuvor genannten, insbesondere die bevorzugten Bedeutungen aufweist und speziell für Methyl, Fluor, Chlor, Brom oder Trifluormethyl steht; R^{a3} die zuvor genannten, insbesondere die bevorzugten Bedeutungen aufweist und speziell für Wasserstoff steht;
A-3, worin U für O steht, X für N steht, R^{a1} die zuvor genannten, insbesondere die bevorzugten Bedeutungen aufweist und speziell für Methyl, Fluor, Chlor, Brom oder Trifluormethyl steht; R^{a3} die zuvor genannten, insbesondere die bevorzugten Bedeutungen aufweist und speziell für Wasserstoff oder Methyl steht;
A-3, worin U für S steht, X für CH steht, R^{a1} die zuvor genannten, insbesondere die bevorzugten Bedeutungen aufweist und speziell für Methyl, Fluor, Chlor, Brom oder Trifluormethyl steht; R^{a3} die zuvor genannten, insbesondere die bevorzugten Bedeutungen aufweist und speziell für Wasserstoff oder Methyl steht;
A-4, worin U für O steht, X für CH oder N steht, R^{a1} die zuvor genannten, insbesondere die bevorzugten Bedeutungen aufweist und speziell für Methyl, Fluor, Chlor, Brom oder Trifluormethyl steht; R^{a3} die zuvor genannten, insbesondere die bevorzugten Bedeutungen aufweist und speziell für Wasserstoff oder Methyl steht;
A-4, worin U für S steht, X für CH oder N steht, R^{a1} die zuvor genannten, insbesondere die bevorzugten Bedeutungen aufweist und speziell für Methyl, Fluor, Chlor, Brom oder Trifluormethyl steht; R^{a3} die zuvor genannten, insbesondere die bevorzugten Bedeutungen aufweist und speziell für Wasserstoff oder Methyl steht;
A-5, worin U für Sauerstoff, Z für CH₂, S, S(=O) oder S(=O)₂ steht und R^{a1} die zuvor genannten, insbesondere die bevorzugten Bedeutungen aufweist und speziell für Methyl, Fluor, Chlor, Brom oder Trifluormethyl steht;
A-6, worin X₁ für Stickstoff steht, R^{a2} die zuvor genannten, Bedeutungen aufweist und speziell für Wasserstoff steht; R^{a1} die zuvor genannten, insbesondere die bevorzugten Bedeutungen aufweist und speziell für Methyl, Fluor, Chlor, Brom oder Trifluormethyl steht.

Insbesondere sind im Hinblick auf ihre Verwendung als Fungizide und Wirkstoffe zur Bekämpfung von Schädlingen die in den folgenden Tabellen 1 bis 42 zusammengestellten Einzelverbindungen der Formel IA (Verbindungen I mit R¹ = H, n = 0) bevorzugt, in denen die Variablen R⁵, R⁶, R^{3m}, R^{4m} und m jeweils die in einer Zeile der Tabelle A angegebenen Bedeutungen aufweisen und die Variable A die in der jeweiligen Tabelle angegebene Bedeutung aufweist. Bei Verbindungen, die Doppelbindungen enthalten, sind sowohl die isomerenreinen E-lsomeren, Z-lsomeren wie auch lsomerengemische davon umfasst.

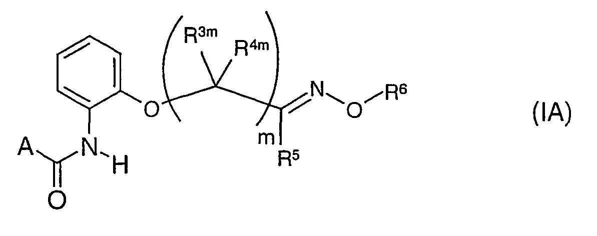

**Tabelle A:**

| Nr. | R⁵ | R⁶ | (C(R^{3m})(R^{4m}))ₘ |
|---|---|---|---|
| 1 | H | CH₃ | -CH₂- |
| 2 | H | C₂H₅ | -CH₂- |
| 3 | H | CH₂CH₂CH₃ | -CH₂- |
| 4 | H | CH(CH₃)₂ | -CH₂₋ |
| 5 | H | CH₂CH₂CH₂CH₃ | -CH₂- |
| 6 | H | i-C₄H₉ | -CH₂- |
| 7 | H | S-C₄H₉ | -CH₂- |
| 8 | H | C(CH₃)₃ | -CH₂- |
| 9 | H | CH₂CH₂CH₂CH₂CH₃ | -CH₂- |
| 10 | H | CH₂CH₂CH₂CH₂CH₂CH₃ | -CH₂- |
| 11 | H | Cyclopentyl | -CH₂- |
| 12 | H | Cyclohexyl | -CH₂- |
| 13 | H | Allyl | -CH₂- |
| 14 | H | But-2-en-1-yl | -CH₂- |
| 15 | H | 4-Chlor-but-2-en-1-yl | -CH₂- |
| 16 | H | Propargyl | -CH₂- |
| 17 | H | C₆H₅ | -CH₂- |
| 18 | H | C₆H₅CH₂ | -CH₂- |
| 19 | H | 2-Phenyleth-1-yl | -CH₂- |
| 20 | H | 4-Cl-C₆H₄ | -CH₂- |
| 21 | H | 4-F-C₆H₄ | -CH₂- |
| 22 | H | CH₃ | -CH(CH₃)- |
| 23 | H | C₂H₅ | -CH(CH₃)- |
| 24 | H | CH₂CH₂CH₃ | -CH(CH₃)- |
| 25 | H | CH(CH₃)₂ | -CH(CH₃)- |
| 26 | H | CH₂CH₂CH₂CH₃ | -CH(CH₃)- |
| 27 | H | i-C₄H₉ | -CH(CH₃)- |
| 28 | H | s-C₄H₉ | -CH(CH₃)- |
| 29 | H | C(CH₃)₃ | -CH(CH₃)- |
| 30 | H | CH₂CH₂CH₂CH₂CH₃ | -CH(CH₃)- |
| 31 | H | CH₂CH₂CH₂CH₂CH₂CH₃ | -CH(CH₃)- |
| 32 | H | Cyclopentyl | -CH(CH₃)- |
| 33 | H | Cyclohexyl | -CH(CH₃)- |
| 34 | H | Allyl | -CH(CH₃)- |
| 35 | H | But-2-en-1-yl | -CH(CH₃)- |
| 36 | H | 4-Chlor-but-2-en-1-yl | -CH(CH₃)- |
| 37 | H | Propargyl | -CH(CH₃)- |
| 38 | H | C₆H₅ | -CH(CH₃)- |
| 39 | H | C₆H₅CH₂ | -CH(CH₃)- |
| 40 | H | 2-Phenyleth-1-yl | -CH(CH₃)- |
| 41 | H | 4-Cl-C₆H₄ | -CH(CH₃)- |
| 42 | H | 4-F-C₆H₄ | -CH(CH₃)- |
| 43 | H | CH₃ | -CH(C₂H₅)- |
| 44 | H | C₂H₅ | -CH(C₂H₅)- |
| 45 | H | CH₂CH₂CH₃ | -CH(C₂H₅)- |
| 46 | H | CH(CH₃)₂ | -CH(C₂H₅)- |
| 47 | H | CH₂CH₂CH₂CH₃ | -CH(C₂H₅)- |
| 48 | H | i-C₄H₉ | -CH(C₂H₅)- |
| 49 | H | s-C₄H₉ | -CH(C₂H₅)- |
| 50 | H | C(CH₃)₃ | -CH(C₂H₅)- |
| 51 | H | CH₂CH₂CH₂CH₂CH₃ | -CH(C₂H₅)- |
| 52 | H | CH₂CH₂CH₂CH₂CH₂CH₃ | -CH(C₂H₅)- |
| 53 | H | Cyclopentyl | -CH(C₂H₅)- |
| 54 | H | Cyclohexyl | -CH(C₂H₅)- |
| 55 | H | Allyl | -CH(C₂H₅)- |
| 56 | H | But-2-en-1-yl | -CH(C₂H₅)- |
| 57 | H | 4-Chlor-but-2-en-1-yl | -CH(C₂H₅)- |
| 58 | H | Propargyl | -CH(C₂H₅)- |
| 59 | H | C₆H₅ | -CH(C₂H₅)- |
| 60 | H | C₆H₅CH₂ | -CH(C₂H₅)- |
| 61 | H | 2-Phenyleth-1-yl | -CH(C₂H₅)- |
| 62 | H | 4-Cl-C₆H₄ | -CH(C₂H₅)- |
| 63 | H | 4-F-C₆H₄ | -CH(C₂H₅)- |
| 64 | H | CH₃ | -C(CH₃)₂- |
| 65 | H | C₂H₅ | -C(CH₃)₂- |
| 66 | H | CH₂CH₂CH₃ | -C(CH₃)₂- |
| 67 | H | CH(CH₃)₂ | -C(CH₃)₂- |
| 68 | H | CH₂CH₂CH₂CH₃ | -C(CH₃)₂- |
| 69 | H | i-C₄H₉ | -C(CH₃)₂- |
| 70 | H | s-C₄H₉ | -C(CH₃)₂- |
| 71 | H | C(CH₃)₃ | -C(CH₃)₂- |
| 72 | H | CH₂CH₂CH₂CH₂CH₃ | -C(CH₃)₂- |
| 73 | H | CH₂CH₂CH₂CH₂CH₂CH₃ | -C(CH₃)₂- |
| 74 | H | Cyclopentyl | -C(CH₃)₂- |
| 75 | H | Cyclohexyl | -C(CH₃)₂- |
| 76 | H | Allyl | -C(CH₃)₂- |
| 77 | H | But-2-en-1-yl | -C(CH₃)₂- |
| 78 | H | 4-Chlor-but-2-en-1-yl | -C(CH₃)₂- |
| 79 | H | Propargyl | -C(CH₃)₂- |
| 80 | H | C₆H₅ | -C(CH₃)₂- |
| 81 | H | C₆H₅CH₂ | -C(CH₃)₂- |
| 82 | H | 2-Phenyleth-1-yl | -C(CH₃)₂- |
| 83 | H | 4-Cl-C₆H₄ | -C(CH₃)₂- |
| 84 | H | 4-F-C₆H₄ | -C(CH₃)₂- |
| 85 | H | CH₃ | -CH₂CH₂- |
| 86 | H | C₂H₅ | -CH₂CH₂- |
| 87 | H | CH₂CH₂CH₃ | -CH₂CH₂- |
| 88 | H | CH(CH₃)₂ | -CH₂CH₂- |
| 89 | H | CH₂CH₂CH₂CH₃ | -CH₂CH₂- |
| 90 | H | i-C₄H₉ | -CH₂CH₂- |
| 91 | H | s-C₄H₉ | -CH₂CH₂- |
| 92 | H | C(CH₃)₃ | -CH₂CH₂- |
| 93 | H | CH₂CH₂CH₂CH₂CH₃ | -CH₂CH₂- |
| 94 | H | CH₂CH₂CH₂CH₂CH₂CH₃ | -CH₂CH₂- |
| 95 | H | Cyclopentyl | -CH₂CH₂- |
| 96 | H | Cyclohexyl | -CH₂CH₂- |
| 97 | H | Allyl | -CH₂CH₂- |
| 98 | H | But-2-en-1-yl | -CH₂CH₂- |
| 99 | H | 4-Chlor-but-2-en-1-yl | -CH₂CH₂- |
| 100 | H | Propargyl | -CH₂CH₂- |
| 101 | H | C₆H₅ | -CH₂CH₂- |
| 102 | H | C₆H₅CH₂ | -CH₂CH₂- |
| 103 | H | 2-Phenyleth-1-yl | -CH₂CH₂- |
| 104 | H | 4-Cl-C₆H₄ | -CH₂CH₂- |
| 105 | H | 4-F-C₆H₄ | -CH₂CH₂- |
| 106 | H | CH₃ | -CH(CH₃)CH₂- |
| 107 | H | C₂H₅ | -CH(CH₃)CH₂- |
| 108 | H | CH₂CH₂CH₃ | -CH(CH₃)CH₂- |
| 109 | H | CH(CH₃)₂ | -CH(CH₃)CH₂- |
| 110 | H | CH₂CH₂CH₂CH₃ | -CH(CH₃)CH₂- |
| 111 | H | i-C₄H₉ | -CH(CH₃)CH₂- |
| 112 | H | S-C₄H₉ | -CH(CH₃)CH₂- |
| 113 | H | C(CH₃)₃ | -CH(CH₃)CH₂- |
| 114 | H | CH₂CH₂CH₂CH₂CH₃ | -CH(CH₃)CH₂- |
| 115 | H | CH₂CH₂CH₂CH₂CH₂CH₃ | -CH(CH₃)CH₂- |
| 116 | H | Cyclopentyl | -CH(CH₃)CH₂- |
| 117 | H | Cyclohexyl | -CH(CH₃)CH₂- |
| 118 | H | Allyl | -CH(CH₃)CH₂- |
| 119 | H | But-2-en-1-yl | -CH(CH₃)CH₂- |
| 120 | H | 4-Chlor-but-2-en-1-yl | -CH(CH₃)CH₂- |
| 121 | H | Propargyl | -CH(CH₃)CH₂- |
| 122 | H | C₆H₅ | -CH(CH₃)CH₂- |
| 123 | H | C₆H₅CH₂ | -CH(CH₃)CH₂- |
| 124 | H | 2-Phenyleth-1-yl | -CH(CH₃)CH₂- |
| 125 | H | 4-Cl-C₆H₄ | -CH(CH₃)CH₂- |
| 126 | H | 4-F-C₆H₄ | -CH(CH₃)CH₂- |
| 127 | H | CH₃ | -CH₂CH(CH₃)- |
| 128 | H | C₂H₅ | -CH₂CH(CH₃)- |
| 129 | H | CH₂CH₂CH₃ | -CH₂CH(CH₃)- |
| 130 | H | CH(CH₃)₂ | -CH₂CH(CH₃)- |
| 131 | H | CH₂CH₂CH₂CH₃ | -CH₂CH(CH₃)- |
| 132 | H | i-C₄H₉ | -CH₂CH(CH₃)- |
| 133 | H | s-C₄H₉ | -CH₂CH(CH₃)- |
| 134 | H | C(CH₃)₃ | -CH₂CH(CH₃)- |
| 135 | H | CH₂CH₂CH₂CH₂CH₃ | -CH₂CH(CH₃)- |
| 136 | H | CH₂CH₂CH₂CH₂CH₂CH₃ | -CH₂CH(CH₃)- |
| 137 | H | Cyclopentyl | -CH₂CH(CH₃)- |
| 138 | H | Cyclohexyl | -CH₂CH(CH₃)- |
| 139 | H | Allyl | -CH₂CH(CH₃)- |
| 140 | H | But-2-en-1-yl | -CH₂CH(CH₃)- |
| 141 | H | 4-Chlor-but-2-en-1-yl | -CH₂CH(CH₃)- |
| 142 | H | Propargyl | -CH₂CH(CH₃)- |
| 143 | H | C₆H₅ | -CH₂CH(CH₃)- |
| 144 | H | C₆H₅CH₂ | -CH₂CH(CH₃)- |
| 145 | H | 2-Phenyleth-1-yl | -CH₂CH(CH₃)- |
| 146 | H | 4-Cl-C₆H₄ | -CH₂CH(CH₃)- |
| 147 | H | 4-F-C₆H₄ | -CH₂CH(CH₃)- |
| 148 | H | CH₃ | -CH(CH₃)CH(CH₃)- |
| 149 | H | C₂H₅ | -CH(CH₃)CH(CH₃)- |
| 150 | H | CH₂CH₂CH₃ | -CH(CH₃)CH(CH₃)- |
| 151 | H | CH(CH₃)₂ | -CH(CH₃)CH(CH₃)- |
| 152 | H | CH₂CH₂CH₂CH₃ | -CH(CH₃)CH(CH₃)- |
| 153 | H | i-C₄H₉ | -CH(CH₃)CH(CH₃)- |
| 154 | H | S-C₄H₉ | -CH(CH₃)CH(CH₃)- |
| 155 | H | C(CH₃)₃ | -CH(CH₃)CH(CH₃)- |
| 156 | H | CH₂CH₂CH₂CH₂CH₃ | -CH(CH₃)CH(CH₃)- |
| 157 | H | CH₂CH₂CH₂CH₂CH₂CH₃ | -CH(CH₃)CH(CH₃)- |
| 158 | H | Cyclopentyl | -CH(CH₃)CH(CH₃)- |
| 159 | H | Cyclohexyl | -CH(CH₃)CH(CH₃)- |
| 160 | H | Allyl | -CH(CH₃)CH(CH₃)- |
| 161 | H | But-2-en-1-yl | -CH(CH₃)CH(CH₃)- |
| 162 | H | 4-Chlor-but-2-en-1-yl | -CH(CH₃)CH(CH₃)- |
| 163 | H | Propargyl | -CH(CH₃)CH(CH₃)- |
| 164 | H | C₆H₅ | -CH(CH₃)CH(CH₃)- |
| 165 | H | C₆H₅CH₂ | -CH(CH₃)CH(CH₃)- |
| 166 | H | 2-Phenyleth-1-yl | -CH(CH₃)CH(CH₃)- |
| 167 | H | 4-Cl-C₆H₄ | -CH(CH₃)CH(CH₃)- |
| 168 | H | 4-F-C₆H₄ | -CH(CH₃)CH(CH₃)- |
| 169 | H | CH₃ | -CH₂CH₂CH₂- |
| 170 | H | C₂H₅ | -CH₂CH₂CH₂- |
| 171 | H | CH₂CH₂CH₃ | -CH₂CH₂CH₂- |
| 172 | H | CH(CH₃)₂ | -CH₂CH₂CH₂- |
| 173 | H | CH₂CH₂CH₂CH₃ | -CH₂CH₂CH₂- |
| 174 | H | i-C₄H₉ | -CH₂CH₂CH₂- |
| 175 | H | s-C₄H₉ | -CH₂CH₂CH₂- |
| 176 | H | C(CHₐ)₃ | -CH₂CH₂CH₂- |
| 177 | H | CH₂CH₂CH₂CH₂CH₃ | -CH₂CH₂CH₂- |
| 178 | H | CH₂CH₂CH₂CH₂CH₂CH₃ | -CH₂CH₂CH₂- |
| 179 | H | Cyclopentyl | -CH₂CH₂CH₂- |
| 180 | H | Cyclohexyl | -CH₂CH₂CH₂- |
| 181 | H | Allyl | -CH₂CH₂CH₂- |
| 182 | H | But-2-en-1-yl | -CH₂CH₂CH₂- |
| 183 | H | 4-Chlor-but-2-en-1-yl | -CH₂CH₂CH₂- |
| 184 | H | Propargyl | -CH₂CH₂CH₂- |
| 185 | H | C₆H₅ | -CH₂CH₂CH₂- |
| 186 | H | C₆H₅CH₂ | -CH₂CH₂CH₂- |
| 187 | H | 2-Phenyleth-1-yl | -CH₂CH₂CH₂- |
| 188 | H | 4-Cl-C₆H₄ | -CH₂CH₂CH₂- |
| 189 | H | 4-F-C₆H₄ | -CH₂CH₂CH₂- |
| 190 | CH₃ | CH₃ | -CH₂- |
| 191 | CH₃ | C₂H₅ | -CH₂- |
| 192 | CH₃ | CH₂CH₂CH₃ | -CH₂- |
| 193 | CH₃ | CH(CH₃)₂ | -CH₂- |
| 194 | CH₃ | CH₂CH₂CH₂CH₃ | -CH₂- |
| 195 | CH₃ | i-C₄H₉ | -CH₂- |
| 196 | CH₃ | s-C₄H₉ | -CH₂- |
| 197 | CH₃ | C(CH₃)₃ | -CH₂- |
| 198 | CH₃ | CH₂CH₂CH₂CH₂CH₃ | -CH₂- |
| 199 | CH₃ | CH₂CH₂CH₂CH₂CH₂CH₃ | -CH₂- |
| 200 | CH₃ | Cyclopentyl | -CH₂- |
| 201 | CH₃ | Cyclohexyl | -CH₂- |
| 202 | CH₃ | Allyl | -CH₂- |
| 203 | CH₃ | But-2-en-1-yl | -CH₂- |
| 204 | CH₃ | 4-Chlor-but-2-en-1-yl | -CH₂- |
| 205 | CH₃ | Propargyl | -CH₂- |
| 206 | CH₃ | C₆H₅ | -CH₂- |
| 207 | CH₃ | C₆H₅CH₂ | -CH₂- |
| 208 | CH₃ | 2-Phenyleth-1-yl | -CH₂- |
| 209 | CH₃ | 4-CI-C₆H₄ | -CH₂- |
| 210 | CH₃ | 4-F-C₆H₄ | -CH₂- |
| 211 | CH₃ | CH₃ | -CH(CH₃)- |
| 212 | CH₃ | C₂H₅ | -CH(CH₃)- |
| 213 | CH₃ | CH₂CH₂CH₃ | -CH(CH₃)- |
| 214 | CH₃ | CH(CH₃)₂ | -CH(CH₃)- |
| 215 | CH₃ | CH₂CH₂CH₂CH₃ | -CH(CH₃)- |
| 216 | CH₃ | i-C₄H₉ | -CH(CH₃)- |
| 217 | CH₃ | s-C₄H₉ | -CH(CH₃)- |
| 218 | CH₃ | C(CH₃)₃ | -CH(CH₃)- |
| 219 | CH₃ | CH₂CH₂CH₂CH₂CH₃ | -CH(CH₃)- |
| 220 | CH₃ | CH₂CH₂CH₂CH₂CH₂CH₃ | -CH(CH₃)- |
| 221 | CH₃ | Cyclopentyl | -CH(CH₃)- |
| 222 | CH₃ | Cyclohexyl | -CH(CH₃)- |
| 223 | CH₃ | Allyl | -CH(CH₃)- |
| 224 | CH₃ | But-2-en-1-yl | -CH(CH₃)- |
| 225 | CH₃ | 4-Chlor-but-2-en-1-yl | -CH(CH₃)- |
| 226 | CH₃ | Propargyl | -CH(CH₃)- |
| 227 | CH₃ | C₆H₅ | -CH(CH₃)- |
| 228 | CH₃ | C₆H₅CH₂ | -CH(CH₃)- |
| 229 | CH₃ | 2-Phenyleth-1-yl | -CH(CH₃)- |
| 230 | CH₃ | 4-Cl-C₆H₄ | -CH(CH₃)- |
| 231 | CH₃ | 4-F-C₆H₄ | -CH(CH₃)- |
| 232 | CH₃ | CH₃ | -CH(C₂H₅)- |
| 233 | CH₃ | C₂H₅ | -CH(C₂H₅)- |
| 234 | CH₃ | CH₂CH₂CH₃ | -CH(C₂H₅)- |
| 235 | CH₃ | CH(CH₃)₂ | -CH(C₂H₅)- |
| 236 | CH₃ | CH₂CH₂CH₂CH₃ | -CH(C₂H₅)- |
| 237 | CH₃ | i-C₄H₉ | -CH(C₂H₅)- |
| 238 | CH₃ | s-C₄H₉ | -CH(C₂H₅)- |
| 239 | CH₃ | C(CH₃)₃ | -CH(C₂H₅)- |
| 240 | CH₃ | CH₂CH₂CH₂CH₂CH₃ | -CH(C₂H₅)- |
| 241 | CH₃ | CH₂CH₂CH₂CH₂CH₂CH₃ | -CH(C₂H₅)- |
| 242 | CH₃ | Cyclopentyl | -CH(C₂H₅)- |
| 243 | CH₃ | Cyclohexyl | -CH(C₂H₅)- |
| 244 | CH₃ | Allyl | -CH(C₂H₅)- |
| 245 | CH₃ | But-2-en-1-yl | -CH(C₂H₅)- |
| 246 | CH₃ | 4-Chlor-but-2-en-1-yl | -CH(C₂H₅)- |
| 247 | CH₃ | Propargyl | -CH(C₂H₅)- |
| 248 | CH₃ | C₆H₅ | -CH(C₂H₅)- |
| 249 | CH₃ | C₆HsCH₂ | -CH(C₂H₅)- |
| 250 | CH₃ | 2-Phenyleth-1-yl | -CH(C₂H₅)- |
| 251 | CH₃ | 4-Cl-C₆H₄ | -CH(C₂H₅)- |
| 252 | CH₃ | 4-F-C₆H₄ | -CH(C₂H₅)- |
| 253 | CH₃ | CH₃ | -C(CH₃)₂- |
| 254 | CH₃ | C₂H₅ | -C(CH₃)₂- |
| 255 | CH₃ | CH₂CH₂CH₃ | -C(CH₃)₂- |
| 256 | CH₃ | CH(CH₃)₂ | -C(CH₃)₂- |
| 257 | CH₃ | CH₂CH₂CH₂CH₃ | -C(CH₃)₂- |
| 258 | CH₃ | i-C₄H₉ | -C(CH₃)₂- |
| 259 | CH₃ | s-C₄H₉ | -C(CH₃)₂- |
| 260 | CH₃ | C(CH₃)₃ | -C(CH₃)₂- |
| 261 | CH₃ | CH₂CH₂CH₂CH₂CH₃ | -C(CH₃)₂- |
| 262 | CH₃ | CH₂CH₂CH₂CH₂CH₂CH₃ | -C(CH₃)₂- |
| 263 | CH₃ | Cyclopentyl | -C(CH₃)₂- |
| 264 | CH₃ | Cyclohexyl | -C(CH₃)₂- |
| 265 | CH₃ | Allyl | -C(CH₃)₂- |
| 266 | CH₃ | But-2-en-1-yl | -C(CH₃)₂- |
| 267 | CH₃ | 4-Chlor-but-2-en-1-yl | -C(CH₃)₂- |
| 268 | CH₃ | Propargyl | -C(CH₃)₂- |
| 269 | CH₃ | C₆H₅ | -C(CH₃)₂- |
| 270 | CH₃ | C₆H₅CH₂ | -C(CH₃)₂- |
| 271 | CH₃ | 2-Phenyleth-1-yl | -C(CH₃)₂- |
| 272 | CH₃ | 4-Cl-C₆H₄ | -C(CH₃)₂- |
| 273 | CH₃ | 4-F-C₆H₄ | -C(CH₃)₂- |
| 274 | CH₃ | CH₃ | -CH₂CH₂- |
| 275 | CH₃ | C₂H₅ | -CH₂CH₂- |
| 276 | CH₃ | CH₂CH₂CH₃ | -CH₂CH₂- |
| 277 | CH₃ | CH(CH₃)₂ | -CH₂CH₂- |
| 278 | CH₃ | CH₂CH₂CH₂CH₃ | -CH₂CH₂- |
| 279 | CH₃ | i-C₄H₉ | -CH₂CH₂- |
| 280 | CH₃ | s-C₄H₉ | -CH₂CH₂- |
| 281 | CH₃ | C(CH₃)₃ | -CH₂CH₂- |
| 282 | CH₃ | CH₂CH₂CH₂CH₂CH₃ | -CH₂CH₂- |
| 283 | CH₃ | CH₂CH₂CH₂CH₂CH₂CH₃ | -CH₂CH₂- |
| 284 | CH₃ | Cyclopentyl | -CH₂CH₂- |
| 285 | CH₃ | Cyclohexyl | -CH₂CH₂- |
| 286 | CH₃ | Allyl | -CH₂CH₂- |
| 287 | CH₃ | But-2-en-1-yl | -CH₂CH₂- |
| 288 | CH₃ | 4-Chlor-but-2-en-1-yl | -CH₂CH₂- |
| 289 | CH₃ | Propargyl | -CH₂CH₂- |
| 290 | CH₃ | C₆H₅ | -CH₂CH₂- |
| 291 | CH₃ | C₆H₅CH₂ | -CH₂CH₂- |
| 292 | CH₃ | 2-Phenyleth-1-yl | -CH₂CH₂- |
| 293 | CH₃ | 4-Cl-C₆H₄ | -CH₂CH₂- |
| 294 | CH₃ | 4-F-C₆H₄ | -CH₂CH₂- |
| 295 | CH₃ | CH₃ | -CH(CH₃)CH₂- |
| 296 | CH₃ | C₂H₅ | -CH(CH₃)CH₂- |
| 297 | CH₃ | CH₂CH₂CH₃ | -CH(CH₃)CH₂- |
| 298 | CH₃ | CH(CH₃)₂ | -CH(CH₃)CH₂- |
| 299 | CH₃ | CH₂CH₂CH₂CH₃ | -CH(CH₃)CH₂- |
| 300 | CH₃ | i-C₄H₉ | -CH(CH₃)CH₂- |
| 301 | CH₃ | s-C₄H₉ | -CH(CH₃)CH₂- |
| 302 | CH₃ | C(CH₃)₃ | -CH(CH₃)CH₂- |
| 303 | CH₃ | CH₂CH₂CH₂CH₂CH₃ | -CH(CH₃)CH₂- |
| 304 | CH₃ | CH₂CH₂CH₂CH₂CH₂CH₃ | -CH(CH₃)CH₂- |
| 305 | CH₃ | Cyclopentyl | -CH(CH₃)CH₂- |
| 306 | CH₃ | Cyclohexyl | -CH(CH₃)CH₂- |
| 307 | CH₃ | Allyl | -CH(CH₃)CH₂- |
| 308 | CH₃ | But-2-en-1-yl | -CH(CH₃)CH₂- |
| 309 | CH₃ | 4-Chlor-but-2-en-1-yl | -CH(CH₃)CH₂- |
| 310 | CH₃ | Propargyl | -CH(CH₃)CH₂- |
| 311 | CH₃ | C₆H₅ | -CH(CH₃)CH₂- |
| 312 | CH₃ | C₆H₅CH₂ | -CH(CH₃)CH₂- |
| 313 | CH₃ | 2-Phenyleth-1-yl | -CH(CH₃)CH₂- |
| 314 | CH₃ | 4-Cl-C₆H₄ | -CH(CH₃)CH₂- |
| 315 | CH₃ | 4-F-C₆H₄ | -CH(CH₃)CH₂- |
| 316 | CH₃ | CH₃ | -CH₂CH(CH₃)- |
| 317 | CH₃ | C₂H₅ | -CH₂CH(CH₃)- |
| 318 | CH₃ | CH₂CH₂CH₃ | -CH₂CH(CH₃)- |
| 319 | CH₃ | CH(CH₃)₂ | -CH₂CH(CH₃)- |
| 320 | CH₃ | CH₂CH₂CH₂CH₃ | -CH₂CH(CH₃)- |
| 321 | CH₃ | i-C₄H₉ | -CH₂CH(CH₃)- |
| 322 | CH₃ | s-C₄H₉ | -CH₂CH(CH₃)- |
| 323 | CH₃ | C(CH₃)₃ | -CH₂CH(CH₃)- |
| 324 | CH₃ | CH₂CH₂CH₂CH₂CH₃ | -CH₂CH(CH₃)- |
| 325 | CH₃ | CH₂CH₂CH₂CH₂CH₂CH₃ | -CH₂CH(CH₃)- |
| 326 | CH₃ | Cyclopentyl | -CH₂CH(CH₃)- |
| 327 | CH₃ | Cyclohexyl | -CH₂CH(CH₃)- |
| 328 | CH₃ | Allyl | -CH₂CH(CH₃)- |
| 329 | CH₃ | But-2-en-1-yl | -CH₂CH(CH₃)- |
| 330 | CH₃ | 4-Chlor-but-2-en-1-yl | -CH₂CH(CH₃)- |
| 331 | CH₃ | Propargyl | -CH₂CH(CH₃)- |
| 332 | CH₃ | C₆H₅ | -CH₂CH(CH₃)- |
| 333 | CH₃ | C₆H₅CH₂ | -CH₂CH(CH₃)- |
| 334 | CH₃ | 2-Phenyleth-1-yl | -CH₂CH(CH₃)- |
| 335 | CH₃ | 4-CI-C₆H₄ | -CH₂CH(CH₃)- |
| 336 | CH₃ | 4-F-C₆H₄ | -CH₂CH(CH₃)- |
| 337 | CH₃ | CH₃ | -CH(CH₃)CH(CH₃)- |
| 338 | CH₃ | C₂H₅ | -CH(CH₃)CH(CH₃)- |
| 339 | CH₃ | CH₂CH₂CH₃ | -CH(CH₃)CH(CH₃)- |
| 340 | CH₃ | CH(CH₃)₂ | -CH(CH₃)CH(CH₃)- |
| 341 | CH₃ | CH₂CH₂CH₂CH₃ | -CH(CH₃)CH(CH₃)- |
| 342 | CH₃ | i-C₄H₉ | -CH(CH₃)CH(CH₃)- |
| 343 | CH₃ | S-C₄H₉ | -CH(CH₃)CH(CH₃)- |
| 344 | CH₃ | C(CH₃)₃ | -CH(CH₃)CH(CH₃)- |
| 345 | CH₃ | CH₂CH₂CH₂CH₂CH₃ | -CH(CH₃)CH(CH₃)- |
| 346 | CH₃ | CH₂CH₂CH₂CH₂CH₂CH₃ | -CH(CH₃)CH(CH₃)- |
| 347 | CH₃ | Cyclopentyl | -CH(CH₃)CH(CH₃)- |
| 348 | CH₃ | Cyclohexyl | -CH(CH₃)CH(CH₃)- |
| 349 | CH₃ | Allyl | -CH(CH₃)CH(CH₃)- |
| 350 | CH₃ | But-2-en-1-yl | -CH(CH₃)CH(CH₃)- |
| 351 | CH₃ | 4-Chlor-but-2-en-1-yl | -CH(CH₃)CH(CH₃)- |
| 352 | CH₃ | Propargyl | -CH(CH₃)CH(CH₃)- |
| 353 | CH₃ | C₆H₅ | -CH(CH₃)CH(CH₃)- |
| 354 | CH₃ | C₆H₅CH₂ | -CH(CH₃)CH(CH₃)- |
| 355 | CH₃ | 2-Phenyleth-1-yl | -CH(CH₃)CH(CH₃)- |
| 356 | CH₃ | 4-Cl-C₆H₄ | -CH(CH₃)CH(CH₃)- |
| 357 | CH₃ | 4-F-C₆H₄ | -CH(CH₃)CH(CH₃)- |
| 358 | CH₃ | CH₃ | -CH₂CH₂CH₂- |
| 359 | CH₃ | C₂H₅ | -CH₂CH₂CH₂- |
| 360 | CH₃ | CH₂CH₂CH₃ | -CH₂CH₂CH₂- |
| 361 | CH₃ | CH(CH₃)₂ | -CH₂CH₂CH₂- |
| 362 | CH₃ | CH₂CH₂CH₂CH₃ | -CH₂CH₂CH₂- |
| 363 | CH₃ | i-C₄H₉ | -CH₂CH₂CH₂- |
| 364 | CH₃ | S-C₄H₉ | -CH₂CH₂CH₂- |
| 365 | CH₃ | C(CH₃)₃ | -CH₂CH₂CH₂- |
| 366 | CH₃ | CH₂CH₂CH₂CH₂CH₃ | -CH₂CH₂CH₂- |
| 367 | CH₃ | CH₂CH₂CH₂CH₂CH₂CH₃ | -CH₂CH₂CH₂- |
| 368 | CH₃ | Cyclopentyl | -CH₂CH₂CH₂- |
| 369 | CH₃ | Cyclohexyl | -CH₂CH₂CH₂- |
| 370 | CH₃ | Allyl | -CH₂CH₂CH₂- |
| 371 | CH₃ | But-2-en-1-yl | -CH₂CH₂CH₂- |
| 372 | CH₃ | 4-Chlor-but-2-en-1-yl | -CH₂CH₂CH₂- |
| 373 | CH₃ | Propargyl | -CH₂CH₂CH₂- |
| 374 | CH₃ | C₆H₅ | -CH₂CH₂CH₂- |
| 375 | CH₃ | C₆H₅CH₂ | -CH₂CH₂CH₂- |
| 376 | CH₃ | 2-Phenyleth-1-yl | -CH₂CH₂CH₂- |
| 377 | CH₃ | 4-Cl-C₆H₄ | -CH₂CH₂CH₂- |
| 378 | CH₃ | 4-F-C₆H₄ | -CH₂CH₂CH₂- |
| 379 | C₂H₅ | CH₃ | -CH₂- |
| 380 | C₂H₅ | C₂H₅ | -CH₂- |
| 381 | C₂H₅ | CH₂CH₂CH₃ | -CH₂- |
| 382 | C₂H₅ | CH(CH₃)₂ | -CH₂- |
| 383 | C₂H₅ | CH₂CH₂CH₂CH₃ | -CH₂- |
| 384 | C₂H₅ | i-C₄H₉ | -CH₂- |
| 385 | C₂H₅ | S-C₄H₉ | -CH₂- |
| 386 | C₂H₅ | C(CH₃)₃ | -CH₂- |
| 387 | C₂H₅ | CH₂CH₂CH₂CH₂CH₃ | -CH₂- |
| 388 | C₂H₅ | CH₂CH₂CH₂CH₂CH₂CH₃ | -CH₂- |
| 389 | C₂H₅ | Cyclopentyl | -CH₂- |
| 390 | C₂H₅ | Cyclohexyl | -CH₂- |
| 391 | C₂H₅ | Allyl | -CH₂- |
| 392 | C₂H₅ | But-2-en-1-yl | -CH₂- |
| 393 | C₂H₅ | 4-Chlor-but-2-en-1-yl | -CH₂- |
| 394 | C₂H₅ | Propargyl | -CH₂- |
| 395 | C₂H₅ | C₆H₅ | -CH₂- |
| 396 | C₂H₅ | C₆H₅CH₂ | -CH₂- |
| 397 | C₂H₅ | 2-Phenyleth-1-yl | -CH₂- |
| 398 | C₂H₅ | 4-Cl-C₆H₄ | -CH₂- |
| 399 | C₂H₅ | 4-F-C₆H₄ | -CH₂- |
| 400 | C₂H₅ | CH₃ | -CH(CH₃)- |
| 401 | C₂H₅ | C₂H₅ | -CH(CH₃)- |
| 402 | C₂H₅ | CH₂CH₂CH₃ | -CH(CH₃)- |
| 403 | C₂H₅ | CH(CH₃)₂ | -CH(CH₃)- |
| 404 | C₂H₅ | CH₂CH₂CH₂CH₃ | -CH(CH₃)- |
| 405 | C₂H₅ | i-C₄H₉ | -CH(CH₃)- |
| 406 | C₂H₅ | s-C₄H₉ | -CH(CH₃)- |
| 407 | C₂H₅ | C(CH₃)₃ | -CH(CH₃)- |
| 408 | C₂H₅ | CH₂CH₂CH₂CH₂CH₃ | -CH(CH₃)- |
| 409 | C₂H₅ | CH₂CH₂CH₂CH₂CH₂CH₃ | -CH(CH₃)- |
| 410 | C₂H₅ | Cyclopentyl | -CH(CH₃)- |
| 411 | C₂H₅ | Cyclohexyl | -CH(CH₃)- |
| 412 | C₂H₅ | Allyl | -CH(CH₃)- |
| 413 | C₂H₅ | But-2-en-1-yl | -CH(CH₃)- |
| 414 | C₂H₅ | 4-Chlor-but-2-en-1-yl | -CH(CH₃)- |
| 415 | C₂H₅ | Propargyl | -CH(CH₃)- |
| 416 | C₂H₅ | C₆H₅ | -CH(CH₃)- |
| 417 | C₂H₅ | C₆H₅CH₂ | -CH(CH₃)- |
| 418 | C₂H₅ | 2-Phenyleth-1-yl | -CH(CH₃)- |
| 419 | C₂H₅ | 4-Cl-C₆H₄ | -CH(CH₃)- |
| 420 | C₂H₅ | 4-F-C₆H₄ | -CH(CH₃)- |
| 421 | C₂H₅ | CH₃ | -CH(C₂H₅)- |
| 422 | C₂H₅ | C₂H₅ | -CH(C₂H₅)- |
| 423 | C₂H₅ | CH₂CH₂CH₃ | -CH(C₂H₅)- |
| 424 | C₂H₅ | CH(CH₃)₂ | -CH(C₂H₅)- |
| 425 | C₂H₅ | CH₂CH₂CH₂CH₃ | -CH(C₂H₅)- |
| 426 | C₂H₅ | i-C₄H₉ | -CH(C₂H₅)- |
| 427 | C₂H₅ | S-C₄H₉ | -CH(C₂H₅)- |
| 428 | C₂H₅ | C(CH₃)₃ | -CH(C₂H₅)- |
| 429 | C₂H₅ | CH₂CH₂CH₂CH₂CH₃ | -CH(C₂H₅)- |
| 430 | C₂H₅ | CH₂CH₂CH₂CH₂CH₂CH₃ | -CH(C₂H₅)- |
| 431 | C₂H₅ | Cyclopentyl | -CH(C₂H₅)- |
| 432 | C₂H₅ | Cyclohexyl | -CH(C₂H₅)- |
| 433 | C₂H₅ | Allyl | -CH(C₂H₅)- |
| 434 | C₂H₅ | But-2-en-1-yl | -CH(C₂H₅)- |
| 435 | C₂H₅ | 4-Chlor-but-2-en-1-yl | -CH(C₂H₅)- |
| 436 | C₂H₅ | Propargyl | -CH(C₂H₅)- |
| 437 | C₂H₅ | C₆H₅ | -CH(C₂H₅)- |
| 438 | C₂H₅ | C₆H₅CH₂ | -CH(C₂H₅)- |
| 439 | C₂H₅ | 2-Phenyleth-1-yl | -CH(C₂H₅)- |
| 440 | C₂H₅ | 4-Cl-C₆H₄ | -CH(C₂H₅)- |
| 441 | C₂H₅ | 4-F-C₆H₄ | -CH(C₂H₅)- |
| 442 | C₂H₅ | CH₃ | -C(CH₃)₂- |
| 443 | C₂H₅ | C₂H₅ | -C(CH₃)₂- |
| 444 | C₂H₅ | CH₂CH₂CH₃ | -C(CH₃)₂- |
| 445 | C₂H₅ | CH(CH₃)₂ | -C(CH₃)₂- |
| 446 | C₂H₅ | CH₂CH₂CH₂CH₃ | -C(CH₃)₂- |
| 447 | C₂H₅ | i-C₄H₉ | -C(CH₃)₂- |
| 448 | C₂H₅ | S-C₄H₉ | -C(CH₃)₂- |
| 449 | C₂H₅ | C(CH₃)₃ | -C(CH₃)₂- |
| 450 | C₂H₅ | CH₂CH₂CH₂CH₂CH₃ | -C(CH₃)₂- |
| 451 | C₂H₅ | CH₂CH₂CH₂CH₂CH₂CH₃ | -C(CH₃)₂- |
| 452 | C₂H₅ | Cyclopentyl | -C(CH₃)₂- |
| 453 | C₂H₅ | Cyclohexyl | -C(CH₃)₂- |
| 454 | C₂H₅ | Allyl | -C(CH₃)₂- |
| 455 | C₂H₅ | But-2-en-1-yl | -C(CH₃)₂- |
| 456 | C₂H₅ | 4-Chlor-but-2-en-1-yl | -C(CH₃)₂- |
| 457 | C₂H₅ | Propargyl | -C(CH₃)₂- |
| 458 | C₂H₅ | C₆H₅ | -C(CH₃)₂- |
| 459 | C₂H₅ | C₆H₅CH₂ | -C(CH₃)₂- |
| 460 | C₂H₅ | 2-Phenyleth-1-yl | -C(CH₃)₂- |
| 461 | C₂H₅ | 4-CI-C₆H₄ | -C(CH₃)₂- |
| 462 | C₂H₅ | 4-F-C₆H₄ | -C(CH₃)₂- |
| 463 | C₂H₅ | CH₃ | -CH₂CH₂- |
| 464 | C₂H₅ | C₂H₅ | -CH₂CH₂- |
| 465 | C₂Hₛ | CH₂CH₂CH₃ | -CH₂CH₂- |
| 466 | C₂H₅ | CH(CH₃)₂ | -CH₂CH₂- |
| 467 | C₂H₅ | CH₂CH₂CH₂CH₃ | -CH₂CH₂- |
| 468 | C₂H₅ | i-C₄H₉ | -CH₂CH₂- |
| 469 | C₂H₅ | S-C₄H₉ | -CH₂CH₂- |
| 470 | C₂H₅ | C(CH₃)₃ | -CH₂CH₂- |
| 471 | C₂H₅ | CH₂CH₂CH₂CH₂CH₃ | -CH₂CH₂- |
| 472 | C₂H₅ | CH₂CH₂CH₂CH₂CH₂CH₃ | -CH₂CH₂- |
| 473 | C₂H₅ | Cyclopentyl | -CH₂CH₂- |
| 474 | C₂H₅ | Cyclohexyl | -CH₂CH₂- |
| 475 | C₂H₅ | Allyl | -CH₂CH₂- |
| 476 | C₂H₅ | But-2-en-1-yl | -CH₂CH₂- |
| 477 | C₂H₅ | 4-Chlor-but-2-en-1-yl | -CH₂CH₂- |
| 478 | C₂H₅ | Propargyl | -CH₂CH₂- |
| 479 | C₂H₅ | C₆H₅ | -CH₂CH₂- |
| 480 | C₂H₅ | C₆H₅CH₂ | -CH₂CH₂- |
| 481 | C₂H₅ | 2-Phenyleth-1-yl | -CH₂CH₂- |
| 482 | C₂H₅ | 4-Cl-C₆H₄ | -CH₂CH₂- |
| 483 | C₂H₅ | 4-F-C₆H₄ | -CH₂CH₂- |
| 484 | C₂H₅ | CH₃ | -CH(CH₃)CH₂- |
| 485 | C₂H₅ | C₂H₅ | -CH(CH₃)CH₂- |
| 486 | C₂H₅ | CH₂CH₂CH₃ | -CH(CH₃)CH₂- |
| 487 | C₂H₅ | CH(CH₃)₂ | -CH(CH₃)CH₂- |
| 488 | C₂H₅ | CH₂CH₂CH₂CH₃ | -CH(CH₃)CH₂- |
| 489 | C₂H₅ | i-C₄H₉ | -CH(CH₃)CH₂- |
| 490 | C₂H₅ | s-C₄H₉ | -CH(CH₃)CH₂- |
| 491 | C₂H₅ | C(CH₃)₃ | -CH(CH₃)CH₂- |
| 492 | C₂H₅ | CH₂CH₂CH₂CH₂CH₃ | -CH(CH₃)CH₂- |
| 493 | C₂H₅ | CH₂CH₂CH₂CH₂CH₂CH₃ | -CH(CH₃)CH₂- |
| 494 | C₂H₅ | Cyclopentyl | -CH(CH₃)CH₂- |
| 495 | C₂H₅ | Cyclohexyl | -CH(CH₃)CH₂- |
| 496 | C₂H₅ | Allyl | -CH(CH₃)CH₂- |
| 497 | C₂H₅ | But-2-en-1-yl | -CH(CH₃)CH₂- |
| 498 | C₂H₅ | 4-Chlor-but-2-en-1-yl | -CH(CH₃)CH₂- |
| 499 | C₂H₅ | Propargyl | -CH(CH₃)CH₂- |
| 500 | C₂H₅ | C₆H₅ | -CH(CH₃)CH₂- |
| 501 | C₂H₅ | C₆H₅CH₂ | -CH(CH₃)CH₂- |
| 502 | C₂H₅ | 2-Phenyleth-1-yl | -CH(CH₃)CH₂- |
| 503 | C₂H₅ | 4-Cl-C₆H₄ | -CH(CH₃)CH₂- |
| 504 | C₂H₅ | 4-F-C₆H₄ | -CH(CH₃)CH₂- |
| 505 | C₂H₅ | CH₃ | -CH₂CH(CH₃)- |
| 506 | C₂H₅ | C₂H₅ | -CH₂CH(CH₃)- |
| 507 | C₂H₅ | CH₂CH₂CH₃ | -CH₂CH(CH₃)- |
| 508 | C₂H₅ | CH(CH₃)₂ | -CH₂CH(CH₃)- |
| 509 | C₂H₅ | CH₂CH₂CH₂CH₃ | -CH₂CH(CH₃)- |
| 510 | C₂H₅ | i-C₄H₉ | -CH₂CH(CH₃)- |
| 511 | C₂H₅ | s-C₄H₉ | -CH₂CH(CH₃)- |
| 512 | C₂H₅ | C(CH₃)₃ | -CH₂CH(CH₃)- |
| 513 | C₂H₅ | CH₂CH₂CH₂CH₂CH₃ | -CH₂CH(CH₃)- |
| 514 | C₂H₅ | CH₂CH₂CH₂CH₂CH₂CH₃ | -CH₂CH(CH₃)- |
| 515 | C₂H₅ | Cyclopentyl | -CH₂CH(CH₃)- |
| 516 | C₂H₅ | Cyclohexyl | -CH₂CH(CH₃)- |
| 517 | C₂H₅ | Allyl | -CH₂CH(CH₃)- |
| 518 | C₂H₅ | But-2-en-1-yl | -CH₂CH(CH₃)- |
| 519 | C₂H₅ | 4-Chlor-but-2-en-1-yl | -CH₂CH(CH₃)- |
| 520 | C₂H₅ | Propargyl | -CH₂CH(CH₃)- |
| 521 | C₂H₅ | C₆H₅ | -CH₂CH(CH₃)- |
| 522 | C₂H₅ | C₆H₅CH₂ | -CH₂CH(CH₃)- |
| 523 | C₂H₅ | 2-Phenyleth-1-yl | -CH₂CH(CH₃)- |
| 524 | C₂H₅ | 4-Cl-C₆H₄ | -CH₂CH(CH₃)- |
| 525 | C₂H₅ | 4-F-C₆H₄ | -CH₂CH(CH₃)- |
| 526 | C₂H₅ | CH₃ | -CH(CH₃)CH(CH₃)- |
| 527 | C₂H₅ | C₂H₅ | -CH(CH₃)CH(CH₃)- |
| 528 | C₂H₅ | CH₂CH₂CH₃ | -CH(CH₃)CH(CH₃)- |
| 529 | C₂H₅ | CH(CH₃)₂ | -CH(CH₃)CH(CH₃)- |
| 530 | C₂H₅ | CH₂CH₂CH₂CH₃ | -CH(CH₃)CH(CH₃)- |
| 531 | C₂H₅ | i-C₄H₉ | -CH(CH₃)CH(CH₃)- |
| 532 | C₂H₅ | s-C₄H₉ | -CH(CH₃)CH(CH₃)- |
| 533 | C₂H₅ | C(CH₃)₃ | -CH(CH₃)CH(CH₃)- |
| 534 | C₂H₅ | CH₂CH₂CH₂CH₂CH₃ | -CH(CH₃)CH(CH₃)- |
| 535 | C₂H₅ | CH₂CH₂CH₂CH₂CH₂CH₃ | -CH(CH₃)CH(CH₃)- |
| 536 | C₂H₅ | Cyclopentyl | -CH(CH₃)CH(CH₃)- |
| 537 | C₂H₅ | Cyclohexyl | -CH(CH₃)CH(CH₃)- |
| 538 | C₂H₅ | Allyl | -CH(CH₃)CH(CH₃)- |
| 539 | C₂H₅ | But-2-en-1-yl | -CH(CH₃)CH(CH₃)- |
| 540 | C₂H₅ | 4-Chlor-but-2-en-1-yl | -CH(CH₃)CH(CH₃)- |
| 541 | C₂H₅ | Propargyl | -CH(CH₃)CH(CH₃)- |
| 542 | C₂H₅ | C₆H₅ | -CH(CH₃)CH(CH₃)- |
| 543 | C₂H₅ | C₆H₅CH₂ | -CH(CH₃)CH(CH₃)- |
| 544 | C₂H₅ | 2-Phenyleth-1-yl | -CH(CH₃)CH(CH₃)- |
| 545 | C₂H₅ | 4-Cl-C₆H₄ | -CH(CH₃)CH(CH₃)- |
| 546 | C₂H₅ | 4-F-C₆H₄ | -CH(CH₃)CH(CH₃)- |
| 547 | C₂H₅ | CH₃ | -CH₂CH₂CH₂- |
| 548 | C₂H₅ | C₂H₅ | -CH₂CH₂CH₂- |
| 549 | C₂H₅ | CH₂CH₂CH₃ | -CH₂CH₂CH₂- |
| 550 | C₂H₅ | CH(CH₃)₂ | -CH₂CH₂CH₂- |
| 551 | C₂H₅ | CH₂CH₂CH₂CH₃ | -CH₂CH₂CH₂- |
| 552 | C₂H₅ | i-C₄H₉ | -CH₂CH₂CH₂- |
| 553 | C₂H₅ | s-C₄H₉ | -CH₂CH₂CH₂- |
| 554 | C₂H₅ | C(CH₃)₃ | -CH₂CH₂CH₂- |
| 555 | C₂H₅ | CH₂CH₂CH₂CH₂CH₃ | -CH₂CH₂CH₂- |
| 556 | C₂H₅ | CH₂CH₂CH₂CH₂CH₂CH₃ | -CH₂CH₂CH₂- |
| 557 | C₂H₅ | Cyclopentyl | -CH₂CH₂CH₂- |
| 558 | C₂H₅ | Cyclohexyl | -CH₂CH₂CH₂- |
| 559 | C₂H₅ | Allyl | -CH₂CH₂CH₂- |
| 560 | C₂H₅ | But-2-en-1-yl | -CH₂CH₂CH₂- |
| 561 | C₂Hₛ | 4-Chlor-but-2-en-1-yl | -CH₂CH₂CH₂- |
| 562 | C₂H₅ | Propargyl | -CH₂CH₂CH₂- |
| 563 | C₂H₅ | C₆H₅ | -CH₂CH₂CH₂- |
| 564 | C₂H₅ | C₆H₅CH₂ | -CH₂CH₂CH₂- |
| 565 | C₂H₅ | 2-Phenyleth-1-yl | -CH₂CH₂CH₂- |
| 566 | C₂H₅ | 4-Cl-C₆H₄ | -CH₂CH₂CH₂- |
| 567 | C₂H₅ | 4-F-C₆H₄ | -CH₂CH₂CH₂- |
| 568 | CH₂CH₂CH₃ | CH₃ | -CH₂- |
| 569 | CH₂CH₂CH₃ | C₂H₅ | -CH₂- |
| 570 | CH₂CH₂CH₃ | CH₂CH₂CH₃ | -CH₂- |
| 571 | CH₂CH₂CH₃ | CH(CH₃)₂ | -CH₂- |
| 572 | CH₂CH₂CH₃ | CH₂CH₂CH₂CH₃ | -CH₂- |
| 573 | CH₂CH₂CH₃ | i-C₄H₉ | -CH₂- |
| 574 | CH₂CH₂CH₃ | S-C₄H₉ | -CH₂- |
| 575 | CH₂CH₂CH₃ | C(CH₃)₃ | -CH₂- |
| 576 | CH₂CH₂CH₃ | CH₂CH₂CH₂CH₂CH₃ | -CH₂- |
| 577 | CH₂CH₂CH₃ | CH₂CH₂CH₂CH₂CH₂CH₃ | -CH₂- |
| 578 | CH₂CH₂CH₃ | Cyclopentyl | -CH₂- |
| 579 | CH₂CH₂CH₃ | Cyclohexyl | -CH₂- |
| 580 | CH₂CH₂CH₃ | Allyl | -CH₂- |
| 581 | CH₂CH₂CH₃ | But-2-en-1-yl | -CH₂- |
| 582 | CH₂CH₂CH₃ | 4-Chlor-but-2-en-1-yl | -CH₂- |
| 583 | CH₂CH₂CH₃ | Propargyl | -CH₂- |
| 584 | CH₂CH₂CH₃ | C₆H₅ | -CH₂- |
| 585 | CH₂CH₂CH₃ | C₆H₅CH₂ | -CH₂- |
| 586 | CH₂CH₂CH₃ | 2-Phenyleth-1-yl | -CH₂- |
| 587 | CH₂CH₂CH₃ | 4-Cl-C₆H₄ | -CH₂- |
| 588 | CH₂CH₂CH₃ | 4-F-C₆H₄ | -CH₂- |
| 589 | CH₂CH₂CH₃ | CH₃ | -CH(CH₃)- |
| 590 | CH₂CH₂CH₃ | C₂H₅ | -CH(CH₃)- , |
| 591 | CH₂CH₂CH₃ | CH₂CH₂CH₃ | -CH(CH₃)- |
| 592 | CH₂CH₂CH₃ | CH(CH₃)₂ | -CH(CH₃)- |
| 593 | CH₂CH₂CH₃ | CH₂CH₂CH₂CH₃ | -CH(CH₃)- |
| 594 | CH₂CH₂CH₃ | i-C₄H₉ | -CH(CH₃)- |
| 595 | CH₂CH₂CH₃ | s-C₄H₉ | -CH(CH₃)- |
| 596 | CH₂CH₂CH₃ | C(CH₃)₃ | -CH(CH₃)- |
| 597 | CH₂CH₂CH₃ | CH₂CH₂CH₂CH₂CH₃ | -CH(CH₃)- |
| 598 | CH₂CH₂CH₃ | CH₂CH₂CH₂CH₂CH₂CH₃ | -CH(CH₃)- |
| 599 | CH₂CH₂CH₃ | Cyclopentyl | -CH(CH₃)- |
| 600 | CH₂CH₂CH₃ | Cyclohexyl | -CH(CH₃)- |
| 601 | CH₂CH₂CH₃ | Allyl | -CH(CH₃)- |
| 602 | CH₂CH₂CH₃ | But-2-en-1-yl | -CH(CH₃)- |
| 603 | CH₂CH₂CH₃ | 4-Chlor-but-2-en-1-yl | -CH(CH₃)- |
| 604 | CH₂CH₂CH₃ | Propargyl | -CH(CH₃)- |
| 605 | CH₂CH₂CH₃ | C₆H₅ | -CH(CH₃)- |
| 606 | CH₂CH₂CH₃ | C₆H₅CH₂ | -CH(CH₃)- |
| 607 | CH₂CH₂CH₃ | 2-Phenyleth-1-yl | -CH(CH₃)- |
| 608 | CH₂CH₂CH₃ | 4-Cl-C₆H₄ | -CH(CH₃)- |
| 609 | CH₂CH₂CH₃ | 4-F-C₆H₄ | -CH(CH₃)- |
| 610 | CH₂CH₂CH₃ | CH₃ | -CH₂CH₂- |
| 611 | CH₂CH₂CH₃ | C₂H₅ | -CH₂CH₂- |
| 612 | CH₂CH₂CH₃ | CH₂CH₂CH₃ | -CH₂CH₂- |
| 613 | CH₂CH₂CH₃ | CH(CH₃)₂ | -CH₂CH₂- |
| 614 | CH₂CH₂CH₃ | CH₂CH₂CH₂CH₃ | -CH₂CH₂- |
| 615 | CH₂CH₂CH₃ | i-C₄H₉ | -CH₂CH₂- |
| 616 | CH₂CH₂CH₃ | S-C₄H₉ | -CH₂CH₂- |
| 617 | CH₂CH₂CH₃ | C(CH₃)₃ | -CH₂CH₂- |
| 618 | CH₂CH₂CH₃ | CH₂CH₂CH₂CH₂CH₃ | -CH₂CH₂- |
| 619 | CH₂CH₂CH₃ | CH₂CH₂CH₂CH₂CH₂CH₃ | -CH₂CH₂- |
| 620 | CH₂CH₂CH₃ | Cyclopentyl | -CH₂CH₂- |
| 621 | CH₂CH₂CH₃ | Cyclohexyl | -CH₂CH₂- |
| 622 | CH₂CH₂CH₃ | Allyl | -CH₂CH₂- |
| 623 | CH₂CH₂CH₃ | But-2-en-1-yl | -CH₂CH₂- |
| 624 | CH₂CH₂CH₃ | 4-Chlor-but-2-en-1-yl | -CH₂CH₂- |
| 625 | CH₂CH₂CH₃ | Propargyl | -CH₂CH₂- |
| 626 | CH₂CH₂CH₃ | C₆H₅ | -CH₂CH₂- |
| 627 | CH₂CH₂CH₃ | C₆H₅CH₂ | -CH₂CH₂- |
| 628 | CH₂CH₂CH₃ | 2-Phenyleth-1-yl | -CH₂CH₂- |
| 629 | CH₂CH₂CH₃ | 4-Cl-C₆H₄ | -CH₂CH₂- |
| 630 | CH₂CH₂CH₃ | 4-F-C₆H₄ | -CH₂CH₂- |
| 631 | CH₂CH₂CH₃ | CH₃ | -CH₂CH₂CH₂- |
| 632 | CH₂CH₂CH₃ | C₂H₅ | -CH₂CH₂CH₂- |
| 633 | CH₂CH₂CH₃ | CH₂CH₂CH₃ | -CH₂CH₂CH₂- |
| 634 | CH₂CH₂CH₃ | CH(CH₃)₂ | -CH₂CH₂CH₂- |
| 635 | CH₂CH₂CH₃ | CH₂CH₂CH₂CH₃ | -CH₂CH₂CH₂- |
| 636 | CH₂CH₂CH₃ | i-C₄H₉ | -CH₂CH₂CH₂- |
| 637 | CH₂CH₂CH₃ | s-C₄H₉ | -CH₂CH₂CH₂- |
| 638 | CH₂CH₂CH₃ | C(CH₃)₃ | -CH₂CH₂CH₂- |
| 639 | CH₂CH₂CH₃ | CH₂CH₂CH₂CH₂CH₃ | -CH₂CH₂CH₂- |
| 640 | CH₂CH₂CH₃ | CH₂CH₂CH₂CH₂CH₂CH₃ | -CH₂CH₂CH₂- |
| 641 | CH₂CH₂CH₃ | Cyclopentyl | -CH₂CH₂CH₂- |
| 642 | CH₂CH₂CH₃ | Cyclohexyl | -CH₂CH₂CH₂- |
| 643 | CH₂CH₂CH₃ | Allyl | -CH₂CH₂CH₂- |
| 644 | CH₂CH₂CH₃ | But-2-en-1-yl | -CH₂CH₂CH₂- |
| 645 | CH₂CH₂CH₃ | 4-Chlor-but-2-en-1-yl | -CH₂CH₂CH₂- |
| 646 | CH₂CH₂CH₃ | Propargyl | -CH₂CH₂CH₂- |
| 647 | CH₂CH₂CH₃ | C₆H₅ | -CH₂CH₂CH₂- |
| 648 | CH₂CH₂CH₃ | C₆H₅CH₂ | -CH₂CH₂CH₂- |
| 649 | CH₂CH₂CH₃ | 2-Phenyleth-1-yl | -CH₂CH₂CH₂- |
| 650 | CH₂CH₂CH₃ | 4-Cl-C₆H₄ | -CH₂CH₂CH₂- |
| 651 | CH₂CH₂CH₃ | 4-F-C₆H₄ | -CH₂CH₂CH₂- |
| 652 | CH(CH₃)₂ | CH₃ | -CH₂- |
| 653 | CH(CH₃)₂ | C₂H₅ | -CH₂- |
| 654 | CH(CH₃)₂ | CH₂CH₂CH₃ | -CH₂- |
| 655 | CH(CH₃)₂ | CH(CH₃)₂ | -CH₂- |
| 656 | CH(CH₃)₂ | CH₂CH₂CH₂CH₃ | -CH₂- |
| 657 | CH(CH₃)₂ | i-C₄H₉ | -CH₂- |
| 658 | CH(CH₃)₂ | s-C₄H₉ | -CH₂- |
| 659 | CH(CH₃)₂ | C(CH₃)₃ | -CH₂- |
| 660 | CH(CH₃)₂ | CH₂CH₂CH₂CH₂CH₃ | -CH₂- |
| 661 | CH(CH₃)₂ | CH₂CH₂CH₂CH₂CH₂CH₃ | -CH₂- |
| 662 | CH(CH₃)₂ | Cyclopentyl | -CH₂- |
| 663 | CH(CH₃)₂ | Cyclohexyl | -CH₂- |
| 664 | CH(CH₃)₂ | Allyl | -CH₂- |
| 665 | CH(CH₃)₂ | But-2-en-1-yl | -CH₂- |
| 666 | CH(CH₃)₂ | 4-Chlor-but-2-en-1-yl | -CH₂- |
| 667 | CH(CH₃)₂ | Propargyl | -CH₂- |
| 668 | CH(CH₃)₂ | C₆H₅ | -CH₂- |
| 669 | CH(CH₃)₂ | C₆H₅CH₂ | -CH₂- |
| 670 | CH(CH₃)₂ | 2-Phenyleth-1-yl | -CH₂- |
| 671 | CH(CH₃)₂ | 4-Cl-C₆H₄ | -CH₂- |
| 672 | CH(CH₃)₂ | 4-F-C₆H₄ | -CH₂- |
| 673 | CH(CH₃)₂ | CH₃ | -CH(CH₃)- |
| 674 | CH(CH₃)₂ | C₂H₅ | -CH(CH₃)- |
| 675 | CH(CH₃)₂ | CH₂CH₂CH₃ | -CH(CH₃)- |
| 676 | CH(CH₃)₂ | CH(CH₃)₂ | -CH(CH₃)- |
| 677 | CH(CH₃)₂ | CH₂CH₂CH₂CH₃ | -CH(CH₃)- |
| 678 | CH(CH₃)₂ | i-C₄H₉ | -CH(CH₃)- |
| 679 | CH(CH₃)₂ | s-C₄H₉ | -CH(CH₃)- |
| 680 | CH(CH₃)₂ | C(CH₃)₃ | -CH(CH₃)- |
| 681 | CH(CH₃)₂ | CH₂CH₂CH₂CH₂CH₃ | -CH(CH₃)- |
| 682 | CH(CH₃)₂ | CH₂CH₂CH₂CH₂CH₂CH₃ | -CH(CH₃)- |
| 683 | CH(CH₃)₂ | Cyclopentyl | -CH(CH₃)- |
| 684 | CH(CH₃)₂ | Cyclohexyl | -CH(CH₃)- |
| 685 | CH(CH₃)₂ | Allyl | -CH(CH₃)- |
| 686 | CH(CH₃)₂ | But-2-en-1-yl | -CH(CH₃)- |
| 687 | CH(CH₃)₂ | 4-Chlor-but-2-en-1-yl | -CH(CH₃)- |
| 688 | CH(CH₃)₂ | Propargyl | -CH(CH₃)- |
| 689 | CH(CH₃)₂ | C₆H₅ | -CH(CH₃)- |
| 690 | CH(CH₃)₂ | C₆H₅CH₂ | -CH(CH₃)- |
| 691 | CH(CH₃)₂ | 2-Phenyleth-1-yl | -CH(CH₃)- |
| 692 | CH(CH₃)₂ | 4-Cl-C₆H₄ | -CH(CH₃)- |
| 693 | CH(CH₃)₂ | 4-F-C₆H₄ | -CH(CH₃)- |
| 694 | CH(CH₃)₂ | CH₃ | -CH₂CH₂- |
| 695 | CH(CH₃)₂ | C₂H₅ | -CH₂CH₂- |
| 696 | CH(CH₃)₂ | CH₂CH₂CH₃ | -CH₂CH₂- |
| 697 | CH(CH₃)₂ | CH(CH₃)₂ | -CH₂CH₂- |
| 698 | CH(CH₃)₂ | CH₂CH₂CH₂CH₃ | -CH₂CH₂- |
| 699 | CH(CH₃)₂ | i-C₄H₉ | -CH₂CH₂- |
| 700 | CH(CH₃)₂ | s-C₄H₉ | -CH₂CH₂- |
| 701 | CH(CH₃)₂ | C(CH₃)₃ | -CH₂CH₂- |
| 702 | CH(CH₃)₂ | CH₂CH₂CH₂CH₂CH₃ | -CH₂CH₂- |
| 703 | CH(CH₃)₂ | CH₂CH₂CH₂CH₂CH₂CH₃ | -CH₂CH₂- |
| 704 | CH(CH₃)₂ | Cyclopentyl | -CH₂CH₂- |
| 705 | CH(CH₃)₂ | Cyclohexyl | -CH₂CH₂- |
| 706 | CH(CH₃)₂ | Allyl | -CH₂CH₂- |
| 707 | CH(CH₃)₂ | But-2-en-1-yl | -CH₂CH₂- |
| 708 | CH(CH₃)₂ | 4-Chlor-but-2-en-1-yl | -CH₂CH₂- |
| 709 | CH(CH₃)₂ | Propargyl | -CH₂CH₂- |
| 710 | CH(CH₃)₂ | C₆H₅ | -CH₂CH₂- |
| 711 | CH(CH₃)₂ | C₆H₅CH₂ | -CH₂CH₂- |
| 712 | CH(CH₃)₂ | 2-Phenyleth-1-yl | -CH₂CH₂- |
| 713 | CH(CH₃)₂ | 4-Cl-C₆H₄ | -CH₂CH₂- |
| 714 | CH(CH₃)₂ | 4-F-C₆H₄ | -CH₂CH₂- |
| 715 | CH(CH₃)₂ | CH₃ | -CH₂CH₂CH₂- |
| 716 | CH(CH₃)₂ | C₂H₅ | -CH₂CH₂CH₂- |
| 717 | CH(CH₃)₂ | CH₂CH₂CH₃ | -CH₂CH₂CH₂- |
| 718 | CH(CH₃)₂ | CH(CH₃)₂ | -CH₂CH₂CH₂- |
| 719 | CH(CH₃)₂ | CH₂CH₂CH₂CH₃ | -CH₂CH₂CH₂- |
| 720 | CH(CH₃)₂ | i-C₄H₉ | -CH₂CH₂CH₂- |
| 721 | CH(CH₃)₂ | s-C₄H₉ | -CH₂CH₂CH₂- |
| 722 | CH(CH₃)₂ | C(CH₃)₃ | -CH₂CH₂CH₂- |
| 723 | CH(CH₃)₂ | CH₂CH₂CH₂CH₂CH₃ | -CH₂CH₂CH₂- |
| 724 | CH(CH₃)₂ | CH₂CH₂CH₂CH₂CH₂CH₃ | -CH₂CH₂CH₂- |
| 725 | CH(CH₃)₂ | Cyclopentyl | -CH₂CH₂CH₂- |
| 726 | CH(CH₃)₂ | Cyclohexyl | -CH₂CH₂CH₂- |
| 727 | CH(CH₃)₂ | Allyl | -CH₂CH₂CH₂- |
| 728 | CH(CH₃)₂ | But-2-en-1-yl | -CH₂CH₂CH₂- |
| 729 | CH(CH₃)₂ | 4-Chlor-but-2-en-1-yl | -CH₂CH₂CH₂- |
| 730 | CH(CH₃)₂ | Propargyl | -CH₂CH₂CH₂- |
| 731 | CH(CH₃)₂ | C₆H₅ | -CH₂CH₂CH₂- |
| 732 | CH(CH₃)₂ | C₆H₅CH₂ | -CH₂CH₂CH₂- |
| 733 | CH(CH₃)₂ | 2-Phenyleth-1-yl | -CH₂CH₂CH₂- |
| 734 | CH(CH₃)₂ | 4-Cl-C₆H₄ | -CH₂CH₂CH₂- |
| 735 | CH(CH₃)₂ | 4-F-C₆H₄ | -CH₂CH₂CH₂- |
| 736 | C₆H₅ | CH₃ | -CH₂- |
| 737 | C₆H₅ | C₂H₅ | -CH₂- |
| 738 | C₆H₅ | CH₂CH₂CH₃ | -CH₂- |
| 739 | C₆H₅ | CH(CH₃)₂ | -CH₂- |
| 740 | C₆H₅ | CH₂CH₂CH₂CH₃ | -CH₂- |
| 741 | C₆H₅ | i-C₄H₉ | -CH₂- |
| 742 | C₆H₅ | S-C₄H₉ | -CH₂- |
| 743 | C₆H₅ | C(CH₃)₃ | -CH₂- |
| 744 | C₆H₅ | CH₂CH₂CH₂CH₂CH₃ | -CH₂- |
| 745 | C₆H₅ | CH₂CH₂CH₂CH₂CH₂CH₃ | -CH₂- |
| 746 | C₆H₅ | Cyclopentyl | -CH₂- |
| 747 | C₆H₅ | Cyclohexyl | -CH₂- |
| 748 | C₆H₅ | Allyl | -CH₂- |
| 749 | C₆H₅ | But-2-en-1-yl | -CH₂- |
| 750 | C₆H₅ | 4-Chlor-but-2-en-1-yl | -CH₂- |
| 751 | C₆H₅ | Propargyl | -CH₂- |
| 752 | C₆H₅ | C₆H₅ | -CH₂- |
| 753 | C₆H₅ | C₆H₅CH₂ | -CH₂- |
| 754 | C₆H₅ | 2-Phenyleth-1-yl | -CH₂- |
| 755 | C₆H₅ | 4-Cl-C₆H₄ | -CH₂- |
| 756 | C₆H₅ | 4-F-C₆H₄ | -CH₂- |
| 757 | C₆H₅ | CH₃ | -CH(CH₃)- |
| 758 | C₆H₅ | C₂H₅ | -CH(CH₃)- |
| 759 | C₆H₅ | CH₂CH₂CH₃ | -CH(CH₃)- |
| 760 | C₆H₅ | CH(CH₃)₂ | -CH(CH₃)- |
| 761 | C₆H₅ | CH₂CH₂CH₂CH₃ | -CH(CH₃)- |
| 762 | C₆H₅ | i-C₄H₉ | -CH(CH₃)- |
| 763 | C₆H₅ | s-C₄H₉ | -CH(CH₃)- |
| 764 | C₆H₅ | C(CH₃)₃ | -CH(CH₃)- |
| 765 | C₆H₅ | CH₂CH₂CH₂CH₂CH₃ | -CH(CH₃)- |
| 766 | C₆H₅ | CH₂CH₂CH₂CH₂CH₂CH₃ | -CH(CH₃)- |
| 767 | C₆H₅ | Cyclopentyl | -CH(CH₃)- |
| 768 | C₆H₅ | Cyclohexyl | -CH(CH₃)- |
| 769 | C₆H₅ | Allyl | -CH(CH₃)- |
| 770 | C₆H₅ | But-2-en-1-yl | -CH(CH₃)- |
| 771 | C₆H₅ | 4-Chlor-but-2-en-1-yl | -CH(CH₃)- |
| 772 | C₆H₅ | Propargyl | -CH(CH₃)- |
| 773 | C₆H₅ | C₆H₅ | -CH(CH₃)- |
| 774 | C₆H₅ | C₆H₅CH₂ | -CH(CH₃)- |
| 775 | C₆H₅ | 2-Phenyleth-1-yl | -CH(CH₃)- |
| 776 | C₆H₅ | 4-Cl-C₆H₄ | -CH(CH₃)- |
| 777 | C₆H₅ | 4-F-C₆H₄ | -CH(CH₃)- |
| 778 | C₆H₅ | CH₃ | -CH₂CH₂- |
| 779 | C₆H₅ | C₂H₅ | -CH₂CH₂- |
| 780 | C₆H₅ | CH₂CH₂CH₃ | -CH₂CH₂- |
| 781 | C₆H₅ | CH(CH₃)₂ | -CH₂CH₂- |
| 782 | C₆H₅ | CH₂CH₂CH₂CH₃ | -CH₂CH₂- |
| 783 | C₆H₅ | i-C₄H₉ | -CH₂CH₂- |
| 784 | C₆H₅ | s-C₄H₉ | -CH₂CH₂- |
| 785 | C₆H₅ | C(CH₃)₃ | -CH₂CH₂- |
| 786 | C₆H₅ | CH₂CH₂CH₂CH₂CH₃ | -CH₂CH₂- |
| 787 | C₆H₅ | CH₂CH₂CH₂CH₂CH₂CH₃ | -CH₂CH₂- |
| 788 | C₆H₅ | Cyclopentyl | -CH₂CH₂- |
| 789 | C₆H₅ | Cyclohexyl | -CH₂CH₂- |
| 790 | C₆H₅ | Allyl | -CH₂CH₂- |
| 791 | C₆H₅ | But-2-en-1-yl | -CH₂CH₂- |
| 792 | C₆H₅ | 4-Chlor-but-2-en-1-yl | -CH₂CH₂- |
| 793 | C₆H₅ | Propargyl | -CH₂CH₂- |
| 794 | C₆H₅ | C₆H₅ | -CH₂CH₂- |
| 795 | C₆H₅ | C₆H₅CH₂ | -CH₂CH₂- |
| 796 | C₆H₅ | 2-Phenyleth-1-yl | -CH₂CH₂- |
| 797 | C₆H₅ | 4-Cl-C₆H₄ | -CH₂CH₂- |
| 798 | C₆H₅ | 4-F-C₆H₄ | -CH₂CH₂- |
| 799 | C₆H₅ | CH₃ | -CH₂CH₂CH₂- |
| 800 | C₆H₅ | C₂Hs | -CH₂CH₂CH₂- |
| 801 | C₆H₅ | CH₂CH₂CH₃ | -CH₂CH₂CH₂- |
| 802 | C₆H₅ | CH(CH₃)₂ | -CH₂CH₂CH₂- |
| 803 | C₆H₅ | CH₂CH₂CH₂CH₃ | -CH₂CH₂CH₂- |
| 804 | C₆H₅ | i-C₄H₉ | -CH₂CH₂CH₂- |
| 805 | C₆H₅ | s-C₄H₉ | -CH₂CH₂CH₂- |
| 806 | C₆H₅ | C(CH₃)₃ | -CH₂CH₂CH₂- |
| 807 | C₆H₅ | CH₂CH₂CH₂CH₂CH₃ | -CH₂CH₂CH₂- |
| 808 | C₆H₅ | CH₂CH₂CH₂CH₂CH₂CH₃ | -CH₂CH₂CH₂- |
| 809 | C₆H₅ | Cyclopentyl | -CH₂CH₂CH₂- |
| 810 | C₆H₅ | Cyclohexyl | -CH₂CH₂CH₂- |
| 811 | C₆H₅ | Allyl | -CH₂CH₂CH₂- |
| 812 | C₆H₅ | But-2-en-1-yl | -CH₂CH₂CH₂- |
| 813 | C₆H₅ | 4-Chlor-but-2-en-1-yl | -CH₂CH₂CH₂- |
| 814 | C₆H₅ | Propargyl | -CH₂CH₂CH₂- |
| 815 | C₆H₅ | C₆H₅ | -CH₂CH₂CH₂- |
| 816 | C₆H₅ | C₆H₅CH₂ | -CH₂CH₂CH₂- |
| 817 | C₆H₅ | 2-Phenyleth-1-yl | -CH₂CH₂CH₂- |
| 818 | C₆H₅ | 4-Cl-C₆H₄ | -CH₂CH₂CH₂- |
| 819 | C₆H₅ | 4-F-C₆H₄ | -CH₂CH₂CH₂- |

s-C₄H₉: -CH(CH₃)(C₂H₅);
i-C₄H₉: -CH₂CH(CH₃)₂;
Allyl: -CH₂CH=CH₂;
Propargyl: -CH₂C≡CH;

### Tabelle 1:

Verbindungen der allgemeinen Formel IA, worin A für 2-Chlorphenyl steht und R⁵, R⁶ und (C(R^{3m})(R^{4m}))ₘ für jede einzelne Verbindung jeweils einer Zeile der Tabelle A entsprechen.

### Tabelle 2:

Verbindungen der allgemeinen Formel IA, worin A für 2-Trifluormethylphenyl steht und R⁵, R⁶ und (C(R^{3m})(R^{4m}))ₘ für jede einzelne Verbindung jeweils einer Zeile der Tabelle A entsprechen.

### Tabelle 3:

Verbindungen der allgemeinen Formel IA, worin A für 2-Difluormethylphenyl steht und R⁵, R⁶ und (C(R^{3m})(R^{4m}))ₘ für jede einzelne Verbindung jeweils einer Zeile der Tabelle A entsprechen.

### Tabelle 4:

Verbindungen der allgemeinen Formel IA, worin A für 2-Methylphenyl steht und R⁵, R⁶ und (C(R^{3m})(R^{4m}))ₘ für jede einzelne Verbindung jeweils einer Zeile der Tabelle A entsprechen.

### Tabelle 5:

Verbindungen der allgemeinen Formel IA, worin A für 2-Chlorpyridin-3-yl steht und R⁵, R⁶ und (C(R^{3m})(R^{4m}))ₘ für jede einzelne Verbindung jeweils einer Zeile der Tabelle A entsprechen.

### Tabelle 6:

Verbindungen der allgemeinen Formel IA, worin A für 2-Trifluormethylpyridin-3-yl steht und R⁵, R⁶ und (C(R^{3m})(R^{4m}))ₘ für jede einzelne Verbindung jeweils einer Zeile der Tabelle A entsprechen.

### Tabelle 7:

Verbindungen der allgemeinen Formel lA, worin A für 2-Difluormethylpyridin-3-yl steht und R⁵, R⁶ und (C(R^{3m})(R^{4m}))ₘ für jede einzelne Verbindung jeweils einer Zeile der Tabelle A entsprechen.

### Tabelle 8:

Verbindungen der allgemeinen Formel IA, worin A für 2-Methylpyridin-3-yl steht und R⁵, R⁶ und (C(R^{3m})(R^{4m}))ₘ für jede einzelne Verbindung jeweils einer Zeile der Tabelle A entsprechen.

### Tabelle 9:

Verbindungen der allgemeinen Formel IA, worin A für 4-Methylpyridimidin-5-yl steht und R⁵, R⁶ und (C(R^{3m})(R^{4m}))ₘ für jede einzelne Verbindung jeweils einer Zeile der Tabelle A entsprechen.

### Tabelle 10:

Verbindungen der allgemeinen Formel IA, worin A für 4-Trifluormethylpyrimidin-5-yl steht und R⁵, R⁶ und (C(R^{3m})(R^{4m}))ₘ für jede einzelne Verbindung jeweils einer Zeile der Tabelle A entsprechen.

### Tabelle 11:

Verbindungen der allgemeinen Formel IA, worin A für 4-Difluormethylpyrimidin-5-yl steht und R⁵, R⁶ und (C(R^{3m})(R^{4m}))m für jede einzelne Verbindung jeweils einer Zeile der Tabelle A entsprechen.

### Tabelle 12:

Verbindungen der allgemeinen Formel IA, worin A für 1-Methyl-3-trifluormethylpyrazol-4-yl steht und R⁵, R⁶ und (C(R^{3m})(R^{4m}))ₘ für jede einzelne Verbindung jeweils einer Zeile der Tabelle A entsprechen.

### Tabelle 13:

Verbindungen der allgemeinen Formel IA, worin A für 1-Methyl-3-difluormethylpyrazol-4-yl steht und R⁵, R⁶ und (C(R^{3m})(R^{4m}))m für jede einzelne Verbindung jeweils einer Zeile der Tabelle A entsprechen.

### Tabelle 14:

Verbindungen der allgemeinen Formel IA, worin A für 1,3-Dimethylpyrazol-4-yl steht und R⁵, R⁶ und (C(R^{3m})(R^{4m}))ₘ für jede einzelne Verbindung jeweils einer Zeile der Tabelle A entsprechen.

### Tabelle 15:

Verbindungen der allgemeinen Formel IA, worin A für 1-Methyl-3-trifluormethyl-5-fluorpyrazol-4-yl steht und R⁵, R⁶ und (C(R^{3m})(R^{4m}))ₘ für jede einzelne Verbindung jeweils einer Zeile der Tabelle A entsprechen.

### Tabelle 16:

Verbindungen der allgemeinen Formel IA, worin A für 1-Methyl-3-difluormethyl-5-fluorpyrazol-4-yl steht und R⁵, R⁶ und (C(R^{3m})(R^{4m}))ₘ für jede einzelne Verbindung jeweils einer Zeile der Tabelle A entsprechen.

### Tabelle 17:

Verbindungen der allgemeinen Formel IA, worin A für 1-Methyl-3-trifluormethyl-5-chlor-pyrazol-4-yl steht und R⁵, R⁶ und (C(R^{3m})(R^{4m}))ₘ für jede einzelne Verbindung jeweils einer Zeile der Tabelle A entsprechen.

### Tabelle 18:

Verbindungen der allgemeinen Formel IA, worin A für 1-Methyl-3-trifluormethylpyrol-4-yl steht und R⁵, R⁶ und (C(R^{3m})(R^{4m}))ₘ für jede einzelne Verbindung jeweils einer Zeile der Tabelle A entsprechen.

### Tabelle 19:

Verbindungen der allgemeinen Formel IA, worin A für 1-Methyl-3-difluormethylpyrol-4-yl steht und R⁵, R⁶ und (C(R^{3m})(R^{4m}))m für jede einzelne Verbindung jeweils einer Zeile der Tabelle A entsprechen.

### Tabelle 20:

Verbindungen der allgemeinen Formel IA, worin A für 2-Methyl-4-trifluormethylthiazol-5-yl steht und R⁵, R⁶ und (C(R^{3m})(R^{4m}))m für jede einzelne Verbindung jeweils einer Zeile der Tabelle A entsprechen.

### Tabelle 21:

Verbindungen der allgemeinen Formel IA, worin A für 2-Methyl-4-difluormethylthiazol-5-yl steht und R⁵, R⁶ und (C(R^{3m})(R^{4m}))ₘ für jede einzelne Verbindung jeweils einer Zeile der Tabelle A entsprechen.

### Tabelle 22:

Verbindungen der allgemeinen Formel IA, worin A für 2,4-Dimethylthiazol-5-yl steht und R⁵, R⁶ und (C(R^{3m})(R^{4m}))ₘ für jede einzelne Verbindung jeweils einer Zeile der Tabelle A entsprechen.

### Tabelle 23:

Verbindungen der allgemeinen Formel lA, worin A für 2-Methyl-5-trifluormethylthiazol-4-yl steht und R⁹, R⁶ und (C(R^{3m})(R^{4m}))ₘ für jede einzelne Verbindung jeweils einer Zeile der Tabelle A entsprechen.

### Tabelle 24:

Verbindungen der allgemeinen Formel IA, worin A für 2-Methyl-5-difluormethylthiazol-4-yl steht und R⁵, R⁶ und (C(R^{3m})(R^{4m}))ₘ für jede einzelne Verbindung jeweils einer Zeile der Tabelle A entsprechen.

### Tabelle 25:

Verbindungen der allgemeinen Formel IA, worin A für 2,5-Dimethylthiazol-4-yl steht und R⁵, R⁶ und (C(R^{3m})(R^{4m}))ₘ für jede einzelne Verbindung jeweils einer Zeile der Tabelle A entsprechen.

### Tabelle 26:

Verbindungen der allgemeinen Formel IA, worin A für 2-Methyl-4-trifluormethyloxazol-5-yl steht und R⁵, R⁶ und (C(R^{3m})(R^{4m}))ₘ für jede einzelne Verbindung jeweils einer Zeile der Tabelle A entsprechen.

### Tabelle 27:

Verbindungen der allgemeinen Formel IA, worin A für 2-Methyl-4-difluormethyloxazol-5-yl steht und R⁵, R⁶ und (C(R^{3m})(R^{4m}))ₘ für jede einzelne Verbindung jeweils einer Zeile der Tabelle A entsprechen.

### Tabelle 28:

Verbindungen der allgemeinen Formel IA, worin A für 2,4-Dimethyloxazol-5-yl steht und R⁵, R⁶ und (C(R^{3m})(R^{4m}))ₘ für jede einzelne Verbindung jeweils einer Zeile der Tabelle A entsprechen.

### Tabelle 29:

Verbindungen der allgemeinen Formel IA, worin A für 2-Trifluormethylthiophen-3-yl steht und R⁵, R⁶ und (C(R^{3m})(R^{4m}))ₘ für jede einzelne Verbindung jeweils einer Zeile der Tabelle A entsprechen.

### Tabelle 30:

Verbindungen der allgemeinen Formel IA, worin A für 5-Methyl-2-trifluormethylthiophen-3-yl steht und R⁵, R⁶ und (C(R^{3m})(R^{4m}))ₘ für jede einzelne Verbindung jeweils einer Zeile der Tabelle A entsprechen.

### Tabelle 31:

Verbindungen der allgemeinen Formel lA, worin A für 2-Methylthiophen-3-yl steht und R⁵, R⁶ und (C(R^{3m})(R^{4m}))ₘ für jede einzelne Verbindung jeweils einer Zeile der Tabelle A entsprechen.

### Tabelle 32:

Verbindungen der allgemeinen Formel IA, worin A für 2,5-Dimethylthiophen-3-yl steht und R⁵, R⁶ und (C(R^{3m})(R^{4m}))ₘ für jede einzelne Verbindung jeweils einer Zeile der Tabelle A entsprechen.

### Tabelle 33:

Verbindungen der allgemeinen Formel IA, worin A für 3-Trifluormethylthiophen-2-yl steht und R⁵, R⁶ und (C(R^{3m})(R^{4m}))ₘ für jede einzelne Verbindung jeweils einer Zeile der Tabelle A entsprechen.

### Tabelle 34:

Verbindungen der allgemeinen Formel IA, worin A für 3-Methylthiophen-2-yl steht und R⁵, R⁶ und (C(R^{3m})(R^{4m}))ₘ für jede einzelne Verbindung jeweils einer Zeile der Tabelle A entsprechen.

### Tabelle 35:

Verbindungen der allgemeinen Formel IA, worin A für 3,5-Dimethylthiophen-2-yl steht und R⁵, R⁶ und (C(R^{3m})(R^{4m}))ₘ für jede einzelne Verbindung jeweils einer Zeile der Tabelle A entsprechen.

### Tabelle 36:

Verbindungen der allgemeinen Formel IA, worin A für 5-Methyl-3-trifluormethylthiophen-2-yl steht und R⁵, R⁶ und (C(R^{3m})(R^{4m}))ₘ für jede einzelne Verbindung jeweils einer Zeile der Tabelle A entsprechen.

### Tabelle 37:

Verbindungen der allgemeinen Formel IA, worin A für 2-Trifluormethyfuran-3-yl steht und R⁵, R⁶ und (C(R^{3m})(R^{4m}))ₘ für jede einzelne Verbindung jeweils einer Zeile der Tabelle A entsprechen.

### Tabelle 38:

Verbindungen der allgemeinen Formel IA, worin A für 5-Methyl-2-trifluormethyfuran-3-yl steht und R⁵, R⁶ und (C(R3^{m})(R4^{m}))ₘ für jede einzelne Verbindung jeweils einer Zeile der Tabelle A entsprechen.

### Tabelle 39:

Verbindungen der allgemeinen Formel IA, worin A für 2-Methylfuran-3-yl steht und R⁵, R⁶ und (C(R^{3m})(R^{4m}))ₘ für jede einzelne Verbindung jeweils einer Zeile der Tabelle A entsprechen.

### Tabelle 40:

Verbindungen der allgemeinen Formel IA, worin A für 2,5-Dimethylfuran-3-yl steht und R⁵, R⁶ und (C(R^{3m})(R^{4m}))ₘ für jede einzelne Verbindung jeweils einer Zeile der Tabelle A entsprechen.

### Tabelle 41:

Verbindungen der allgemeinen Formel IA, worin A für 2-Methyl-5,6-dihydro-[1,4]oxathiin-3-yl steht und R⁵, R⁶ und (C(R^{3m})(R^{4m}))ₘ für jede einzelne Verbindung jeweils einer Zeile der Tabelle A entsprechen.

### Tabelle 42:

Verbindungen der allgemeinen Formel lA, worin A für 2-Methyl-5,6-dihydro-4H-thiopyran-3-yl steht und R⁵, R⁶ und (C(R^{3m})(R^{4m}))ₘ für jede einzelne Verbindung jeweils einer Zeile der Tabelle A entsprechen.

Die erfindungsgemäßen Verbindungen der Formel I können in Analogie zu an sich bekannten Verfahren aus dem Stand der Technik hergestellt werden, beispielsweise gemäß Schema 1 durch Umsetzung aktivierter (Heterocyclyl)carbonsäurederivate II mit einem Anilin III [Houben-Weyl: "Methoden der organ. Chemie", Georg-Thieme-Verlag, Stuttgart, New York 1985, Band E5, S. 941-1045.]. Aktivierte Carbonsäurederivate II sind beispielsweise Halogenide, Aktivester, Anhydride, Azide, z.B. Chloride, Fluoride, Bromide, para-Nitrophenylester, Pentafluorphenylester, N-Hydroxysuccinimidester, Hydroxybenzotriazol-1-yl-ester. In Schema 1 weisen die Reste A, Y, R¹, R², R^{3m}, R^{4m}, R⁵, R⁶, n und m die zuvor genannten Bedeutungen und insbesondere die als bevorzugt genannten Bedeutungen auf.

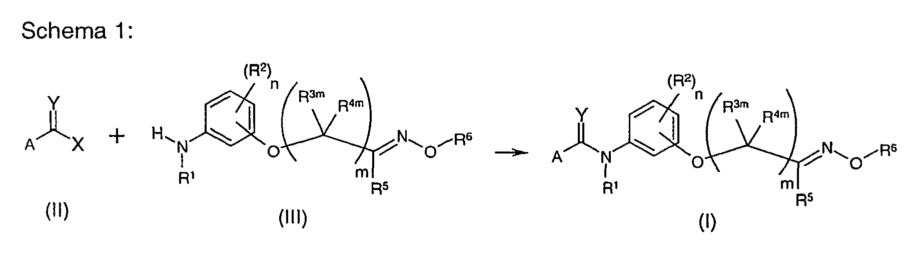

Die Wirkstoffe I können beispielsweise auch durch Umsetzung der Säuren IV mit einem Anilin III in Gegenwart eines Kupplungsreagenzes gemäß Schema 2 hergestellt werden. In Schema 2 weisen die Reste A, Y, R¹, R², R^{3m} , R^{4m}, R⁵, R⁶, n und m die zuvor genannten Bedeutungen und insbesondere die als bevorzugt genannten Bedeutungen auf.

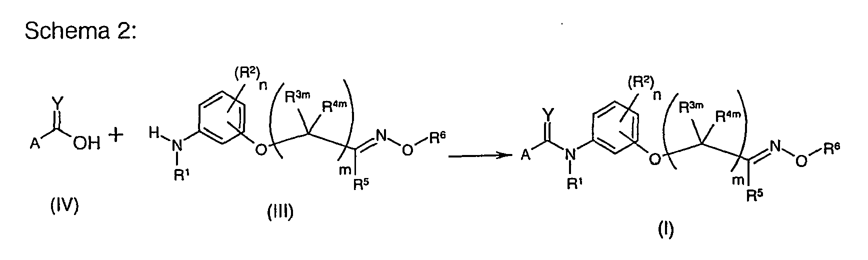

Geeignete Kupplungsreagenzien sind beispielsweise:
- Kupplungsreagenzien auf Carbodiimid-Basis, z.B. N,N'-Dicyclohexylcarbodiimid [J.C. Sheehan, G.P. Hess, J. Am. Chem. Soc. 1955, 77, 1067], N-(3-Dimethylaminopropyl)-N'-ethyl-carbodiimid;
- Kupplungsreagenzien, die gemischte Anhydride mit Kohlensäureestern bilden, z.B. 2-Ethoxy-1-ethoxycarbonyl-1,2-dihydrochinolin [B. Belleau, G. Malek, J. A-mer. Chem. Soc. 1968, 90, 1651.], 2-iso-Butyloxy-1-iso-butyloxycarbonyl-1,2-dihydrochinolin [Y. Kiso, H. Yajima, J. Chem. Soc., Chem. Commun. 1972, 942.];
- Kupplungsreagenzien auf Phosphonium-Basis, z.B. (Benzotriazol-1-yloxy)-tris-(dimethylamino)-phosophonium-hexafluorophosphat [B. Castro, J.R. Domoy, G. Evin, C. Selve, Tetrahedron Lett. 1975, 14, 1219.], (Benzotriazol-1-yl-oxy)-tripyrrolidinophosphonium-hexafluorophosphat [J. Coste et.al., Tetrahedron Lett. 1990, 31, 205.];
- Kupplungsreagenzien auf Uroniumbasis bzw. mit Guanidinium-N-oxid-Struktur, z.B. N,N,N',N'-Tetramethyl-O-(l H-benzotriazol-1 -yl)-uronium-hexafluorophosphat [R. Knorr, A. Trzeciak, W. Bannwarth, D. Gillessen, Tetrahedron Lett. 1989, 30, 1927. ], N,N,N',N'-Tetramethyl-O-(benzotriazol-1-yl)-uronium-tetrafluoroborat, (Benzotriazol-1-yloxy)-dipiperidinocarbenium-hexafluorophosphat [S. Chen, J. Xu, Tetrahedron Lett. 1992, 33, 647.];
- Kupplungsreagenzien, die Säurechloride bilden, z.B. Phosphorsäure-bis-(2-oxo-oxazolidid)-chlorid [J. Diago-Mesequer, Synthesis 1980, 547.].

Verbindungen 1 mit R¹ = gegebenenfalls mit Halogen substituiertes Alkyl oder gegebenenfalls substituiertes Cycloalkyl können auch durch Alkylierung der Amide I (worin R¹ für Wasserstoff steht und die gemäß Schema 1 oder 2 zugänglich sind) mit geeigneten Alkylierungsmitteln in Gegenwart von Basen hergestellt werden, siehe Schema 3.

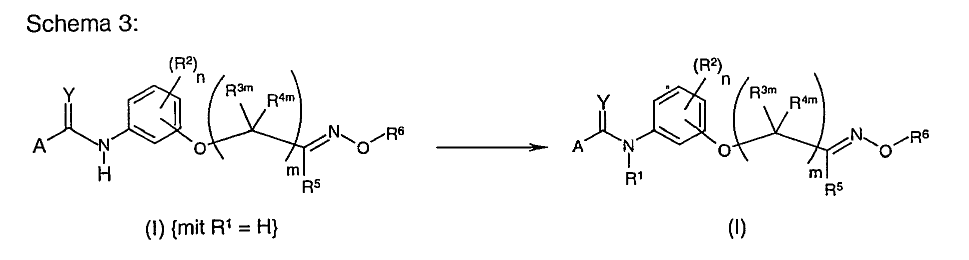

Die (Heterocyclyl)carbonsäuren IV können nach literaturbekannten Verfahren hergestellt werden und daraus sind die (Heterocyclyl)carbonsäuren-Derivate II nach literaturbekannten Verfahren herstellbar [beispielsweise EP 0589313, EP 915868, US 4,877,441].

Die Aniline III können beispielsweise gemäß dem Schema 4 hergestellt werden. In Schema 4 weisen die Reste R¹, R², R^{3m}, R^{4m}, R⁵, R⁶, n und m die zuvor genannten Bedeutungen und insbesondere die als bevorzugt genannten Bedeutungen auf. Die Verbindungen V und X sind literaturbekannt oder können nach literaturbekannten Verfahren hergestellt werden.

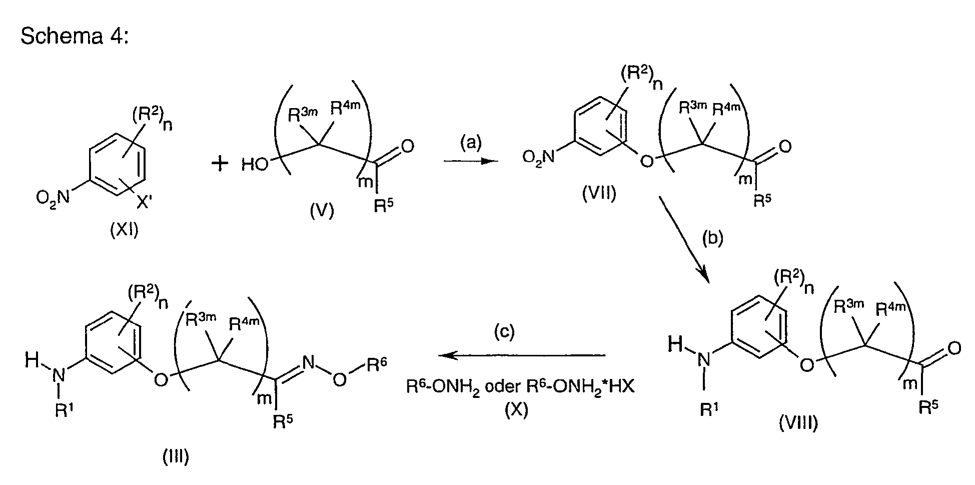

In Schritt a in Schema 4 setzt man den Nitroaromaten Xl, worin X' für Halogenid, beispielsweise Chlorid oder Fluorid steht, mit einem Ketoalkohol V im Sinne einer nucleophilen aromatischen Substitution um, wobei man den Nitrophenylether VII erhält. Die Umsetzung erfolgt in Anlehnung an bekannte Verfahren, beispielsweise nach Organikum, 21. Auflage, Wiley-VCH 2001, S. 394ff. S. Raeppel, F. Raeppel, J. Suffert; Synlett [SYNLES] 1998, (7), 794-796. R. Beugelmans, A. Bigot, J. Zhu; Tetrahedron Lett [TE-LEAY] 1994, 35 (31), 5649-5652. Die Umsetzung erfolgt üblicherweise in Gegenwart einer Base. Geeignete Basen sind Alkalimetallcarbonate, Erdalkalimetallcarbonate wie Natriumcarbonat, Kaliumcarbonat, Calciumcarbonat, Magnesiumcarbonat, Alkalimetallhydroxide oder Erdalkalimetallhydroxide wie Natriumhydroxid oder Kaliumhydroxid. In der Regel führt man die Umsetzung in einem inerten organischen Lösungsmittel durch. Als Lösungsmittel kommen Ether wie Diethylether, Methyl-tert-butylether, Dioxan, Tetrahydrofuran, Ethylenglycoldimethylether, Diethylenglycol in Betracht.

In Schritt b reduziert man den Nitrophenylether VII zum Aminophenylether VIII, beispielsweise wie im Organikum, 21. Auflage, Wiley-VCH 2001, S. 627ff beschrieben. Die katalytische Reduktion der Nitrogruppe des Nitrophenylethers VII erfolgt in der Regel mit Hydrazin als Wasserstoffquelle und in Gegenwart von Raney-Nickel als Katalysator. Die Reduktion erfolgt in der Regel in einem inerten Lösungsmittel, beispielsweise in einem C₁-C₄-Alkohol wie Methanol oder Ethanol. Die Reduktion des Nitrophenylethers VII zum Aminophenylether VIII kann beispielsweise durch Umsetzung des Nitrophenylethers VII mit einer Metallverbindung wie Zinn(II)-chlorid unter sauren Reaktionsbedingungen wie konzentrierter Salzsäure erfolgen.

In Schritt c setzt man den Aminophenylether VIII mit einem Hydroxylamin X beziehungsweise dem Säureadditionssalz davon, vorzugsweise das Hydrochloridsalz, um. Die Umsetzung erfolgt in der Regel in einem Lösungsmittel. Geeignete Lösungsmittel sind beispielsweise C₁-C₄-Alkohole oder C₁-C₄-Alkohol/Wasser-Gemische. Die Umsetzung kann in Gegenwart einer Base stattfinden. Geeignete Basen sind aromatische Amine wie Pyridin oder Alkalimetallhydroxide oder Erdalkalimetallhydroxide wie Natriumhydroxid, Kaliumhydroxid oder Calciumhydroxid. Die Oximierung der Ketogruppe in X kann beispielsweise in Anlehnung an Organikum, 21. Auflage, Wiley-VCH 2001, S. 467 oder D. Dhanak, C. Reese, S. Romana, G. Zappia, J. Chem. Soc. Chem. Comm. 1986 (12), 903-904, DE 3004871 oder AU 580091 erfolgen.

Alternativ können die Aniline III auch gemäß Schema 5 hergestellt werden. In Schema 5 weisen die Reste R¹, R², R^{3m} , R^{4m}, R⁵, R⁶, X', n und m die zuvor genannten Bedeutungen und insbesondere die als bevorzugt genannten Bedeutungen auf.

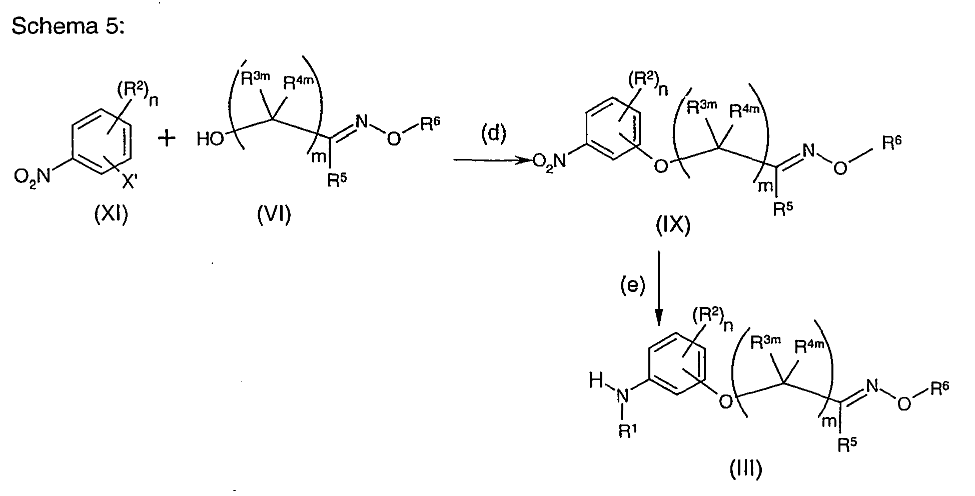

Schritt d in Schema 5 erfolgt analog zu Schritt a in Schema 4. Schritt e in Schema 5 erfolgt analog zu Schritt b in Schema 4.

Das Oxim IX ist auch durch Umsetzung des Nitrophenylethers VII mit dem Hydroxylamin X oder dem Säureadditionssalz von X in Anlehnung an das in Schritt a in Schema 4 beschriebene Verfahren erhältlich.

Das Oxim VI ist beispielsweise durch Umsetzung des Ketoalkohols V mit dem Hydroxylamin X oder dem Säureadditionssalz von X in Anlehnung an das in Schritt a in Schema 4 beschriebene Verfahren erhältlich.

Die erfindungsgemäßen Verbindungen 1 können auch gemäß Schema 6 hergestellt werden. In Schema 6 weisen die Reste A, Y, R¹, R², R^{3m}, R^{4m}, R⁵, R⁶, n und m die zuvor genannten Bedeutungen und insbesondere die als bevorzugt genannten Bedeutungen auf, Hal, Hal' stehen unabhängig voneinander für Halogen, beispielsweise für Chlorid, Bromid oder lodid.

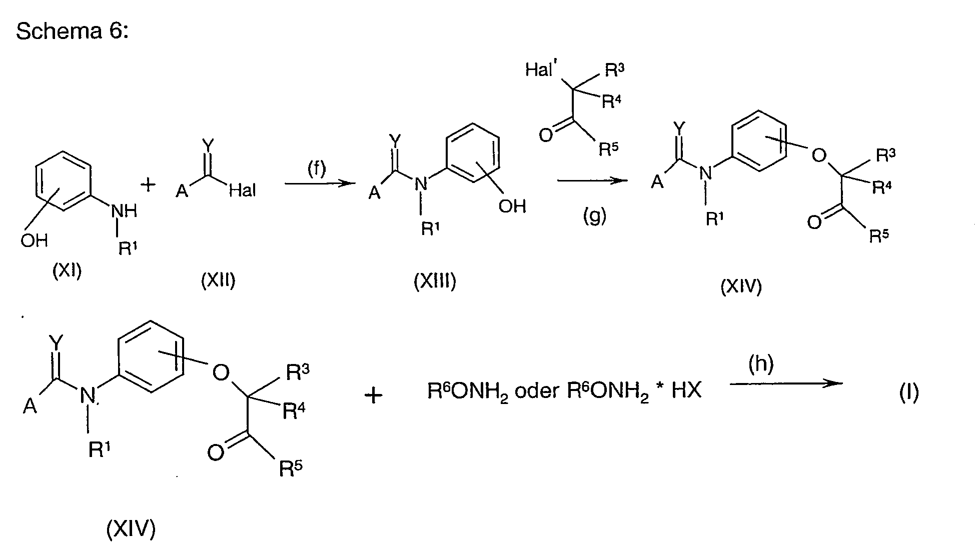

In Schritt f in Schema 6 setzt man das Aminophenol XI mit einem (Heterocyclyl)carbonsäurehalogenid XII um, wobei man das Anilid XIII erhält. Üblicherweise führt man die Umsetzung in Gegenwart einer Base durch, beispielsweise ein tertiäres Amin wie Trimethylamin oder Triethylamin. In der Regel führt man die Umsetzung in einem inerten organischen Lösungsmittel durch. Als Lösungsmittel kommen beispielsweise Ether wie Diethylether, Methyl-tert-butylether, Dioxan, Tetrahydrofuran, Ethylenglycoldimethylether, Diethylenglycol oder chlorierte Kohlenwasserstoffe wie Dichlormethan, Dichlorethan oder Trichlormethan in Betracht.

Die Umsetzung des Anilids XIII mit dem Keton XIV in Schritt g in Schema 6 kann in Gegenwart einer Base erfolgen. Geeignete Basen sind Alkalimetallcarbonate, Erdalkalimetallcarbonate wie Natriumcarbonat, Kaliumcarbonat, Calciumcarbonat, Magnesiumcarbonat, Alkalimetallhydroxide oder Erdalkalimetallhydroxide wie Natriumhydroxid oder Kaliumhydroxid. In der Regel führt man die Umsetzung in einem inerten organischen Lösungsmittel durch. Als Lösungsmittel kommen beispielsweise Carbonsäureamide wie N,N-Dimethylformamid, Diethylformamid oder Dimethylacetamid in Betracht.

Die Umwandlung der Verbindung XIV in die Verbindung I in Schritt h in Schema 6 erfolgt beispielsweise in Anlehnung an Schritt c in Schema 4.

Die Verbindungen I eignen sich als Fungizide. Sie zeichnen sich durch eine hervorragende Wirksamkeit gegen ein breites Spektrum von pflanzenpathogenen Pilzen, insbesondere aus der Klasse der *Ascomyceten, Deuteromyceten, Phycomyceten* und *Basidiomyceten,* aus. Sie sind zum Teil systemisch wirksam und können im Pflanzenschutz als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Bananen, Baumwolle, Soja, Kaffee, Zuckerrohr, Wein, Obst- und Zierpflanzen und Gemüsepflanzen wie Gurken, Bohnen, Tomaten, Kartoffeln und Kürbisgewächsen, sowie an den Samen dieser Pflanzen.

Speziell eignen sie sich zur Bekämpfung folgender Pflanzenkrankheiten:
- *Alternaria-*Arten an Gemüse und Obst,
- *Botrytis cinerea* (Grauschimmel) an Erdbeeren, Gemüse, Zierpflanzen und Reben,
- Cercospora arachidicola an Erdnüssen,
- Erysiphe cichoracearum und Sphaerotheca fuliginea an Kürbisgewächsen,
- Erysiphe graminis (echter Mehltau) an Getreide,
- Fusarium- und Verticillium-Arten an verschiedenen Pflanzen,
- Helminthosporium-Arten an Getreide,
- Mycosphaerella-Arten an Bananen und Erdnüssen,
- Phytophthora infestans an Kartoffeln und Tomaten,
- Plasmopara viticola an Reben,
- Podosphaera leucotricha an Äpfeln,
- Pseudocercosporella herpotrichoides an Weizen und Gerste,
- Pseudoperonospora-Arten an Hopfen und Gurken,
- Puccinia-Arten an Getreide,
- Pyricularia oryzae an Reis,
- Rhizoctonia-Arten an Baumwolle, Reis und Rasen,
- Septoria nodorum an Weizen,
- Sphaerotheca fuliginea (Gurkenmehltau) an Gurken,
- Uncinula necator an Reben,
- Ustilago-Arten an Getreide und Zuckerrohr, sowie
- Venturia-Arten (Schorf) an Äpfeln und Birnen.
- Septoria tritici
- Pyrenophora-Arten
- Leptosphaeria nodorum
- Rhynchosporium-Arten
- Typhula-Arten

Die Verbindungen eignen sich außerdem zur Bekämpfung von Schadpilzen wie *Paecilomyces variotii* im Materialschutz (z.B. Holz, Papier, Dispersionen für den Anstrich, Fasern bzw. Gewebe) und im Vorratsschutz.

Die Verbindungen I werden angewendet, indem man die Pilze oder die vor Pilzbefall zu schützenden Pflanzen, Saatgüter, Materialien oder den Erdboden mit einer fungizid wirksamen Menge der Wirkstoffe behandelt. Die Anwendung kann sowohl vor als auch nach der Infektion der Materialien, Pflanzen oder Samen durch die Pilze erfolgen.

Die fungiziden Mittel enthalten im allgemeinen zwischen 0,1 und 95, vorzugsweise zwischen 0,5 und 90 Gew.% Wirkstoff.

Die Aufwandmengen liegen bei der Anwendung im Pflanzenschutz je nach Art des gewünschten Effektes zwischen 0,01 und 2,0 kg Wirkstoff pro ha.

Bei der Saatgutbehandlung werden im allgemeinen Wirkstoffmengen von 0,001 bis 0,1 g; vorzugsweise 0,01 bis 0,05 g je Kilogramm Saatgut benötigt.

Bei der Anwendung im Material- bzw. Vorratsschutz richtet sich die Aufwandmenge an Wirkstoff nach der Art des Einsatzgebietes und des gewünschten Effekts. Übliche Aufwandmengen sind im Materialschutz beispielsweise 0,001 g bis 2 kg, vorzugsweise 0,005 g bis 1 kg Wirkstoff pro Qubikmeter behandelten Materials.

Die Verbindungen I können in die üblichen Formulierungen überführt werden, z.B. Lösungen, Emulsionen, Suspensionen, Stäube, Pulver, Pasten und Granulate. Die Anwendungsform richtet sich nach dem jeweiligen Verwendungszweck; sie soll in jedem Fall eine feine und gleichmäßige Verteilung der erfindungsgemäßen Verbindung gewährleisten.

Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können. Als Hilfsstoffe kommen dafür im wesentlichen in Betracht: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B.Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Lignin-Sulfitablaugen und Methylcellulose.

Als oberflächenaktive Stoffe kommen Alkali-, Erdalkali-, Ammoniumsalze von Ligninsulfonsäure, Naphthalinsulfonsäure, Phenolsulfonsäure, Dibutylnaphthalinsulfonsäure, Alkylarylsulfonate, Alkylsulfate, Alkylsulfonate, Fettalkoholsulfate und Fettsäuren sowie deren Alkali- und Erdalkalisalze, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphtalinsulfonsäure mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Alkylarylpolyetheralkohole, lsotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Ligninsulfitablaugen und Methylbellulose in Betracht.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, Methanol, Ethanol, Propanol, Butanol, Chloroform, Tetrachlorkohlenstoff, Cyclohexanol, Cyclohexanon, Chlorbenzol, Isophoron, stark polare Lösungsmittel, z.B. Dimethylformamid, Dimethylsulfoxid, N-Methylpyrrolidon, Wasser, in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, lmprägnierungs- und Homogengranulate, können durch Bindung der Wirkstoffe an feste Trägerstoffe hergestellt werden. Feste Trägerstoffe sind z.B. Mineralerden, wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten im allgemeinen zwischen 0,01 und 95 Gew.%, vorzugsweise zwischen 0,1 und 90 Gew.-% des Wirkstoffs. Die Wirkstoffe werden dabei in einer Reinheit von 90% bis 100%, vorzugsweise 95% bis 100% (nach NMR-Spektrum) eingesetzt.

Beispiele für Formulierungen sind:
I. 5 Gew.-Teile einer erfindungsgemäßen Verbindung werden mit 95 Gew.-Teilen feinteiligem Kaolin innig vermischt. Man erhält auf diese Weise ein Stäubemittel, das 5 Gew.-% des Wirkstoffs enthält.
II. 30 Gew.-Teile einer erfindungsgemäßen Verbindung werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit (Wirkstoffgehalt 23 Gew.-%).
III. 10 Gew.-Teile einer erfindungsgemäßen Verbindung werden in einer Mischung gelöst, die aus 90 Gew.-Teilen Xylol, 6 Gew.-Teilen des Anlagerungsproduktes von 8 bis 10 Mol Ethylenoxid an 1 Mol Ölsäure-N-monoethanolamid, 2 Gew.-Teilen Calciumsalz der Dodecylbenzolsulfonsäure und 2 Gew.-Teilen des Anlagerungsproduktes von 40 Mol Ethylenoxid an 1 Mol Ricinusöl besteht (Wirkstoffgehalt 9 Gew.-%).
IV. 20 Gew.-Teile einer erfindungsgemäßen Verbindung werden in einer Mischung gelöst, die aus 60 Gew.-Teilen Cyclohexanon, 30 Gew.-Teilen Isobutanol, 5 Gew.-Teilen des Anlagerungsproduktes von 7 Mol Ethylenoxid an 1 Mol Isooctylphenol und 5Gew.-Teilen des Anlagerungsproduktes von 40 Mol Ethylenoxid an 1 Mol Ricinusöl besteht (Wirkstoffgehalt 16 Gew.-%).
V. 80 Gew.-Teile einer erfindungsgemäßen Verbindung werden mit 3 Gew.-Teilen des Natriumsalzes der Diisobutylnaphthalin-alpha-sulfonsäure, 10 Gew.-Teilen des Natriumsalzes einer Ligninsulfonsäure aus einer Sulfit-Ablauge und 7 Gew.-Teilen pulverförmigem Kieselsäuregel gut vermischt und in einer Hammermühle vermahlen (Wirkstoffgehalt 80 Gew.-%).
VI. Man vermischt 90 Gew.-Teile einer erfindungsgemäßen Verbindung mit 10 Gew.-Teilen N-Methyl-a-pyrrolidon und erhält eine Lösung, die zur Anwendung in Form kleinster Tropfen geeignet ist (Wirkstoffgehalt 90 Gew.-%).
VII. 20 Gew.-Teile einer erfindungsgemäßen Verbindung werden in einer Mischung gelöst, die aus 40 Gew.-Teilen Cyclohexanon, 30 Gew.-Teilen Isobutanol, 20 Gew.-Teilen des Anlagerungsproduktes von 7 Mol Ethylenoxid an 1 Mol Isooctylphenol und 10 Gew.-Teilen des Anlagerungsproduktes von 40 Mol Ethylenoxid an 1 Mol Ricinusöl besteht. Durch Eingießen und feines Verteilen der Lösung in 100 000 Gew.-Teilen Wasser erhält man eine wäßrige Dispersion, die 0,02 Gew.-% des Wirkstoffs enthält.
VIII. 20 Gew.-Teile einer erfindungsgemäßen Verbindung werden mit 3 Gew.-Teilen des Natriumsalzes der Diisobutylnaphthalin-α-sulfonsäure, 17 Gew.-Teilen des Natriumsalzes einer Ligninsulfonsäure aus einer Sulfit-Ablauge und 60 Gew.-Teilen pulverförmigem Kieselsäuregel gut vermischt und in einer Hammermühle vermahlen. Durch feines Verteilen der Mischung in 20 000 Gew.-Teilen Wasser erhält man eine Spritzbrühe, die 0,1 Gew.-% des Wirkstoffs enthält.
IX. 10 Gew.-Teile der erfindungsgemäßen Verbindung werden in 63 Gew.-Teilen Cyclohexanon, 27 Gew.-Teilen Dispergiermittel (beispielsweise eine Mischung aus 50 Gew.-Teilen des Anlagerungsprodukts von 7 Mol Ethylenoxid an 1 Mol Isooctylphenol und 50 Gew.-Teilen des Anlagerungsprodukts von 40 Mol Ethylenoxid an 1 Mol Ricinusöl) gelöst. Die Stammlösung wird anschließend durch Verteilen in Wasser auf die gewünschte Konzentration verdünnt, z.B. auf eine Konzentration im Bereich von 1 bis 100 ppm.

Die Wirkstoffe können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Wässrige Anwendungsformen können aus Emulsionskonzentraten, Pasten oder netzbaren Pulvern (Spritzpulver, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermitttel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Die Wirkstoffkonzentrationen in den anwendungsfertigen Zubereitungen können in größeren Bereichen variiert werden. Im allgemeinen liegen sie zwischen 0,0001 und 10%. Häufig reichen bereits geringe Wirkstoffmengen an Verbindung I in der anwendungsfertigen Zubereitung aus, z.B. 2 bis 200 ppm. Ebenso sind anwendungsfertige Zubereitungen mit Wirkstoffkonzentrationen im Bereich von 0,01 bis 1 % bevorzugt.

Die Wirkstoffe können auch mit gutem Erfolg im Ultra-Low-Volume-Verfahren (ULV) verwendet werden, wobei es möglich ist, Formulierungen mit mehr als 95 Gew.-% Wirkstoff oder sogar den Wirkstoff ohne Zusätze auszubringen.

Zu den Wirkstoffen können Öle verschiedenen Typs, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1:10 bis 10:1 zugemischt werden.

Die erfindungsgemäßen Mittel können in der Anwendungsform als Fungizide auch zusammen mit anderen Wirkstoffen vorliegen, der z.B. mit Herbiziden, Insektiziden, Wachstumsregulatoren, Fungiziden oder auch mit Düngemitteln. Beim Vermischen der Verbindungen I bzw. der sie enthaltenden Mittel in der Anwendungsform als Fungizide mit anderen Fungiziden erhält man in vielen Fällen eine Vergrößerung des fungiziden Wirkungsspektrums.

Die folgende Liste von Fungiziden, mit denen die erfindungsgemäßen Verbindungen gemeinsam angewendet werden können, soll die Kombinationsmöglichkeiten erläutern, nicht aber einschränken:
- Schwefel, Dithiocarbamate und deren Derivate, wie Ferridimethyldithiocarbamat, Zinkdimethyldithiocarbamat, Zinkethylenbisdithiocarbamat, Manganethylenbisdithiocarbamat, Mangan-Zink-ethylendiamin-bis-dithiocarbamat, Tetramethylthiuramdisulfide, Ammoniak-Komplex von Zink-(N,N-ethylen-bis-dithiocarbamat), Ammoniak-Komplex von Zink-(N,N'-propylen-bis-dithiocarbamat), Zink-(N,N'-propylenbis-dithiocarbamat), N,N'-Polypropylen-bis-(thiocarbamoyl)disulfid;
- Nitroderivate, wie Dinitro-(1-methylheptyl)-phenylcrotonat, 2-sec-Butyl-4,6-dinitrophenyl-3,3-dimethylacrylat, 2-sec-Butyl-4,6-dinitrophenyl-isopropylcarbonat, 5-Nitro-isophthalsäure-di-isopropylester;
- heterocyclische Substanzen, wie 2-Heptadecyl-2-imidazolin-acetat, 2,4-Dichlor-6-(o-chloranilino)-s-triazin, O,O-Diethyl-phthalimidophosphonothioat, 5-Amino-1-[bis-(dimethylamino)-phosphinyl]-3-phenyl-1,2,4- triazol, 2,3-Dicyano-1,4-dithioanthrachinon, 2-Thio-1,3-dithiolo[4,5-b]chinoxalin, 1-(Butylcarbamoyl)-2-benzimidazol-carbaminsäuremethylester, 2-Methoxycarbonylaminobenzimidazol, 2-(Furyl-(2))-benzimidazol, 2-(Thiazolyl-(4))-benzimidazol, N-(1,1,2,2-Tetrachlorethylthio)-tetrahydrophthalimid, N-Trichlormethylthiotetrahydrophthalimid, N-Trichlormethylthio-phthalimid,
- N-Dichlorfluormethylthio-N',N'-dimethyl-N-phenyl-schwefelsäure- diamid, 5-Ethoxy-3-trichlormethyl-1,2,3-thiadiazol, 2-Rhodanmethylthiobenzthiazol, 1,4-Dichlor-2,5-dimethoxybenzol, 4-(2-Chlorphenylhydrazono)-3-methyl-5-isoxazolon, Pyridin-2-thio-1-oxid, 8-Hydroxychinolin bzw. dessen Kupfersalz, 2,3-Dihydro-5-carboxanilido-6-methyl-1,4-oxathün, 2,3-Dihydro-5-carboxanilido-6-methyl-1,4-oxathiin-4,4-dioxid, 2-Methyl-5,6-dihydro-4H-pyran-3-carbonsäure-anilid, 2-Methyl-furan-3-carbonsäureanilid, 2,5-Dimethyl-furan-3-carbonsäureanilid, 2,4,5-Trimethyl-furan-3-carbonsäureanilid, 2,5-Dimethyl-furan-3-carbonsäurecyclohexylamid, N-Cyclohexyl-N-methoxy-2,5-dimethyl-furan-3-carbonsäureamid, 2-Methyl-benzoesäure-anilid, 2-lod-benzoesäure-anilid, N-Formyl-N-morpholin-2,2,2-trichlorethylacetal, Piperazin-1,4-diylbis-1-(2,2,2-trichlorethyl)-formamid, 1-(3,4-Dichloranilino)-1 -formylamino-2,2,2-trichlorethan, 2,6-Dimethyl-N-tridecyl-morpholin bzw. dessen Salze, 2,6-Dimethyl-N-cyclododecyl-morpholin bzw. dessen Salze, N-[3-(p-tert.-Butylphenyl)-2-methylpropyl]-cis-2,6-dimethyl-morpholin, N-[3-(p-tert.-Butylphenyl)-2-methylpropyl]-piperidin, 1-[2-(2,4-Dichlorphenyl)-4-ethyl-1,3-dioxolan-2-yl-ethyl]-1 H-1,2,4-triazol, 1-[2-(2,4-Dichlorphenyl)-4-n-propyl-1,3-dioxolan-2-yl-ethyl]-1 H-1,2,4-triazol, N-(n-Propyl)-N-(2,4,6-trichlorphenoxyethyl)-N'-imidazol-yl-harnstoff, 1-(4-Chlorphenoxy)-3,3-dimethyl-1-(1 H-1,2,4-triazol-1-yl)-2-butanon, 1-(4-Chlorphenoxy)-3,3-dimethyl-1-(1 H-1,2,4-triazol-1-yl)-2-butanol, (2RS,3RS)-1-[3-(2-Chlorphenyl)-2-(4-fluorphenyl)-oxiran-2-ylmethyl]-1 H-1,2,4-triazol, α-(2-Chlorphenyl)- a -(4-chlorphenyl)-5-pyrimidin-methanol, 5-Butyl-2-dimethylamino-4-hydroxy-6-methyl-pyrimidin, Bis-(p-chlorphenyl)-3-pyridinmethanol, 1,2-Bis-(3-ethoxycarbonyl-2-thioureido)-benzol, 1,2-Bis-(3-methoxycarbonyl-2-thioureido)-benzol,
- Strobilurine wie Methyl-E-methoxyimino-[α -(o-tolyloxy)-o-tolyl]acetat, Methyl-E-2-{2-[6-(2-cyanophenoxy)-pyrimidin-4-yloxy]-pheny)}-3-methoxyacrylat, Methyl-E-methoxyimino-[α-(2-phenoxyphenyl)]-acetamid, Methyl-E-methoxyimino-[α -(2,5-dimethylphenoxy)-o-tolyl]-acetamid,
- Anilinopyrimidine wie N-(4,6-Dimethylpyrimidin-2-yl)-anilin, N-[4-Methyl-6-(1-propinyl)-pyrimidin-2-yl]-anilin, N-[4-Methyl-6-cyclopropyl-pyrimidin-2-yl]-anilin,
- Phenylpyrrole wie 4-(2,2-Difluor-1,3-benzodioxol-4-yl)-pyrrol-3-carbonitril,
- Zimtsäureamide wie 3-(4-Chlorphenyl)-3-(3,4-dimethoxyphenyl)-acrylsäuremorpholid,
- sowie verschiedene Fungizide, wie Dodecylguanidinacetat, 3-[3-(3,5-Dimethyl-2-oxycyclohexyl)-2-hydroxyethyl]-glutarimid, Hexachlorbenzol, DL-Methyl-N-(2,6-dimethyl-phenyl)-N-furoyl(2)-alaninat, DL-N-(2,6-Dimethyl-phenyl)-N-(2'-methoxyacetyl)-alanin-methyl- ester, N-(2,6-Dimethylphenyl)-N-chloracetyl-D,L-2-aminobutyrolacton, DL-N-(2,6-Dimethylphenyl)-N-(phenylacetyl)-alaninmethylester, 5-Methyl-5-vinyl-3-(3,5-dichlorphenyl)-2,4-dioxo-1,3-oxazolidin, 3-[3,5-Dichlorphenyl(-5-methyl-5-methoxymethyl]-1,3-oxazolidin- 2,4-dion, 3-(3,5-Dichlorphenyl)-1-isopropylcarbamoylhydantoin, N-(3,5-Dichlorphenyl)-1,2-dimethylcyclopropan-1,2-dicarbonsäureimid, 2-Cyano-[N-(ethylaminocarbonyl)-2-methoximino]-acetamid, 1-[2-(2,4-Dichlorphenyl)-pentyl]-1 H-1,2,4-triazol, 2,4-Difluor-α-(1 H-1,2,4-triazolyi-1-methyl)-benzhydrylalkohol, N-(3-Chlor-2,6-dinitro-4-trifluormethyl-phenyl)-5-trifluormethyl-3-chlor-2-aminopyridin, 1-((bis-(4-Fluorphenyl)-methylsilyl)-methyl)-1H-1,2,4-triazol.

### Herstellungsbeispiele:

### Beispiel 1:

### 2-Chlor-N-(2-(2-benzyloxyimino-1-methyl-n-propoxy)-phenyl)-nicotinsäureamid

### 1.1 2-Chlor-N-(2-hydroxy-phenyl)-nicotinsäureamid

Zu einer Lösung von 13,1 g ortho-Aminophenol und 24,2 g Triethylamin in 200 ml Dichlormethan gab man bei 10°C eine Lösung von 21 g 2-Chlornicotinsäurechlorid in 100 ml Dichlormethan und rührte 1 Stunde bei 10°C und 60 h bei Raumtemperatur. Danach engte man das Reaktionsgemisch im Vakuum ein und nahm den erhaltenen Rückstand in Ethylacetat auf. Man wusch die organische Phase zweimal mit verd. Salzsäure und 3%iger Natronlauge. Nach dem Trocknen über Natriumsulfat dampfte man das Lösungsmittel im Vakuum ab, wobei man 27.6 g der Titelverbindung mit einem Schmelzpunkt von 142-145°C erhielt.

### 1.2 2-Chlor-N-(2-(1-methyl-2-oxo-n-propoxy)-phenyl)-nicotinsäureamid

Eine Lösung von 1,24 g 2-Chlor-N-(2-hydroxy-phenyl)-nicotinsäureamid, 1,58 g 3-Brombutan-2-on und 0,34 g Kaliumcarbonat in 20 ml N,N-Dimethylformamid rührte man 1 h bei Raumtemperatur und anschließend 2 h bei 60°C. Danach gab man eine Mischung aus Wasser und Ethylacetat zu und trennte die Phasen. Die wässrige Phase extrahierte man zweimal mit Ethylacetat. Die vereinigte organische Phase wurde mit gesättigter NaCl-Lösung gewaschen, über Natriumsulfat getrocknet und im Vakuum eingeengt. Den erhaltenen Rückstand reinigte man chromatographisch an Kieselgel (Eluierungsmittel: Cyclohexan/Methyl-tert-butylether) und erhielt nach dem Abdampfen des Eluierungsmittels 1,0 g der Titelverbindung als Öl.

### 1.3 2-Chlor-N-(2-(2-benzyloxyimino-1-methyl-n-propoxy)-phenyl)nicotinsäureamid

Zu einer Lösung von 0,36 g 2-Chlor-N-(2-(1-methyl-2-oxo-n-propoxy)-phenyl)-nicotinsäureamid und 0,12 g Pyridin in 10 ml Methanol gab man 0,18 g O-Benzylhydroxylamin. Man rührte 15 Minuten bei Raumtemperatur, engte das Lösungsmittel im Vakuum ein und nahm den erhaltenen Rückstand in Methyl-tert-butylether auf. Man wusch das Gemisch mit 1 %iger Salzsäure und ges. NaCl-Lösung, trocknete über Natriumsulfat und engte das Gemisch im Vakuum ein. Die ausgefallenen Kristalle filtrierte man ab und trocknete sie im Vakuum, wobei man 0,3 g der Titelverbindung mit einem Schmelzpunkt von 53-55°C erhielt.

In analoger Weise wurden die in Tabelle 43 angegebenen Verbindungen der Formel IA hergestellt.

**Tabelle 43:**

| | | | | | | |
|---|---|---|---|---|---|---|
| 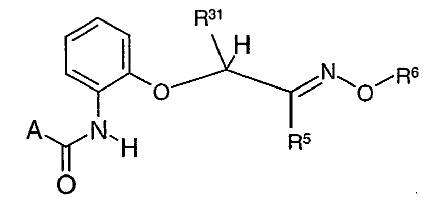 | | | | | | |

| (^{IA}) {mit R¹ = H, n = 0 und R⁴¹ = H} | | | | | | |
|---|---|---|---|---|---|---|
| Nr. | A | R³¹ | R⁵ | R⁶ | Schmp. [°C]; Konsistenz | spektroskopische Daten |
| IA-1 | 2-Chlorpyridin-3-yl | CH₃ | CH₃ | C₆H₅CH₂ | 53-55 | |
| lA-2 | 2-Chlorpyridin-3-yl | CH₃ | CH₃ | Allyl | Öl | *¹H-NMR (CDCl₃), δ[ppm]:* 1.57 (d, 3H); 1.83 (s, 3H); 4.58 (m, 2H); 5.04 (m, 1 H); 5.18-5.31 (m, 2H); 5.93 (m, 1 H); 6.99-7.10 (m, 3H); 7.22 (m, 1 H); 8.18 (m, 1 H); 8.51 (m, 1 H); 9.22 (S_{breit}, 1 H). |
| IA-3 | 2-Chlorpyridin-3-yl | CH₃ | CH₃ | trans-2-Buten-1-yl | Öl | *¹H-NMR (COCl₃), δ[ppm]:* 1.57 (d, 3H); 1.70 (m, 3H); 1.80 (s, 3H); 4.49 (m, 2H); 5.02 (q, 1H); 5.58-5.80 (m, 2H); 6.99-7.10 (m, 3H); 7.22 (m, 1 H); 8.16 (m, 1 H); 8.51 (m, 2H); 9.20 (S_{breit}, 1H). |
| IA-4 | 2-Methyl-4-trifluormethylthiazol-5-yl | CH₃ | CH₃ | CH₃ | Öl | *¹H-NMR (CDCl₃), δ[ppm]*: 1.52 (d, 3H); 1.77 (s, 3H); 2.79 (s, 3H); 3.90 (s, 3H); 5.01 (q, 1 H); 6.93-7.11 (m, 4H); 8.43 (m, 1 H); 8.70 (m, 1 H). |
| IA-5 | 2-Methyl-4-trifluormethylthiazol-5-yl | CH₃ | CH₃ | trans-3-Chlorallyl | Öl | *¹H-NMR (CDCl₃), δ[ppm]:* 1.53 (d, 3H); 1.77 (s, 3H); 2.75 (s, 3H); 4.53 (d, 2H); 5.01 (q, 1 H); 6.07 (m, 1H); 6.20-6.33 (m, 1 H); 6.93-7.11 (m, 3H); 8.45 (m, 1H); 8.84 (S_{breit}, 1H). |
| IA-6 | 1-Methyl-3-trifluormethylpyrazol-4-yl | CH₃ | CH₃ | trans-3-Chlorallyl | Öl | *¹H-NMR (CDCl₃), δ[ppm]:* 1.53 (d, 3H); 1.79 (s, 3H); 3.95 (s, 3H); 4.54 (d, 2H); 5.00 (q, 1 H); 6.08 (m, 1 H); 6.17-6.29 (m, 1 H); 6.96-7.10 (m, 2H); 8.10 (m, 1 H); 8.45 (m, 1 H); 8.59 (S_{breit}, 1 H). |
| IA-7 | 1-Methyl-3-trifluormethylpyrazol-4-yl | CH₃ | CH₃ | CH₃ | 100-102 | |
| IA-8 | 1-Methyl-3-trifluormethylpyrazol-4-yl | CH₃ | CH₃ | C₆H₅CH₂ | Öl | *¹H-NMR (CDCl₃), δ[ppm]:* 1.58 (d, 3H); 1.80 (s, 3H); 3.95 (s, 3H); 4.98 (m, 1 H); 5.17 (s, 2H); 6.82-6.99 (m, 3H); 7.25-7.45 (m, 4H); 8.07 (m, 1 H); 8.46 (m, 1 H); 8.59 (S_{breit}, 1H). |
| IA-9 | 2-Chlorpyridin-3-yl | CH₃ | CH₃ | CH(CH₃)₂ | Öl | *¹H-NMR (CDCl₃), δ[ppm]*: 1.20 (m, 6H); 1.53 (d, 3H); 1.80 (s, 3H); 4.29 (m, 1 H); 5.03 (m, 1 H); 6.95-7.15 (m, 3H); 7.43 (m, 1 H); 8.31 (m, 1 H); 8.47-8.51 (m, 2H); 9.23 (S_{breit}, 1H). |
| IA-10 | 2-Chlorpyridin-3-yl | CH₃ | CH₃ | trans-3-Chlorallyl | Öl | *¹H-NMR (CDCl₃), δ[ppm]*: 1.57 (d, 3H); 1.80 (s, 3H); 4.52 (d, 2H); 5.01 (q, 1 H); 6.09 (m, 1 H); 6.18-6.30 (m, 1 H); 6.99-7.13 (m, 3H); 7.03 (m, 1 H); 8.35 (m, 1 H); 8.51 (m, 2H); 9.21 (S_{breit,} 1 H). |
| IA-11 | 2-Chlorpyridin-3-yl | CH₃ | CH₃ | CH₃ | 74-75 | |
| IA-12 | 2-Chlor-pyridin-3-yl | CH₃ | CH₃ | C₂H₅ | Öl | *¹H-NMR (CDCl*₃*), δ[ppm]:*1.25 (d, 3H); 1.58 (d, 3H); 1.80 (s, 3H); 4.11 (m, 2H); 5.02 (m, 1 H); 6.97-7.10 (m, 3H); 7.47 (m, 1 H); 8.31 (m, 1 H); 8.23-8.28 (m, 2H); 9.22 (S_{breit,} 1H). |

| | | | | | | |
|---|---|---|---|---|---|---|
| Allyl: CH₂CH=CH₂; Schmp.: Schmelzpunkt | | | | | | |

### Anwendungsbeispiele:

Die Wirkstoffe wurden als Stammlösung aufbereitet mit 0,25 Gew.% Wirkstoff in Aceton oder Dimethylsulfoxid (DMSO). Dieser Lösung wurde 1 Gew.-% Emulgator Uniperol® EL (Netzmittel mit Emulgier- und Dispergierwirkung auf der Basis ethoxylierter Alkylphenole) zugesetzt und entsprechend der gewünschten Konzentration mit Wasser verdünnt.

### Kurative Wirksamkeit gegen Weizenbraunrost

Blätter von in Töpfen gewachsenen Weizensämlingen der Sorte "Kanzler" wurden mit Sporen des Braunrostes *(Puccinia recondita)* bestäubt. Danach wurden die Töpfe für 24 Stunden in eine Kammer mit hoher Luftfeuchtigkeit (90 bis 95 %) und 20 bis 22 °C gestellt. Während dieser Zeit keimten die Sporen aus und die Keimschläuche drangen in das Blattgewebe ein. Die infizierten Pflanzen wurden am nächsten Tag mit einer wässrigen Suspension in der unten angegebenen Wirkstoffkonzentration bis zur Tropfnässe besprüht. Die Suspensionen oder Emulsionen wurden wie oben beschrieben hergestellt. Nach dem Antrocknen des Spritzbelages wurden die Versuchspflanzen im Gewächshaus bei Temperaturen zwischen 20 und 22 °C und 65 bis 70 % relativer Luftfeuchtigkeit für 7 Tage kultiviert. Danach wurde das Ausmaß der Rostpilzentwicklung auf den Blättern ermittelt.

| Nr. | Befall bei 63 ppm (% Blattfläche) |
|---|---|
| IA-4 | 0 |
| IA-5 | 7 |
| IA-7 | 3 |
| unbehandelt | 90 |

### Protektive Wirksamkeit gegen Puccinia recondita an Weizen (Weizenbraunrost)

Blätter von in Töpfen gewachsenen Weizensämlingen der Sorte "Kanzler" wurden mit einer wässrigen Suspension in der unten angegebenen Wirkstoffkonzentration bis zur Tropfnässe bestäubt. Am nächsten Tag wurden die behandelten Pflanzen mit Sporen des Weizenbraunrostes *(Puccinia recondita)* bestäubt. Anschließend wurden die Pflanzen für 24 Stunden in eine Kammer mit hoher Luftfeuchtigkeit (90 bis 95 %) und 20 bis 22 °C gestellt. Während dieser Zeit keimten die Sporen aus und die Keimschläuche drangen in das Blattgewebe ein. Am folgenden Tag wurden die Versuchspflanzen ins Gewächshaus zurückgestellt und bei Temperaturen zwischen 20 und 22 °C und 65 bis 70 % relativer Luftfeuchtigkeit für weitere 7 Tage kultiviert. Danach wurde das Ausmaß der Rostentwicklung auf den Blättern visuell ermittelt.

| Nr. | Befall bei 63 ppm (% Blattfläche) |
|---|---|
| IA-1 | 10 |
| IA-4 | 3 |
| IA-5 | 3 |
| IA-6 | 5 |
| IA-7 | 3 |
| IA-8 | 3 |
| IA-9 | 5 |
| IA-10 | 5 |
| IA-11 | 10 |
| IA-12 | 3 |
| unbehandelt | 90 |

## Patentansprüche

1. (Hetero)cyclylcarboxanilide der allgemeinen Formel I, 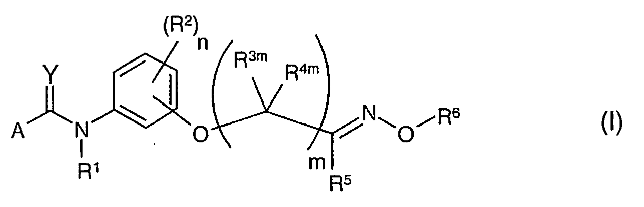 in denen die Variablen die folgenden Bedeutungen haben:
A Phenyl oder ein wenigstens einfach ungesättigter 5- oder 6-gliedrigen Heterocyclus mit 1, 2 oder 3, unter N, O, S, S(=O) und S(=O)₂ ausgewählten Heteroatomen als Ringglieder, wobei Phenyl und der wenigstens einfach ungesättigte 5- oder 6-gliedrige Heterocyclus unsubstituiert sein können oder 1, 2 oder 3 Reste R^{a} tragen können, wobei
R^{a} für Halogen, Nitro, CN, C₁-C₄-Alkyl, C₃-C₆-Cycloalkyl, C₂-C₄-Alkenyl, C₂-C₄-Alkinyl, C₁-C₄-Alkoxy, C₁-C₄-Halogenalkyl, C₃-C₆-Halogencycloalkyl, C₂-C₄-Halogenalkenyl, C₂-C₄-Halogenalkinyl, C₁-C₄-Halogenalkoxy oder Phenyl steht, wobei Phenyl unsubstituiert sein kann oder ein, zwei oder drei Reste R^{b} trägt, die ausgewählt sind unter Halogen, Nitro, CN, C₁-C₄-Alkyl, C₃-C₆-Cycloalkyl, C₂-C₄-Alkenyl, C₂-C₄-Alkinyl, C₁-C₄-Alkoxy, C₁-C₄-Halogenalkyl, C₃-C₆-Halogencycloalkyl, C₂-C₄-Halogenalkenyl, C₂-C₄-Halogenalkinyl und C₁-C₄-Halogenalkoxy;
Y Sauerstoff oder Schwefel;
R¹ H, OH, C₁-C₄-Alkyl, C₃-C₆-Cycloalkyl, C₁-C₄-Alkoxy, C₁-C₄-Halogenalkyl, C₃-C₆-Halogencycloalkyl oder C₁-C₄-Halogenalkoxy;
R² Halogen, Nitro, CN, C₁-C₄-Alkyl, C₃-C₆-Cycloalkyl, C₂-C₄-Alkenyl, C₂-C₄-Alkinyl, C₁-C₄-Alkoxy, C₁-C₄-Halogenalkyl, C₃-C₆-Halogencycloalkyl, C₂-C₄-Halogenalkenyl, C₂-C₄-Halogenalkinyl oder C₁-C₄-Halogenalkoxy;
R^{3m}, R^{4m} jeweils unabhängig voneinander Halogen, Wasserstoff, C₁-C₆-Alkyl, C₃-C₆-Cycloalkyl, C₂-C₆-Alkenyl, C₂-C₆-Alkinyl, Phenyl, Phenyl-C₁-C₄-alkyl, Phenyl-C₂-C₄-alkenyl, Phenyl-C₂-C₄-alkinyl, C₁-C₆-Halogenalkyl, C₃-C₆-Halogencycloalkyl, C₂-C₆-Halogenalkenyl, C₂-C₆-Halogenalkinyl, Phenyl-C₁-C₄-halogenalkyl, Phenyl-C₂-C₄-halogenalkenyl oder Phenyl-C₂-C₄-halogenalkinyl, wobei Phenyl oder der Phenylteil von Phenyl-C₁-C₄-alkyl, Phenyl-C₂-C₄-alkenyl, Phenyl-C₂-C₄-alkinyl, Phenyl-C₁-C₄-halogenalkyl, Phenyl-C₂-C₄-halogenalkenyl und Phenyl-C₂-C₄-halogenalkinyl unsubstituiert sein können oder ein, zwei oder drei Reste R^{b} tragen können; für m = 2 oder 3, die Variablen R³², R⁴² beziehungsweise R³³, R⁴³ auch für C₁-C₆-Alkoxy stehen können;
R⁵ Wasserstoff, C₁-C₆-Alkyl, C₃-C₆-Cycloalkyl, C₂-C₆-Alkenyl, C₂-C₆-Alkinyl, Phenyl, Phenyl-C₁-C₄-alkyl, Phenyl-C₂-C₄-alkenyl, Phenyl-C₂-C₄-alkinyl, C₁-C₆-Halogenalkyl, C₃-C₆-Halogencycloalkyl, C₂-C₆-Halogenalkenyl, C₂-C₆-Halogenalkinyl, Phenyl-C₁-C₄-halogenalkyl, Phenyl-C₂-C₄-halogenalkenyl oder Phenyl-C₂-C₄-halogenalkinyl, wobei Phenyl oder der Phenylteil von Phenyl-C₁-C₄-alkyl, Phenyl-C₂-C₄-alkenyl, Phenyl-C₂-C₄-alkinyl, Phenyl-C₁-C₄-halogenalkyl, Phenyl-C₂-C₄-halogenalkenyl, Phenyl-C₂-C₄-halogenalkinyl unsubstituiert sein können oder ein, zwei oder drei Reste R^{b} tragen können;
R⁶ Wasserstoff, C₁-C₈-Alkyl, C₃-C₆-Cycloalkyl, C₂-C₈-Alkenyl, C₂-C₈-Alkinyl, C₁-C₈-Halogenalkyl, C₃-C₆-Halogencycloalkyl, C₂-C₈-Halogenalkenyl, C₂-C₈-Halogenalkinyl, Phenyl, Naphthyl, Phenyl-C₁-C₆-alkyl, Naphthyl-C₁-C₆-alkyl, Phenyl-C₂-C₆-alkenyl, Phenyl-C₂-C₆-alkinyl, Phenyl-C₁-C₆-halogenalkyl, Phenyl-C₂-C₆-halogenalkenyl oder Phenyl-C₂-C₆-halogenalkinyl, wobei Phenyl und Naphthyl in den 9 zuletzt genannten Gruppen unsubstituiert sein können oder 1, 2 oder 3 unter R^{b} und R⁷ ausgewählte Substituenten tragen können, wobei R⁷ für-(CR⁸)=NOR⁹ steht, worin
R⁸ Wasserstoff, C₁-C₆-Alkyl, C₃-C₆-Cycloalkyl, C₂-C₆-Alkenyl, C₂-C₆-Alkinyl, C₁-C₆-Halogenalkyl, C₃-C₆-Halogencycloalkyl, C₂-C₆-Halogenalkenyl, C₂-C₆-Halogenalkinyl, Phenyl, Benzyl; wobei Phenyl und die Phenylgruppe in Benzyl unsubstituiert sein können oder ein, zwei oder drei Reste R^{b} tragen können; und
R⁹ C₁-C₆-Alkyl, C₃-C₆-Cycloalkyl, C₂-C₆-Alkenyl, C₂-C₆-Alkinyl, C₁-C₆-Halogenalkyl, C₃-C₆-Halogencycloalkyl, C₂-C₆-Halogenalkenyl, C₂-C₆-Halogenalkinyl, Phenyl, Phenyl-C₁-C₄-alkyl, Phenyl-C₁-C₄-halogenalkyl, Phenyl-C₂-C₄-alkenyl, Phenyl-C₂-C₄-halogenalkenyl, Phenyl-C₂-C₄-alkinyl, Phenyl-C₂-C₄-halogenalkinyl, wobei Phenyl und die Phenylgruppe in Phenyl-C₁-C₄-alkyl, Phenyl-C₁-C₄-halogenalkyl, Phenyl-C₂-C₄-alkenyl, Phenyl-C₂-C₄-halogenalkenyl, Phenyl-C₂-C₄-alkinyl und Phenyl-C₂-C₄-halogenalkinyl unsubstituiert sein können oder ein, zwei oder drei Reste R^{b} tragen können;
n 0, 1, 2, 3 oder 4; und
m 1, 2 oder 3;
und die landwirtschaftlich brauchbaren Salze davon.

2. (Hetero)cyclylcarboxanilide der allgemeinen Formel I, worin A für einen Rest der allgemeinen Formeln 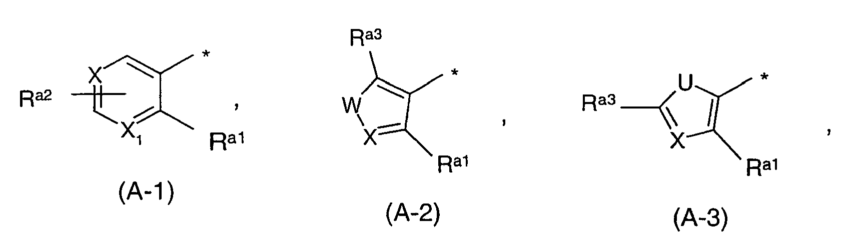 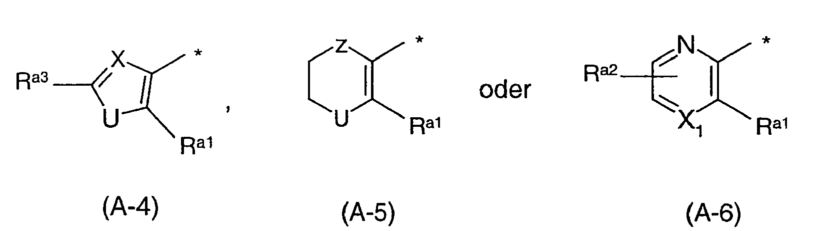 steht, worin * die Bindungsstelle an C(=Y) bedeutet und die Variablen die folgende Bedeutung haben:
X, X₁ jeweils unabhängig voneinander N oder CR^{c}, wobei R^{c} für H steht oder die für R^{b} genannten Bedeutungen aufweist;
W S oder N-R^{a4}, worin R^{a4} für Wasserstoff, C₁-C₄-Alkyl, C₁-C₄-Halogenalkyl, C₁-C₄-Alkoxy, C₁-C₄-Halogenalkoxy oder Phenyl steht, das unsubstituiert sein kann oder 1, 2 oder 3 Reste R^{b} tragen kann;
U Sauerstoff oder Schwefel;
Z S, S(=O), S(=O)₂ oder CH₂;
R^{a1} Wasserstoff, C₁-C₄-Alkyl, C₁-C₄-Alkoxy, C₁-C₄-Halogenalkyl, C₁-C₄-Halogenalkoxy oder Halogen;
R^{a2} jeweils unabhängig voneinander Wasserstoff, Halogen, Nitro, CN, C₁-C₄-Alkyl, C₃-C₆-Cycloalkyl, C₂-C₄-Alkenyl, C₂-C₄-Alkinyl, C₁-C₄-Alkoxy, wobei die 5 zuletzt genannten Gruppen durch Halogen substituiert sein können; und
_{R}^{a3} Wasserstoff, Halogen, Nitro, CN, C₁-C₄-Alkyl, C₃-C₆-Cycloalkyl, C₂-C₄-Alkenyl, C₂-C₄-Alkinyl, C₁-C₄-Alkoxy, wobei die 5 zuletzt genannten Gruppen durch Halogen substituiert sein können.

3. (Hetero)cyclylcarboxanilide der allgemeinen Formel nach Anspruch 2, worin R^{a1} für Wasserstoff, Halogen, C₁-C₂-Alkyl, C₁-C₂-Alkoxy oder C₁-C₂-Fluoralkyl steht.

4. (Hetero)cyclylcarboxanilide der allgemeinen Formel nach Anspruch 2 oder 3, worin A für einen Rest der Formel A-1a, A-2a oder A-3a steht, 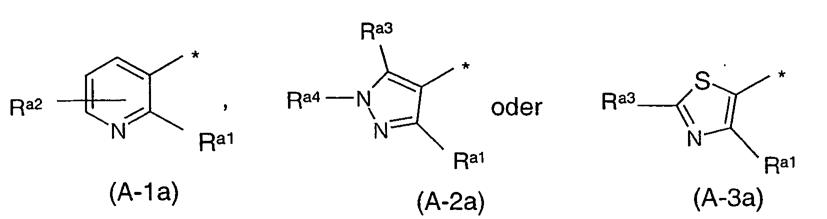 worin R^{a1}, R^{a2}, R^{a3} und R^{a4} die in Anspruch 2 genannten Bedeutungen aufweisen.

5. (Hetero)cyclylcarboxanilide der allgemeinen Formel I nach Anspruch 4, worin A für einen Rest A-1 a mit R^{a1} = Halogen und R^{a2} = Wasserstoff, oder für einen Rest A-2a mit R^{a1} = C₁-C₂-Fluoralkyl, R^{a3} = Wasserstoff und R^{a4} = C₁-C₄-Alkyl oder für einen Rest A-3a mit R^{a1} = C₁-C₂-Fluoralkyl und R^{a3} = C₁-C₄-Alkyl steht.

6. (Hetero)cyclylcarboxanilide der allgemeinen Formel I nach einem der vorhergehenden Ansprüche, worin R¹ für Wasserstoff steht.

7. (Hetero)cyclylcarboxanilide der allgemeinen Formel I nach einem der vorhergehenden Ansprüche, worin R² für C₁-C₄-Alkyl, C₁-C₄-Alkoxy, C₁-C₄-Halogenalkyl, C₁-C₄-Halogenalkoxy, Nitro, Cyano oder Halogen steht.

8. (Hetero)cyclylcarboxanilide der allgemeinen Formel 1 nach einem der vorhergehenden Ansprüche, worin n für 0 oder 1 steht.

9. (Hetero)cyclylcarboxanilide der allgemeinen Formel I nach einem der vorhergehenden Ansprüche, worin m für 1 steht.

10. (Hetero)cyclylcarboxanilide der allgemeinen Formel I nach Anspruch 9, worin R³¹ und R⁴¹ jeweils unabhängig voneinander für Wasserstoff oder C₁-C₄-Alkyl stehen.

11. (Hetero)cyclylcarboxanilide der allgemeinen Formel 1 nach einem der vorhergehenden Ansprüche, worin R⁵ für Wasserstoff, C₁-C₆-Alkyl, C₁-C₆-Halogenalkyl, C₃-C₆-Cycloalkyl, C₃-C₆-Halogencycloalkyl, Phenyl, Phenyl-C₁-C₄-alkyl, Phenyl-C₁-C₄-halogenalkyl steht, wobei Phenyl in den drei zuletzt genannten Resten unsubstituiert sein kann oder ein, zwei oder drei Reste R^{b} tragen kann.

12. (Hetero)cyclylcarboxanilide der allgemeinen Formel I nach einem der vorhergehenden Ansprüche, worin R⁶ für C₁-C₆-Alkyl, C₁-C₆-Halogenalkyl, C₃-C₆-Cycloalkyl, C₃-C₆-Halogencycloalkyl, C₂-C₆-Alkenyl, C₂-C₆-Halogenalkenyl, C₂-C₄-Alkinyl, C₂-C₄-Halogenalkinyl, Phenyl-C₁-C₂-alkyl oder Phenyl steht, wobei Phenyl in den zwei zuletzt genannten Resten unsubstituiert sein kann oder ein oder zwei Halogengruppen tragen kann.

13. (Hetero)cyclylcarboxanilide der allgemeinen Formel I nach einem der vorhergehenden Ansprüche, worin Y für Sauerstoff steht.

14. Verwendung von (Hetero)cyclylcarboxaniliden der allgemeinen Formel I gemäß einem der vorhergehenden Ansprüche und von deren landwirtschaftlich brauchbaren Salzen zur Bekämpfung von Schadpilzen.

15. Pflanzenschutzmittel, enthaltend mindestens ein (Hetero)cyclylcarboxanilid der allgemeinen Formel I gemäß einem der Ansprüche 1 bis 13 oder ein landwirtschaftlich brauchbares Salz davon.

16. Verfahren zur Bekämpfung von Schadpilzen, **dadurch gekennzeichnet, dass** man die Schadpilze, deren Lebensraum oder die von ihnen freizuhaltenden Pflanzen, Flächen, Materialien oder Räume mit einer fungizid wirksamen Menge mindestens eines (Hetero)cyclylcarboxanilids der allgemeinen Formel I gemäß einem der Ansprüche 1 bis 13 oder eines landwirtschaftlich brauchbaren Salzes davon behandelt.

## Claims

1. A (hetero)cyclylcarboxanilide of the formula I, 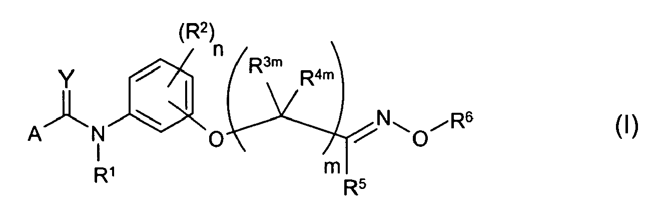 in which variables are as defined below:
A is phenyl or an at least monounsaturated 5- or 6-membered heterocycle having 1, 2 or 3 heteroatoms selected from the group consisting of N, O, S, S(=O) and S(=O)₂ as ring members, where phenyl and the at least monounsaturated 5- or 6-membered heterocycle may be unsubstituted or may carry 1, 2 or 3 radicals R^{a}, where
R^{a} is halogen, nitro, CN, C₁-C₄-alkyl, C₃-C₆-cycloalkyl, C₂-C₄-alkenyl, C₂-C₄-alkynyl, C₁-C₄-alkoxy, C₁-C₄-haloalkyl, C₃-C₆-halocycloalkyl, C₂-C₄-haloalkenyl, C₂-C₄-haloalkynyl, C₁-C₄-haloalkoxy or phenyl, where phenyl may be unsubstituted or carries one, two or three radicals R^{b} selected from the group consisting of halogen, nitro, CN, C₁-C₄-alkyl, C₃-C₆-cycloalkyl, C₂-C₄-alkenyl, C₂-C₄-alkynyl, C₁-C₄-alkoxy, C₁-C₄-haloalkyl, C₃-C₆-halocycloalkyl, C₂-C₄-haloalkenyl, C₂-C₄-haloalkynyl and C₁-C₄-haloalkoxy;
Y is oxygen or sulfur;
R¹ is H, OH, C₁-C₄-alkyl, C₃-C₆-cycloalkyl, C₁-C₄-alkoxy, C₁-C₄-haloalkyl, C₃-C₆-halocycloalkyl or C₁-C₄-haloalkoxy;
R² is halogen, nitro, CN, C₁-C₄-alkyl, C₃-C₆-cycloalkyl, C₂-C₄-alkenyl, C₂-C₄-alkynyl, C₁-C₄-alkoxy, C₁-C₄-haloalkyl, C₃-C₆-halocycloalkyl, C₂-C₄-haloalkenyl, C₂-C₄-haloalkynyl or C₁-C₄-haloalkoxy;
R^{3m}, R^{4m} are each independently of one another halogen, hydrogen, C₁-C₆-alkyl, C₃-C₆-cycloalkyl, C₂-C₆-alkenyl, C₂-C₆-alkynyl, phenyl, phenyl-C₁-C₄-alkyl, phen_{Y}l-C₂-C₄-alkenyl, phenyl-C₂-C₄-alkynyl, C₁-C₆-haloalkyl, C₃-C₆-halocycloalkyl, C₂-C₆-haloalkenyl, C₂-C₆-haloalkynyl, phenyl-C₁-C₄-haloalkyl, phenyl-C₂-C₄-haloalkenyl or phenyl-C₂-C₄-haloalkynyl, where phenyl or the phenyl moiety of phenyl-C₁-C₄-alkyl, phenyl-C₂-C₄-alkenyl, phenyl-C₂-C₄-alkynyl, phenyl-C₁-C₄-haloalkyl, phenyl-C₂-C₄-haloalkenyl and phenyl-C₂-C₄-haloalkynyl may be unsubstituted or may carry one, two or three radicals R^{b}; for m = 2 or 3 the variables R³², R⁴² and R³³, R⁴³, respectively, may also be C₁-C₆-alkoxy;
R⁵ is hydrogen, C₁-C₆-alkyl, C₃-C₆-cycloalkyl, C₂-C₆-alkenyl, C₂-C₆-alkynyl, phenyl, phenyl-C₁-C₄-alkyl, phenyl-C₂-C₄-alkenyl, phenyl-C₂-C₄-alkynyl, C₁-C₆-haloalkyl, C₃-C₆-halocycloalkyl, C₂-C₆-haloalkenyl, C₂-C₆-haloalkynyl, phenyl-C₁-C₄-haloalkyl, phenyl-C₂-C₄-haloalkenyl or phenyl-C₂-C₄-halo-alkynyl, where phenyl or the phenyl moiety of phenyl-C₁-C₄-alkyl, phenyl-C₂-C₄-alkenyl, phenyl-C₂-C₄-alkynyl, phenyl-C₁-C₄-haloalkyl, phenyl-C₂-C₄-haloalkenyl, phenyl-C₂-C₄-haloalkynyl may be unsubstituted or may carry one, two or three radicals R^{b};
R⁶ is hydrogen, C₁-C₈-alkyl, C₃-C₆-cycloalkyl, C₂-C₈-alkenyl, C₂-C₈-alkynyl, C₁-C₈-haloalkyl, C₃-C₆-halocycloalkyl, C₂-C₈-haloalkenyl, C₂-C₈-haloalkynyl, phenyl, naphthyl, phenyl-C₁-C₆-alkyl, naphthyl-C₁-C₆-alkyl, phenyl-C₂-C₆-alkenyl, phenyl-C₂-C₆-alkynyl, phenyl-C₁-C₆-haloalkyl, phenyl-C₂-C₆-haloalkenyl or phenyl-C₂-C₆-haloalkynyl, where phenyl and naphthyl in the 9 last-mentioned groups may be unsubstituted or may carry 1, 2 or 3 substituents selected from the group consisting of R^{b} and R⁷, where R⁷ is -(CR⁸)=NOR⁹, where
R⁸ is hydrogen, C₁-C₆-alkyl, C₃-C₆-cycloalkyl, C₂-C₆-alkenyl, C₂-C₆-alkynyl, C₁-C₆-haloalkyl, C₃-C₆-halocycloalkyl, C₂-C₆-haloalkenyl, C₂-C₆-haloalkynyl, phenyl, benzyl; where phenyl and the phenyl group in benzyl may be unsubstituted or may carry one, two or three radicals R^{b}; and
R⁹ is C₁-C₆-alkyl, C₃-C₆-cycloalkyl, C₂-C₆-alkenyl, C₂-C₆-alkynyl, C₁-C₆-haloalkyl, C₃-C₆-halocycloalkyl, C₂-C₆-haloalkenyl, C₂-C₆-haloalkynyl, phenyl, phenyl-C₁-C₄-alkyl, phenyl-C₁-C₄-haloalkyl, phenyl-C₂-C₄-alkenyl, phenyl-C₂-C₄-haloalkenyl, phenyl-C₂-C₄-alkynyl, phenyl-C₂-C₄-haloalkynyl, where phenyl and the phenyl group in phenyl-C₁-C₄-alkyl, phenyl-C₁-C₄-haloalkyl, phenyl-C₂-C₄-alkenyl, phenyl-C₂-C₄-haloalkenyl, phenyl-C₂-C₄-alkynyl and phenyl-C₂-C₄-haloalkynyl may be unsubstituted or may carry one, two or three radicals R^{b};
n is 0, 1, 2, 3 or 4; and
m is 1, 2 or 3;
or an agriculturally useful salt thereof.

2. A (hetero)cyclylcarboxanilide of the formula I in which A is a radical of the formula 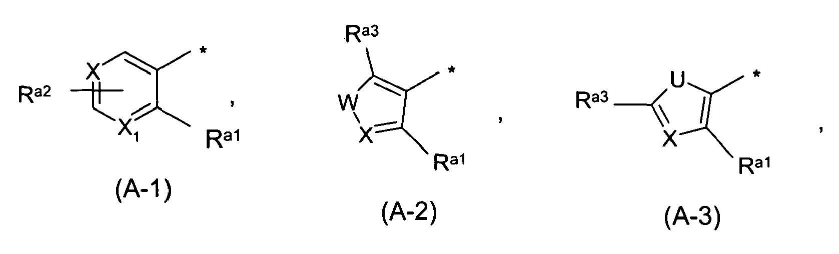 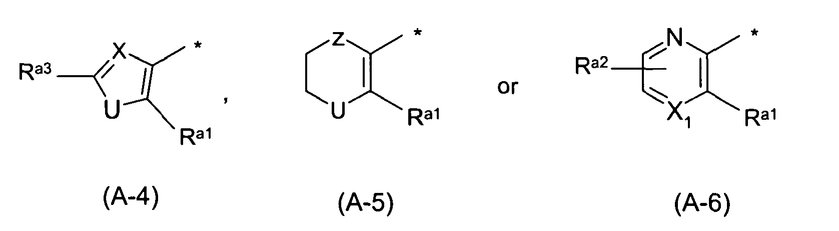 where * means the point of attachment to C(=Y) and the variables are as defined below:
X, X₁ are each independently of one another N or CR^{c}, where R^{c} is H or has one of the meanings mentioned for R^{b};
W is S or N-R^{a4}, where R^{a4} is hydrogen, C₁-C₄-alkyl, C₁-C₄-haloalkyl, C₁-C₄-alkoxy, C₁-C₄-haloalkoxy or phenyl which may be unsubstituted or may carry 1, 2 or 3 radicals R^{b};
U is oxygen or sulfur;
Z is S, S(=O), S(=O)₂ or CH₂,
R^{a1} is hydrogen, C₁-C₄-alkyl, C₁-C₄-alkoxy, C₁-C₄-haloalkyl, C₁-C₄-haloalkoxy or halogen;
R^{a2} are each independently of one another hydrogen, halogen, nitro, CN, C₁-C₄-alkyl, C₃-C₆-cycloalkyl, C₂-C₄-alkenyl, C₂-C₄-alkynyl, C₁-C₄-alkoxy, where the 5 last-mentioned groups may be substituted by halogen; and
R^{a3} is hydrogen, halogen, nitro, CN, C₁-C₄-alkyl, C₃-C₆-cycloalkyl, C₂-C₄-alkenyl, C₂-C₄-alkynyl, C₁-C₄-alkoxy, where the 5 last-mentioned groups may be substituted by halogen.

3. The (hetero)cyclylcarboxanilide of the formula according to claim 2 in which R^{a1} is hydrogen, halogen, C₁-C₂-alkyl, C₁-C₂-alkoxy or C₁-C₂-fluoroalkyl.

4. The (hetero)cyclylcarboxanilide of the formula according to claim 2 or 3 in which A is a radical of the formula A-1 a, A-2a or A-3a, 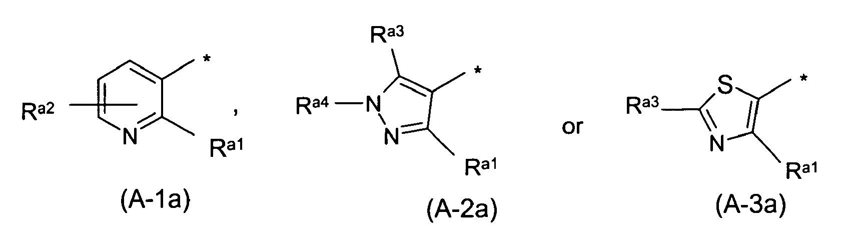 in which R^{a1}, R^{a2}, R^{a3} and R^{a4} are as defined in claim 2.

5. The (hetero)cyclylcarboxanilide of the formula I according to claim 4 in which A is a radical A-1 a where R^{a1} = halogen and R^{a2} = hydrogen, or is a radical A-2a where R^{a1} = C₁-C₂-fluoroalkyl, R^{a3} = is hydrogen and R^{a4} = C₁-C₄-alkyl or is a radical A-3a where R^{a1} = C₁-C₂-fluoroalkyl and R^{a3} = C₁-C₄-alkyl.

6. The (hetero)cyclylcarboxanilide of the formula I according to any of the preceding claims in which R¹ is hydrogen.

7. The (hetero)cyclylcarboxanilide of the formula I according to any of the preceding claims in which R² is C₁-C₄-alkyl, C₁-C₄-alkoxy, C₁-C₄-haloalkyl, C₁-C₄-haloalkoxy, nitro, cyano or halogen.

8. The (hetero)cyclylcarboxanilide of the formula I according to any of the preceding claims in which n is 0 or 1.

9. The (hetero)cyclylcarboxanilide of the formula I according to any of the preceding claims in which m is 1.

10. The (hetero)cyclylcarboxanilide of the formula I according to claim 9 in which R³¹ and R⁴¹ are each independently of one another hydrogen or C₁-C₄-alkyl.

11. The (hetero)cyclylcarboxanilide of the formula I according to any of the preceding claims in which R⁵ is hydrogen, C₁-C₆-alkyl, C₁-C₆-haloalkyl, C₃-C₆-cycloalkyl, C₃-C₆-halocycloalkyl, phenyl, phenyl-C₁-C₄-alkyl, phenyl-C₁-C₄-haloalkyl, where phenyl in the three last-mentioned radicals may be unsubstituted or may carry one, two or three radicals R^{b}.

12. The (hetero)cyclylcarboxanilide of the formula I according to any of the preceding claims in which R⁶ is C₁-C₆-alkyl, C₁-C₆-haloalkyl, C₃-C₆-cycloalkyl, C₃-C₆-halocycloalkyl, C₂-C₆-alkenyl, C₂-C₆-haloalkenyl, C₂-C₄-alkynyl, C₂-C₄-haloalkynyl, phenyl-C₁-C₂-alkyl or phenyl, where phenyl in the two last-mentioned radicals may be unsubstituted or may carry one or two halogen groups.

13. The (hetero)cyclylcarboxanilide of the formula I according to any of the preceding claims in which Y is oxygen.

14. The use of (hetero)cyclylcarboxanilides of the formula I according to any of the preceding claims and of agriculturally useful salts thereof for controlling harmful fungi.

15. A crop protection composition, comprising at least one (hetero)cyclylcarboxanilide of the formula I according to any of claims 1 to 13 or an agriculturally useful salt thereof.

16. A method for controlling harmful fungi, which comprises treating the harmful fungi, their habitat or the plants, areas, materials or spaces to be kept free from them with a fungicidally effective amount of at least one (hetero)cyclylcarboxanilide of the formula I according to any of claims 1 to 13 or an agriculturally useful salt thereof.

## Revendications

1. (Hétéro)cyclylcarboxanilides de formule générale I, 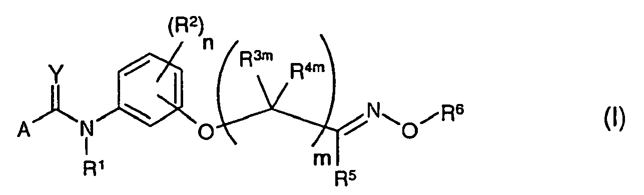 dans laquelle les variables ont la signification suivante:
A phényle ou un hétérocycle à 5 ou 6 chaînons insaturé au moins une fois ayant 1, 2 ou 3 hétéroatomes choisis parmi N, O, S, S(=O) et S(=O)₂ en tant que chaînons, le phényle et l'hétérocycle à 5 ou 6 chaînons insaturé au moins une fois pouvant être non substitués ou pouvant porter 1, 2 ou 3 radicaux R^{a},
R^{a} représentant halogène, nitro, CN, alkyle en C₁-C₄, cycloalkyle en C₃-C₆, alcényle en C₂-C₄, alcynyle en C₂-C₄, alcoxy en C₁-C₄, halogénoalkyle en C₁-C₄, halogénocycloalkyle en C₃-C₆, halogénoalcényle en C₂-C₄, halogénoalcynyle en C₂-C₄, halogénoalcoxy en C₁-C₄, ou phényle, le phényle pouvant être non substitué ou portant un, deux ou trois radicaux R^{b}, qui sont choisis parmi halogène, nitro, CN, alkyle en C₁-C₄, cycloalkyle en C₃-C₆, alcényle en C₂-C₄, alcynyle en C₂-C₄, alcoxy en C₁-C₄, halogénoalkyle en C₁-C₄, halogénocycloalkyle en C₃-C₆, halogénoalcényle en C₂-C₄, halogénoalcynyle en C₂-C₄, halogénoalcoxy en C₁-C₄ ;
Y oxygène ou soufre ;
R¹ H, OH, alkyle en C₁-C₄, cycloalkyle en C₃-C₆, alcoxy en C₁-C₄, halogénoalkyle en C₁-C₄, halogénocycloalkyle en C₃-C₆ ou halogénoalcoxy en C₁-C₄;
R² halogène, nitro, CN, alkyle en C₁-C₄, cycloalkyle en C₃-C₆, alcényle en C₂-C₄, alcynyle en C₂-C₄, alcoxy en C₁-C₄, halogénoalkyle en C₁-C₄, halogénocycloalkyle en C₃-C₆, halogénoalcényle en C₂-C₄, halogénoalcynyle en C₂-C₄ ou halogénoalcoxy en C₁-C₄ ;
R^{3m}, R^{4m} respectivement indépendamment l' un de l'autre halogène, hydrogène, alkyle en C₁-C₆, cycloalkyle en C₃-C₆, alcényle en C₂-C₆, alcynyle en C₂-C₆, phényle, phényl(alkyle en C₁-C₄), phényl(alcényle en C₂-C₄), phényl(alcynyle en C₂-C₄), halogénoalkyle en C₁-C₆, halogénocycloalkyle en C₃-C₆, halogénoalcényle en C₂-C₆, halogénoalcynyle en C₂-C₆, phényl(halogénoalkyle en C₁-C₄), phényl(halogénoalcényle en C₂-C₄) ou phényl(halogénoalcynyle en C₂-C₄), le phényle ou la partie phényle d'un groupe phényl(alkyle en C₁-C₄), phényl(alcényle en C₂-C₄), phényl(alcynyle en C₂-C₄), phényl(halogénoalkyle en C₁-C₄), phényl(halogénoalcényle en C₂-C₄) et phényl(halogénoalcynyle en C₂-C₄) pouvant être non substitué(e) ou pouvant porter un, deux ou trois radicaux R^{b} ; pour m = 2 ou 3, les variables R³², R⁴² ou bien R³³, R⁴³ pouvant représenter aussi de l'alcoxy en C₁-C₆ ;
R⁵ hydrogène, alkyle en C₁-C₆, cycloalkyle en C₃-C₆, alcényle en C₂-C₆, alcynyle en C₂-C₆, phényle, phényl(alkyle en C₁-C₄), phényl(alcényle en C₂-C₄), phényl (alcynyle en C₂-C₄), halogénoalkyle en C₁-C₆, halogénocycloalkyle en C₃-C₆, halogénoalcényle en C₂-C₆, halogénoalcynyle en C₂-C₆, phényl(halogénoalkyle en C₁-C₄), phényl(halogénoalcényle en C₂-C₄) ou phényl(halogénoalcynyle en C₂-C₄), le phényle ou la partie phényle d'un groupe phényl(alkyle en C₁-C₄), phényl(alcényle en C₂-C₄), phényl(alcynyle en C₂-C₄), phényl(halogénoalkyle en C₁-C₄), phényl(halogénoalcényle en C₂-C₄), phényl(halogénoalcynyle en C₂-C₄) pouvant être non substitué(e) ou pouvant porter un, deux ou trois radicaux R^{b} ;
R⁶ hydrogène, alkyle en C₁-C₈, cycloalkyle en C₃-C₆, alcényle en C₂-C₈, alcynyle en C₂-C₈, halogénoalkyle en C₁-C₈, halogénocycloalkyle en C₃-C₆, halogénoalcényle en C₂-C₈, halogénoalcynyle en C₂-C₈, phényle, naphtyle, phényl (alkyle en C₁-C₆), naphtyl (alkyle en C₁-C₆), phényl(alcényle en C₂-C₆), phényl(alcynyle en C₂-C₆), phényl(halogénoalkyle en C₁-C₆), phényl(halogénoalcényle en C₂-C₆) ou phényl(halogénoalcynyle en C₂-C₆), le phényle ou le naphtyle dans les 9 groupes mentionnés en dernier pouvant être non substitué ou pouvant porter 1, 2 ou 3 substituants choisis parmi R^{b} et R⁷, R⁷ représentant - (CR⁸) =NOR⁹,
R⁸ signifiant hydrogène, alkyle en C₁-C₆, cycloalkyle en C₃-C₆, alcényle en C₂-C₆, alcynyle en C₂-C₆, halogénoalkyle en C₁-C₆, halogénocycloalkyle en C₃-C₆, halogénoalcényle en C₂-C₆, halogénoalcynyle en C₂-C₆, phényle, benzyle ; le phényle et le groupe phényle dans le benzyle pouvant être non substitués ou pouvant porter un, deux ou trois radicaux R^{b} ; et
R⁹ signifiant alkyle en C₁-C₆, cycloalkyle en C₃-C₆, alcényle en C₂-C₆, alcynyle en C₂-C₆, halogénoalkyle en C₁-C₆, halogénocycloalkyle en C₃-C₆, halogénoalcényle en C₂-C₆, halogénoalcynyle en C₂-C₆, phényle, phényl (alkyle en C₁-C₄), phényl (halogénoalkyle en C₁-C₄), phényl(alcényle en C₂-C₄), phényl(halogénoalcényle en C₂-C₄), phényl(alcynyle en C₂-C₄), phényl(halogénoalcynyle en C₂-C₄), phényle et le groupe phényle dans les groupes phényl(alkyle en C₁-C₄), phényl(halogénoalkyle en C₁-C₄), phényl(alcényle en C₂-C₄), phényl(halogénoalcényle en C₂-C₄), phényl(alcynyle en C₂-C₄) et phényl(halogénoalcynyle en C₂-C₄) pouvant être non substitués ou pouvant porter un, deux ou trois radicaux R^{b} ;
n 0, 1, 2, 3 ou 4 ; et
m 1, 2 ou 3 ;
et les sels de ceux-ci pouvant être utilisés en agriculture.

2. (Hétéro)cyclylcarboxanilides de formule générale I, dans laquelle A représente un radical des formules générales 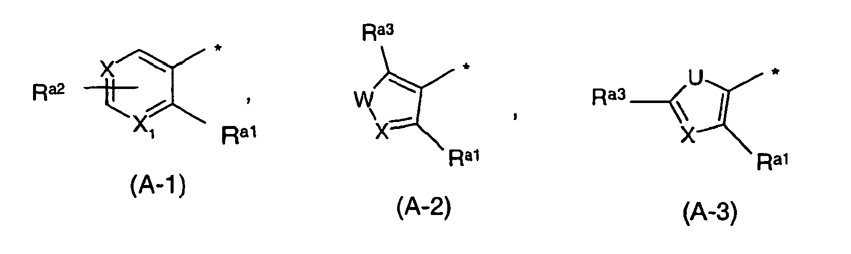 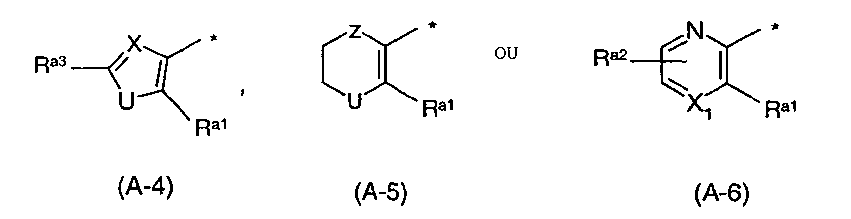 dans lesquelles * signifie le point de liaison sur C(=Y) et les variables ont la signification suivante :
X, X₁ respectivement indépendamment l'un de l'autre N ou CR^{c}, R^{c} représentant H ou ayant les significations données pour R^{b} ;
W S ou N-R^{a4}, R^{a4} représentant hydrogène, alkyle en C₁-C₄, halogénoalkyle en C₁-C₄, alcoxy en C₁-C₄, halogénoalcoxy en C₁-C₄ ou phényle qui peut être non substitué ou porter 1, 2 ou 3 radicaux R^{b} ;
U oxygène ou soufre ;
Z S, S (=O), S (=O) ou CH₂ ;
R^{a1} hydrogène, alkyle en C₁-C₄, alcoxy en C₁-C₄, halogénoalkyle en C₁-C₄, halogénoalcoxy en C₁-C₄ ou halogène ;
R^{a2} respectivement indépendamment l'un de l'autre hydrogène, halogène, nitro, CN, alkyle en C₁-C₄, cycloalkyle en C₃-C₆, alcényle en C₂-C₄, alcynyle en C₂-C₄, alcoxy en C₁-C₄, les 5 groupes mentionnés en dernier pouvant être substitués par de l'halogène ; et
R^{a3} hydrogène, halogène, nitro, CN, alkyle en C₁-C₄, cycloalkyle en C₃-C₆, alcényle en C₂-C₄, alcynyle en C₂-C₄, alcoxy en C₁-C₄, les 5 groupes mentionnés en dernier pouvant être substitués par de l'halogène.

3. (Hétéro)cyclylcarboxanilides de formule générale I selon la revendication 2, dans laquelle R^{a1} représente hydrogène, halogène, alkyle en C₁-C₂, alcoxy en C₁-C₂ ou fluoroalkyle en C₁-C₂.

4. (Hétéro)cyclylcarboxanilides de formule générale I selon la revendication 2 ou 3, dans laquelle A représente un radical de formule A-1a, A-2a ou A-3a, 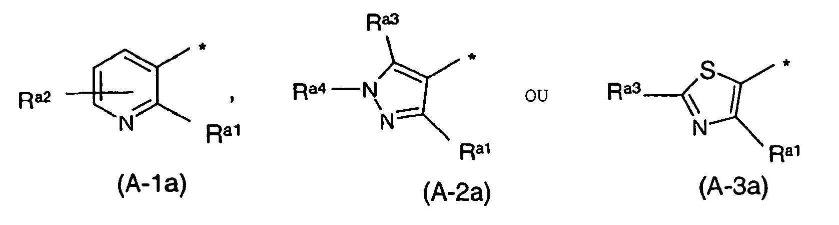
R^{a1}, R^{a2}, R^{a3} et R^{a4} ayant les significations mentionnées dans la revendication 2.

5. (Hétéro)cyclylcarboxanilides de formule générale I selon la revendication 4, dans laquelle A représente un radical A-1a avec R^{a1} = halogène et R^{a2} = hydrogène, ou représente un radical A-2a avec R^{a1} = fluoroalkyle en C₁-C₂, R^{a3} = hydrogène et R^{a4} = alkyle en C₁-C₄ ou représente un radical A-3a avec R^{a1} = fluoroalkyle en C₁-C₂ et R^{a3} = alkyle en C₁-C₄.

6. (Hétéro)cyclylcarboxanilides de formule générale I selon l'une quelconque des revendications précédentes, dans laquelle R¹ représente de l'hydrogène.

7. (Hétéro)cyclylcarboxanilides de formule générale I selon l'une quelconque des revendications précédentes, dans laquelle R² représente alkyle en C₁-C₄, alcoxy en C₁-C₄, halogénoalkyle en C₁-C₄, halogénoalcoxy en C₁-C₄, nitro, cyano ou halogène.

8. (Hétéro)cyclylcarboxanilides de formule générale I selon l'une quelconque des revendications précédentes, dans laquelle n représente 0 ou 1.

9. (Hétéro)cyclylcarboxanilides de formule générale I selon l'une quelconque des revendications précédentes, dans laquelle m représente 1.

10. (Hétéro)cyclylcarboxanilides de formule générale I selon la revendication 9, dans laquelle R³¹ et R⁴¹ représentent respectivement indépendamment l'un de l'autre hydrogène ou alkyle en C₁-C₄.

11. (Hétéro)cyclylcarboxanilides de formule générale I selon l'une quelconque des revendications précédentes, dans laquelle R⁵ représente hydrogène, alkyle en C₁-C₆, halogénoalkyle en C₁-C₆, cycloalkyle en C₃-C₆, halogénocycloalkyle en C₃-C₆, phényle, phényl(alkyle en C₁-C₄), phénylhalogénoalkyle en C₁-C₄, le phényle dans les trois radicaux mentionnés en dernier pouvant être non substitué ou pouvant porter un, deux ou trois radicaux R^{b}.

12. (Hétéro)cyclylcarboxanilides de formule générale I selon l'une quelconque des revendications précédentes, dans laquelle R⁶ représente alkyle en C₁-C₆, halogénoalkyle en C₁-C₆, cycloalkyle en C₃-C₆, halogénocycloalkyle en C₃-C₆, alcényle en C₂-C₆, halogénoalcényle en C₂-C₆, alcynyle en C₂-C₄, halogénoalcynyle en C₂-C₄, phényl(alkyle en C₁-C₂) ou phényle, le phényle dans les deux radicaux mentionnés en dernier pouvant être non substitué ou pouvant porter un ou deux groupes halogène.

13. (Hétéro)cyclylcarboxanilides de formule générale I selon l'une quelconque des revendications précédentes, dans laquelle Y représente de l'oxygène.

14. Utilisation des (hétéro)cyclylcarboxanilides de formule générale I selon l'une quelconque des revendications précédentes et des sels de ceux-ci pouvant être utilisés en agriculture pour la lutte contre des champignons nuisibles.

15. Produit phytosanitaire, contenant au moins un (hétéro)cyclylcarboxanilide de formule générale selon l'une quelconque des revendications 1 à 13 ou un sel de celui-ci pouvant être utilisé en agriculture.

16. Procédé de lutte contre des champignons nuisibles, **caractérisé en ce que** les champignons nuisibles, leur espace vital ou les plantes, les surfaces, les matériaux ou les espaces à préserver de ceux-ci sont traités avec une quantité efficace de manière fongicide d'au moins un (hétéro)cyclylcarboxanilide de formule générale I selon l'une quelconque des revendications 1 à 13 ou d'un sel de celui-ci pouvant être utilisé en agriculture.
